(19) Europäisches Patentamt / European Patent Office / Office européen des brevets

(11) EP 4 357 347 B1

(12) **EUROPEAN PATENT SPECIFICATION**

(45) Date of publication and mention of the grant of the patent: **11.12.2024 Bulletin 2024/50**

(21) Application number: **22827573.1**

(22) Date of filing: **21.06.2022**

(51) International Patent Classification (IPC):
***C07D 487/04*** (2006.01) ***A61P 35/00*** (2006.01)
***A61K 31/4188*** (2006.01) ***A61K 31/454*** (2006.01)
***A61K 31/4545*** (2006.01) ***A61K 31/496*** (2006.01)
***A61K 31/438*** (2006.01)

(52) Cooperative Patent Classification (CPC):
**C07D 487/04; A61P 35/00**

(86) International application number:
**PCT/CN2022/100186**

(87) International publication number:
**WO 2022/268080 (29.12.2022 Gazette 2022/52)**

(54) **TRK KINASE INHIBITOR COMPOUND AND USE THEREOF**

TRK-KINASEHEMMERVERBINDUNG UND VERWENDUNG DAVON

COMPOSÉ INHIBITEUR DE LA KINASE TRK ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **21.06.2021 CN 202110685264**

(43) Date of publication of application:
**24.04.2024 Bulletin 2024/17**

(73) Proprietor: **Henan Medinno Pharmaceutical Technology Co., Ltd.**
**Zhengzhou, Henan 450000 (CN)**

(72) Inventors:
- **LU, Liang**
  **Henan 450000 (CN)**
- **HUANG, Hai**
  **Henan 450000 (CN)**
- **ZHANG, Longzheng**
  **Henan 450000 (CN)**
- **ZHAO, Saisai**
  **Henan 450000 (CN)**
- **ZHANG, Jixuan**
  **Henan 450000 (CN)**
- **WANG, Xiaolong**
  **Henan 450000 (CN)**
- **ZHU, Junjie**
  **Henan 450000 (CN)**
- **LIAO, Xinwei**
  **Henan 450000 (CN)**
- **CHEN, Jiaxin**
  **Henan 450000 (CN)**
- **LING, Shancun**
  **Henan 450000 (CN)**

(74) Representative: **Kraus & Lederer PartGmbB**
**Thomas-Wimmer-Ring 15**
**80539 München (DE)**

(56) References cited:
**EP-A1- 4 056 558** **WO-A1-2012/034091**
**WO-A1-2017/077283** **WO-A1-2021/088859**
**CN-A- 102 056 927** **CN-A- 103 534 257**
**CN-A- 111 606 908** **CN-A- 112 979 654**
**KR-A- 20140 019 055**

- **DUAN YUNXIN ET AL: "Design, synthesis, and Structure Activity Relationships (SAR) of 3-vinylindazole derivatives as new selective tropomyosin receptor kinases (Trk) inhibitors", EUROPEAN JOURNAL OF MEDICINAL CHEMISTRY, vol. 203, 13 July 2020 (2020-07-13), XP055810040, DOI: 10.1016/j.ejmech.2020.112552**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- SHIRAHASHI HIROMITSU ET AL: "The discovery of novel 3-aryl-indazole derivatives as peripherally restricted pan-Trk inhibitors for the treatment of pain", 17 June 2019, BIOORGANIC & MEDICINAL CHEMISTRY LETTERS, ELSEVIER, AMSTERDAM NL, PAGE(S) 2320 - 2326, ISSN: 0960-894X, XP085759046
- WANG BEILEI ET AL: "Discovery of (E)-N-(4-methyl-5-(3-(2-(pyridin-2-yl)vinyl)-1H-indazol-6-yl)thiazol-2-yl)-2-(4-methylpiperazin-1-yl)acetamide (IHMT-TRK-284) as a novel orally available type II TRK kinase inhibitor capable of overcoming multiple resistant mutants", 30 August 2020, EUROPEAN JOURNAL OF MEDICINAL CHEMISTRY, ELSEVIER, AMSTERDAM, NL, ISSN: 0223-5234, XP086336490

## Description

### TECHNICAL FIELD

The present disclosure provides a class of novel compounds with pharmacological activity, which can be used to inhibit TRK kinase activity. The present disclosure also relates to a composition comprising the compound, and use of the compound and the composition in the preparation of a medicament for the treatment of TRK kinase or NTRK gene-related diseases or disorders.

### BACKGROUND

Protein kinases (PK) are a key regulator of cell growth, proliferation and survival, and malfunction of the protein kinases is a hallmark of many diseases. A large proportion of oncogenes and proto-oncogenes associated with human cancers encode PK, and a TRK kinase (tropomyosin receptor kinase), as a type of protein kinase, has attracted the attention of researchers in recent years.

The TRK kinase belongs to a receptor tyrosine kinase family, which consists of three members, TRKA, TRKB and TRKC, encoded by the neurotrophic receptor tyrosine kinase (NTRK) genes: NTRK1, NTRK2 and NTRK3, respectively. Previous studies have confirmed that these TRK kinases, which are receptors for nerve growth factor (NGF), brain-derived neurotrophic factor (BDNF), neurotrophic factor 4/5 (NT-4/5), and neurotrophic factor 3 (NT-3), can regulate neuronal cell signal maintenance, neuronal cell signaling, cell proliferation, differentiation, metabolism, and even apoptosis. TRK kinases are constitutively activated in human malignancies by several mechanisms. The most recognized mechanism is NTRK gene fusion, in which 3' NTRK gene regions are rearranged within or between chromosomes and then sequenced with 5' fusion partner genes, resulting in a high risk of tumorigenesis. Activation or dysregulation of TRK kinases, as well as fusion of NTRK genes, have been shown to be closely associated with the development, progression, and deterioration of a wide variety of tumors or cancers; TRK is an important target for tumor therapy in patients with NTRK fusion gene expression. Therefore, small molecule TRK kinase inhibitors are considered a promising broad-spectrum anticancer drug. In addition, TRK inhibitors have been shown to be effective in preclinical pain animal models and preclinical inflammation animal models.

Larotrectinib, also known as LOXO-101 or Vitrakvi, is the first TRK inhibitor anticancer drug approved for marketing. It has shown significant efficacy in the treatment of some cancers, but it is extremely expensive and has insurmountable resistance problems with long-term use. The second TRK inhibitor drug approved for marketing is Entrectinib, also known as Rozlytrek, which has also shown good broad-spectrum anti-cancer activity. Other TRK inhibitors have been disclosed, for example, in WO 2021/088859 and KR 2014 0019055.

The success of Larotrectinib and Entrectinib has led researchers to recognize the importance and promise of TRK inhibitors in cancer treatment. However, there is still an urgent need for other alternative TRK inhibitors.

### SUMMARY

In a first aspect, the present disclosure provides a compound of Formula (I) as a TRK kinase inhibitor,

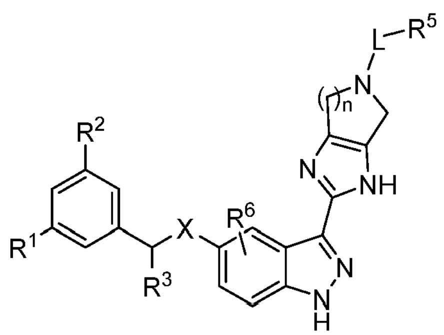

(I)

or an isotopically labeled compound thereof, or an optical isomer thereof, a geometric isomer thereof, a tautomer thereof, or a pharmaceutically acceptable salt thereof, wherein

$R^1$ and $R^2$ are each independently selected from H, -CN, $C_{1-4}$ alkyl, $C_{1-4}$ haloalkyl, $C_{1-4}$ alkoxy, and halogen; and

$R^3$ is selected from H, $C_{1-4}$ alkyl, $C_{1-4}$ haloalkyl, $C_{1-4}$ alkoxy, $C_{3-6}$ alicyclic group, and 4-6 membered heteroalicyclic group; and

1, 2 or 3 $R^6$(s) are present in formula (I), and each $R^6$ is independently selected from H, halogen, -CN, -OH, $-NO_2$, $-N(R^7)(R^8)$, $C_{1-4}$ alkyl, $C_{1-4}$ haloalkyl, $C_{1-4}$ alkoxy, $C_{3-6}$ alicyclic group, and 4-6 membered heteroalicyclic group; and

X is a bond, O, S or $(NR^4)$ in which $R^4$ is selected from H, $C_{1-4}$ alkyl, $C_{1-4}$ haloalkyl, $C_{1-4}$ alkoxy, $C_{3-6}$ alicyclic group, and 4-6 membered heteroalicyclic group; and

$R^7$ and $R^8$ are each independently selected from H, $C_{1-6}$ alkyl, $C_{1-4}$ haloalkyl, and $C_{1-4}$ alkoxy, or $R^7$ and $R^8$ and the atom(s) attached thereto together form a 3-6-membered ring; and

n=1, 2 or 3; and

L is (C=O), (O=S=O), $CR^aR^b$ or a bond in which $R^a$ and $R^b$ are each indepdnently selected from H, $C_{1-4}$ alkyl, $C_{1-4}$ haloalkyl, $C_{1-4}$ alkoxy, $C_{3-6}$ alicyclic group, and 4-6 membered heteroalicyclic group, or $R^a$, $R^b$, and the carbon atom(s) attached thereto together form a 3-6-membered ring; and

$R^5$ is selected from H, halogen, -OH, $-NO_2$, -CN, $-SF_5$, -SH, $-S-C_{1-4}$ alkyl, $C_{1-6}$ alkyl, $C_{1-6}$ haloalkyl, $C_{1-6}$ alkoxy, $C_{1-6}$ haloalkoxy, $C_{2-8}$ alkenyl, $C_{2-8}$ alkynyl, $C_{3-7}$ alicyclic group, 3-10 membered heteroalicyclic group, $C_{6-12}$ bicyclic alicyclic group, 6-12 membered bicyclic heteroalicyclic group, $C_{8-15}$ tricyclic alicyclic group, 8-15 membered tricyclic heteroalicyclic group, $C_{5-8}$ aryl, 5-10 membered heteroaryl, $C_{7-11}$ bicyclic aryl, 7-11 membered bicyclic heteroaryl, $-C_{1-4}$ alkyl-($C_{3-7}$ alicyclic group), $-C_{1-4}$ alkyl-(3-10 membered heteroalicyclic group), $-C_{1-4}$ alkyl-($C_{6-12}$ bicyclic alicyclic group), $-C_{1-4}$ alkyl-(6-12 membered bicyclic heteroalicyclic group), $-C_{1-4}$ alkyl-($C_{8-15}$ tricyclic alicyclic group), $-C_{1-4}$ alkyl-(8-15 membered tricyclic heteroalicyclic group), $-C_{1-4}$ alkyl-($C_{5-8}$ aryl), $-C_{1-4}$ alkyl-(5-10 membered heteroaryl), $-N(R^{10})(R^{11})$, $-N(R^{10})(C(=O)R^{11})$, $-N(R^{10})(C(=O)-OR^{11})$, $-N(R^{12})(C(=O)-N(R^{10})(R^{11}))$, $-C(=O)-N(R^{10})(R^{11})$, $-C(=O)-R^{12}$, $-C(=O)-OR^{12}$, $-OC(=O)R^{12}$, $- N(R^{10})S(=O)_2R^{11})$, $-S(=O)_2-N(R^{10})(R^{11})$, $-SR^{12}$, and $-OR^{12}$, wherein the $-S-C_{1-4}$ alkyl, $C_{1-6}$ alkyl, $C_{1-6}$ alkoxy, $C_{1-6}$ haloalkoxy, $C_{2-8}$ alkenyl, $C_{2-8}$ alkynyl, $C_{3-7}$ alicyclic group, 3-10 membered heteroalicyclic group, $C_{6-12}$ bicyclic alicyclic group, 6-12 membered bicyclic heteroalicyclic group, $C_{8-15}$ tricyclic alicyclic group, 8-15 membered tricyclic heteroalicyclic group, $C_{5-8}$ aryl, 5-7 membered heteroaryl, $C_{7-11}$ bicycloaryl, 7-11 membered bicyclic heteroaryl, $-C_{1-4}$ alkyl-($C_{3-7}$ alicyclic group), $-C_{1-4}$ alkyl-(3-10 membered heteroalicyclic group), $-C_{1-4}$ alkyl-($C_{6-12}$ bicyclic alicyclic group), $-C_{1-4}$ alkyl-(6-12 membered bicyclic heteroalicyclic group), $-C_{1-4}$ alkyl-($C_{8-15}$ tricyclic alicyclic group), and $-C_{1-4}$ alkyl-(8-15 membered tricyclic heteroalicyclic group) are each optionally substituted with 0, 1, 2, 3 or 4 $R^{5a}$; and $R^{5a}$ is independently selected from halogen, -OH, $-NO_2$, -CN, $-SF_5$, -SH, $-S-C_{1-4}$ alkyl, oxo, $C_{1-4}$ alkyl, $C_{1-4}$ haloalkyl, $C_{1-4}$ alkoxy, $C_{1-4}$ haloalkoxy, $C_{2-8}$ alkenyl, $C_{2-8}$ alkynyl, $C_{3-7}$ alicyclic group, 3-10 membered heteroalicyclic group, $C_{5-8}$ aryl, 5-7 membered heteroaryl, $- N(R^{13})(R^{14})$, $-N(R^{13})(C(=O)R^{14})$, $-N(R^{13})(C(=O)-OR^{14})$, $-N(R^{15})(C(=O)-N(R^{13})(R^{14}))$, $- C(=O)-N(R^{13})(R^{14})$, $-C(=O)-R^{15}$, $-C(=O)-OR^{15}$, $-OC(=O)R^{15}$, $-N(R^{13})(S(=O)_2R^{14})$, $-S(=O)_2-N(R^{13})(R^{14})$, $-SR^{15}$, and $-OR^{15}$; and

$R^{10}$, $R^{11}$, $R^{12}$, $R^{13}$, $R^{14}$ and $R^{15}$, at each occurrence, are each independently selected from H, $C_{1-6}$ alkyl, $C_{1-6}$ haloalkyl, $C_{3-7}$ alicyclic group, 3-10 membered heteroalicyclic group, $C_{5-8}$ aryl, 5-7 membered heteroaryl, $C_{7-11}$ bicycloaryl, 7-11 membered bicyclic heteroaryl, $-C_{1-4}$ alkyl-($C_{3-7}$ alicyclic group), $-C_{1-4}$ alkyl-(3-10 membered heteroalicyclic group), $-C_{1-4}$ alkyl-($C_{6-12}$ bicyclic alicyclic group), $-C_{1-4}$ alkyl-(6-12 membered bicyclic heteroalicyclic group), $- C_{1-4}$ alkyl-($C_{8-15}$ tricyclic alicyclic group), $-C_{1-4}$ alkyl-(8-15 membered tricyclic heteroalicyclic group), $-C_{1-4}$ alkyl-($C_{5-8}$ aryl), $-C_{1-4}$ alkyl-(5-10 membered heteroaryl), wherein each substituent listed in the group is optionally substituted with 0, 1, 2, 3, or 4 substituents each independently selected from a group consisting of: halogen, -OH, $-NH_2$, $-NH(CH_3)$, $-N(CH_3)_2$, -CN, $-NO_2$, $-SF_5$, -SH, $-S-C_{1-4}$ alkyl, oxo, $C_{1-4}$ alkyl, $C_{2-6}$ alkenyl, $C_{2-6}$ alkynyl, $C_{3-7}$ alicyclic group, 3-10 membered heteroalicyclic group, $C_{5-8}$ aryl, 5-7 membered heteroaryl, $C_{7-11}$ bicycloaryl, 7-11 membered bicyclic heteroaryl, $C_{1-4}$ hydroxyalkyl, $-S-C_{1-4}$ alkyl, -C(=O)H, $-C(=O)-C_{1-4}$ alkyl, $-C(=O)-O-C_{1-4}$ alkyl, $-C(=O)-NH_2$, $-C(=O)-N(C_{1-4}\ alkyl)_2$, $C_{1-4}$ haloalkyl, $C_{1-4}$ alkoxy and $C_{1-4}$ haloalkoxy;

or $R^{10}$, $R^{11}$, and the atom(s) attached thereto together form a 3-14-membered ring;

or $R^{13}$, $R^{14}$, and the atom(s) attached thereto together form a 3-14-membered ring.

Unless otherwise indicated, the term "a compound as shown by Formula (I)", "a compound of Formula (I)" or "a compound according to the present application" also encompasses any optical isomer, geometric isomer, tautomer or a mixture of various optical or geometric isomers of the compound.

The term "optical isomer" refers that when a compound has one or more chiral centers, each chiral center may have an R configuration or an S configuration, and the various isomers thus constituted are known as an optical isomer. Optical isomers comprise all diastereomers, enantiomers, meso forms, racemates or mixtures thereof. For example, optical isomers can be separated by a chiral chromatography or by chiral synthesis.

The term "geometric isomer" refers that when a double bond is present in a compound, the compound may exist as a cis isomer, a trans isomer, an E isomer, or a Z isomer. A geometric isomer comprises a cis isomer, trans isomer, E isomer, Z isomer, or a mixture thereof.

The term "tautomer" refers to an isomer that is formed by rapid movement of an atom at two positions in a single molecule. It will be understood by those skilled in the art that tautomers can be mutually transformed, and in a

certain state, may coexist by reaching an equilibrium state.

Unless otherwise indicated, reference to "a compound as shown by Formula (I)", "a compound of Formula (I)", or "a compound according to the present application" herein also encompasses isotopically-labeled compounds obtained by replacing any atom of the compound with its isotopic atom.

Examples of isotopes suitable for inclusion in the compounds of the invention include isotopes of hydrogen, such as $^{2}H$ (D) and $^{3}H$ (T), of carbon, such as $^{11}C$, $^{13}C$ and $^{14}C$, of chlorine, such as $^{36}Cl$, of fluorine, such as $^{18}F$, of iodine, such as $^{123}I$ and $^{125}I$, of nitrogen, such as $^{13}N$ and $^{15}N$, of oxygen, such as $^{15}O$, $^{17}O$ and $^{18}O$, and of sulphur, such as $^{35}S$.

The isotopically-labelled compounds, for example, those incorporating a radioactive isotope, are useful in drug and/or substrate tissue distribution studies. The radioactive isotopes deuterium, i.e. $^{2}H$, and carbon-14, i.e. $^{14}C$, are particularly useful for this purpose in view of their ease of incorporation and ready means of detection.

Substitution with heavier isotopes such as deuterium, i.e. D, may afford certain therapeutic advantages resulting from greater metabolic stability, for example, increased in vivo half-life or reduced dosage requirements, and hence may be preferred in some circumstances. Thus, in some embodiments, the compounds according to the present application are isotopically labeled compounds, wherein H is optionally replaced by D at each occurrence.

Substitution with positron emitting isotopes, such as $^{11}C$, $^{18}F$, $^{15}O$ and $^{13}N$, can be useful in Positron Emission Topography (PET) studies for examining substrate receptor occupancy.

The isotopically-labeled compounds can generally be prepared by conventional techniques known to those skilled in the art or using an appropriate isotopically-labeled reagents in place of the non-labeled reagent previously employed.

The compounds according to the present application may exist in the form of a pharmaceutically acceptable salt.

The term "pharmaceutically acceptable" means that the corresponding compound, vehicle or molecule is suitable for administration to humans. Preferably, the term refers to the use for mammalian, preferably human approved by any national regulatory agency such as CFDA (China), EMEA (Europe), FDA (USA), etc.

The pharmaceutically acceptable salt include acid addition salts and base addition salts thereof. Suitable acid addition salts are formed from acids that form non-toxic salts. Examples include but are not limited to: acetate, adipate, aspartate, benzoate, benzenesulfonate, bicarbonate/carbonate, bisulfate/sulfate, borate, camphor sulfonate, citrate, cyclohexamine sulfonate, ethanedisulfonate, formate, fumarate, glucoheptonate, gluconate, glucuronate, hexafluorophosphate, 2-(4-hydroxybenzyl) benzoate, hydrochloride/chloride, hydrobromide/bromide, hydroiodide/iodide, 2-isethionate, lactate, malate, maleate, malonate, methanesulfonate, methyl sulfate, naphthalate, 2-naphthalenesulfonate, nicotinate, nitrate, orotate, oxalate, palmitate, phosphate/hydrogen phosphate/dihydrogen phosphate, pyroglutamate, glucarate, stearate, salicylate, tannate, tartrate, tosylate and trifluoroacetate. Suitable base addition salts are formed from bases that form non-toxic salts. Examples thereof include, but are not limited to: aluminum, arginine, calcium, choline, diethylamine, diethanolamine, glycine, lysine, magnesium, meglumine, ethanolamine, potassium, sodium, tromethamine, and zinc salts. It is also possible to form half salts of acids and bases, such as hemisulfate and hemicalcium salts. For a review of suitable salts, please refer to Handbook of Pharmaceutical Salts: Properties, Selection and Use by Stahl and Wermuth (Wiley-VCH, 2002). Methods for preparing pharmaceutically acceptable salts of the compounds described herein are known to those skilled in the art.

Moreover, the compounds according to the present application may exist in unsolvated form as well as solvated forms with pharmaceutically acceptable solvents such as water, ethanol and the like. The compounds may exist in one or more crystalline forms, or in an amorphous form. These forms are included within the scope of the invention.

Disclosed but not falling under the scope of the invention are prodrugs of the compounds according to the present application. The "prodrug" refers to a derivative that is converted into a compound according to the present disclosure by a reaction with enzymes, gastric acid, and the like in a living body under physiological conditions, for example, through oxidation, reduction, hydrolysis, and the like catalyzed by enzymes. Thus, some derivatives of the compounds according to the present application may have little or no pharmacological activity on their own, and can be converted into compounds according to the present application with the desired activity when administered to the body or on the body of a patient.

Disclosed but not falling under the scope of the invention are metabolites of the compounds according to the present application. The "metabolite" refers to all molecules derived from any compound according to the present disclosure in a cell or organism, preferably a human.

As used herein, the term "substituted" means that one or more (preferably 1 to 5, more preferably 1 to 3) hydrogen atoms in a group are independently replaced by a corresponding number of substituents.

As used herein, the term "independently" means that when the number of substituents is more than one, these substituents may be the same or different.

As used herein, the term "optional" or "optionally" means that the event described therein may or may not occur. For example, an "optionally substituted" group means that the group may be unsubstituted or substituted.

The term "halogen" or "halo" refers to -F, -Cl, -Br, or -I.

As used herein, the term "alkyl" refers to saturated aliphatic hydrocarbons, including straight and branched chains. In some embodiments, the alkyl group has 1-8, or 1-6, or 1-4, or 1-3 carbon atoms. For example, the term "$C_{1-8}$ alkyl" refers to a straight or branched chain group of atoms having 1-8 carbon atoms. The term "$C_{1-8}$ alkyl" includes the terms "$C_{1-6}$ alkyl", "$C_1$-$_3$ alkyl" and "$C_1$-$_4$ alkyl" in its definition. Examples of alkyl groups include, but are not limited to, methyl, ethyl, propyl, isopropyl, butyl, isobutyl, sec-butyl, tert-butyl, pentyl, 2-pentyl, 3-pentyl, isopentyl, neopentyl, (R)-2-methylbutyl, (S)-2-methylbutyl, 3-methylbutyl, 2,3-dimethylpropyl, 2,3-dimethylbutyl, hexyl, and the like. The alkyl group may be optionally substituted with one or more (for example, 1 to 5) suitable substituent(s).

As used herein, the term "alkenyl" refers to an aliphatic hydrocarbon having at least one carbon-carbon double bond, including straight and branched chains having at least one carbon-carbon double bond. In some embodiments, alkenyl groups have 2-8 carbon atoms, 2-6 carbon atoms, 3-6 carbon atoms, or 2-4 carbon atoms. For example, the term "$C_{2-8}$ alkenyl" refers to a linear or branched unsaturated atomic group (having at least one carbon-carbon double bond) having 2-8 carbon atoms. The double bond may or may not be the point of attachment of another group. Alkenyl groups include, but are not limited to, vinyl, 1-propenyl, 2-propenyl, 2-methyl-2-propenyl, butenyl, pentenyl, 3-hexenyl, and the like. Alkenyl groups may be optionally substituted with one or more (for example, 1 to 5) suitable substituent(s). When the compound of formula (I) contains an alkenyl group, the alkenyl group may be present in the pure E form, the pure Z form, or any mixture thereof.

As used herein, the term "alkynyl" refers to an aliphatic hydrocarbon having at least one carbon-carbon triple bond, including straight and branched chains having at least one carbon-carbon triple bond. In some embodiments, an alkynyl group has 2-8 carbon atoms, 2-6 carbon atoms, 3-6 carbon atoms, or 2-4 carbon atoms. For example, the term "$C_{2-8}$ alkynyl" refers to a linear or branched unsaturated atomic group (having at least one carbon-carbon triple bond) having 2-8 carbon atoms. The triple bond may or may not be the point of attachment of another group. Alkynyl groups include, but are not limited to, ethynyl, 1-propynyl, 2-propynyl, 2-methyl-2-propynyl, butynyl, pentynyl, 3-hexynyl, and the like. The alkynyl group may be optionally substituted with one or more (for example, 1 to 5) suitable substituent(s).

As used herein, the term "$C_{3-8}$ alicyclic group" refers to an alicyclic group having 3-8 ring-forming carbon atoms. The term "$C_{3-7}$ alicyclic group" refers to an alicyclic group having 3-7 ring-forming carbon atoms. The term "$C_{3-6}$ alicyclic group" refers to an alicyclic group having 3-6 ring-forming carbon atoms. The alicyclic group may be a monocyclic ring. The definition of alicyclic group also includes an unsaturated non-aromatic alicyclic group. Examples of alicyclic group are, for example, cyclopropyl, cyclobutyl, cyclopentyl, cyclohexyl, cyclohexenyl, cycloheptyl, cyclooctyl, cyclohexadienyl, cyclopentenyl, cycloheptenyl and cyclooctenyl. The alicyclic group may be optionally substituted with one or more suitable substituent(s).

As used herein, the term "$C_{6-12}$ bicyclic alicyclic group" is an alicyclic group containing two rings with 6-12 ring-forming carbon atoms. The bicyclic alicyclic group may be fused or may include a bridged bicyclic alicyclic group system.

As used herein, the term "$C_{8-15}$ membered tricyclic alicyclic group" is an alicyclic group containing three rings with 8-15 ring-forming carbon atoms. The tricyclic alicyclic group may be fused or bridged.

As used herein, the term "n-membered heteroalicyclic group" refers to an alicyclic group having m ring-forming carbon atoms and (n-m) ring-forming heteroatoms, the heteroatoms being selected from O, S and N. For example, the term "4-8-membered heteroalicyclic group" refers to a heteroalicyclic group substituent containing a total of 4 to 8 ring atoms, at least one of which is a heteroatom; the term "4-6-membered heteroalicyclic group" refers to a heteroalicyclic group substituent containing a total of 4 to 6 ring atoms, at least one of which is a heteroatom; and the term "3-10-membered heteroalicyclic group" refers to a heteroalicyclic group substituent containing a total of 3 to 10 ring atoms, at least one of which is a heteroatom. The term "n-membered bicyclic heteroalicyclic group" refers to a bicyclic heteroalicyclic group having m ring-forming carbon atoms and (n-m) ring-forming heteroatoms, the heteroatoms being selected from O, S and N. Examples of heteroalicyclic group includes, but not limited to, azetidinyl, thietidinyl, dihydrofuranyl, dihydrothiophenyl, tetrahydrothienyl, tetrahydrofuranyl, tetrahydrotriazinyl, tetrahydropyrazolyl, tetrahydrooxazinyl, tetrahydropyrimidinyl, octahydrobenzofuranyl, octahydrobenzimidazolyl, octahydrobenzothiazolyl, imidazolidinyl, pyrrolidinyl, piperidinyl, piperazinyl, oxazolidinyl, thiazolidinyl, pyrazolidinyl, thiomorpholinyl, tetrahydropyrimidinyl tetrahydrothiopyranyl, tetrahydrothiazinyl, tetrahydrothiadiazinyl, tetrahydrooxazolyl, morpholinyl, oxetanyl, tetrahydrodiazinyl, oxazinyl, oxathiadiazinyl, quinuclidinyl, benzodipyranyl (chromanyl), isobenzodipyranyl (isochromanyl), dihydrobenzodioxinyl, benzodioxolyl, benzoxazinyl, dihydroindolyl, dihydrobenzofuranyl, tetrahydroquinolinyl, isochromanyl, dihydro-1H-isoindolyl, 2-azabicyclo[2.2.1]heptanoyl, 3-azabicyclo[3.1.0]hexyl, 3-azabicyclo[4.1.0]heptyl, oxacycloheptyl, thiacycloheptyl, azacycloheptyl, and the like. The heteroalicyclic group may be optionally substituted with one or more suitable substituent(s).

As used herein, the term "$C_{5-8}$ aryl" refers to an aryl group having an aromatic ring containing 5-8 carbon atoms, for example phenyl.

As used herein, the term "n-membered heteroaryl" refers to a heteroaryl group having m aromatic ring-forming carbon atoms and (n-m) aromatic ring-forming heteroatoms, the heteroatoms being selected from O, S and N. For example, 5-7 membered heteroaryl includes but not limited to furanyl, thienyl, pyrrolyl, thiazolyl, pyrazolyl, imidazolyl, pyridinyl, pyranyl, pyridazinyl, pyrimidinyl, pyrazinyl. The heteroaryl may be optionally substituted with one or more suitable substituent(s).

As used herein, the term "$C_{7-11}$ bicycloaryl" refers to a bicycloaryl group having 7-11 carbon atoms, such as naphthalene, indene and the like. The bicycloaryl may be optionally substituted with one or more suitable substituent(s).

As used herein, the term "n-membered bicycloheteroaryl" refers to a bicycloheteroaryl group having m carbon atoms forming an aromatic bicyclic ring and (n-m) heteroatoms forming an aromatic bicyclic ring, and the heteroatoms are selected from O, S and N. For example, 7-11 membered bicycloheteroaryl includes, but not limited to, quinolinyl, isoquinolinyl, indolyl, purinyl, benzothiazolyl. The bicycloheteroaryl may be optionally substituted with one or more suitable substituent(s).

As used herein, the term "11-15 membered tricyclyl" includes but not limited to acridine and the like. The 11-15 membered tricyclyl may be optionally substituted with one or more suitable substituent(s).

As used herein, the term "haloalkyl" refers to an alkyl group having one or more halogen substituent(s) (up to perhaloalkyl, that is, each hydrogen atom of the alkyl is replaced by a halogen atom). For example, the term "$C_{1-6}$ haloalkyl" refers to a $C_{1-6}$ alkyl group with one or more halogen substituent(s) (up to perhaloalkyl, that is, each hydrogen atom of the alkyl group is replaced by a halogen atom). As another example, the term "$C_{1-4}$ haloalkyl" refers to a $C_{1-4}$ alkyl group with one or more halogen substituent(s) (up to perhaloalkyl, that is, each hydrogen atom of the alkyl group is replaced by a halogen atom); the term "$C_{1-3}$ haloalkyl" refers to a $C_{1-3}$ alkyl group with one or more halogen substituent(s) (up to perhaloalkyl, that is, each hydrogen atom of the alkyl group is replaced by a halogen atom); and the term "$C_{1-2}$ haloalkyl" refers to a $C_{1-2}$ alkyl group (i.e. methyl or ethyl) with one or more halogen substituent(s) (up to perhaloalkyl, that is, each hydrogen atom of the alkyl group is replaced by a halogen atom). As another example, the term "$C_1$ haloalkyl" refers to a methyl group with 1, 2, or 3 halogen substituent(s). Examples of haloalkyl groups include: $CF_3$, $C_2F_5$, $CHF_2$, $CH_2F$, $CH_2CF_3$, $CH_2Cl$, and the like.

As used herein, the term "alkoxy" refers to alkyl with a single bond attached to an oxygen atom. The point of attachment of the alkoxy group to a molecule is through the oxygen atom. Alkoxy can be described as alkyl-O-. The term "$C_{1-6}$ alkoxy" refers to a linear or branched alkoxy group containing 1 to 6 carbon atoms. The term "$C_{1-6}$ alkoxy" includes the term "$C_{1-3}$ alkoxy" in its definition. Alkoxy includes, but not limited to, methoxy, ethoxy, propoxy, isopropoxy, butoxy, hexoxy, and the like. The alkoxy group may be optionally substituted with one or more suitable substituent(s).

As used herein, the term "3-14-membered ring" refers to a saturated or unsaturated ring system with 3-14 ring-forming atoms. Similarly, the term "3-6-membered ring" refers to a saturated or unsaturated ring system with 3-6 ring-forming atoms, and term "3-8-membered ring" refers to a saturated or unsaturated ring system with 3-8 ring-forming atoms.

Herein, a numerical range relating to the number of substituents, the number of carbon atoms, or the number of ring members represents an enumeration of all integers in the range, and the range is only a simplified representation thereof. For example: "4 to 6-membered ring" means a 4, 5 or 6-membered ring; "5 to 7-membered ring" means a 5, 6 or 7-membered ring; "7 to 11-membered ring" means a 7, 8, 9, 10 or 11-membered ring; "4 to 8-membered ring" means a 4, 5, 6, 7 or 8-membered ring; "3 to 10-membered ring" means a 3, 4, 5, 6, 7, 8, 9 or 10-membered ring; "3 to 14-membered ring" means a 3, 4, 5, 6, 7, 8, 9, 10, 11, 12, 13 or 14-membered ring; "$C_{1-3}$" means 1 ($C_1$), 2 ($C_2$), or 3 ($C_3$) carbon atoms; "$C_{1-4}$" means 1 ($C_1$), 2 ($C_2$), 3 ($C_3$) or 4 ($C_4$) carbon atoms; "$C_{3-6}$" means 3 ($C_3$), 4 ($C_4$), 5 ($C_5$), or 6 ($C_6$) carbon atoms; "$C_{3-8}$" means 3 ($C_3$), 4 ($C_4$), 5 ($C_5$), 6 ($C_6$), 7 ($C_7$), or 8 ($C_8$) carbon atoms; "$C_{5-7}$" means 5 ($C_5$), 6 ($C_6$), or 7 ($C_7$) carbon atoms; "$C_{7-11}$" means 7 ($C_7$), 8 ($C_8$), 9 ($C_9$), 10 ($C_{10}$), or 11 ($C_{11}$) carbon atoms. Thus, a numerical range associated with the number of substituents, the number of carbon atoms, or the number of ring members also encompasses any one of its subranges, and each subrange is also considered to be disclosed herein.

In formula (I) as described above, $R^1$ is selected from H, -CN, $C_{1-4}$ alkyl, $C_{1-4}$ haloalkyl, $C_{1-4}$ alkoxy, and halogen.

In some embodiments, $R^1$ is H.

In some embodiments, $R^1$ is halogen, e.g., $R^1$ is selected from F, Cl, Br, and I.

In some embodiments, $R^1$ is $C_{1-4}$ alkyl or $C_{1-4}$ haloalkyl, optionally e.g., methyl, ethyl, propyl, isopropyl, n-butyl and isobutyl optionally substituted with one or more halogen atoms (e.g., fluorine, chlorine, bromine, iodine).

In some embodiments, $R^1$ is $C_{1-4}$ alkoxy, e.g., methoxy, ethoxy, propoxy, isopropoxy, butoxy and isobutoxy.

In some embodiments, $R^1$ is CN.

It should be understood that any of the above embodiments of $R^1$ may be combined with any of the embodiments of $R^2$, $R^3$, $R^5$, $R^6$, X, L and n as described above and below in arbitrary combination.

In formula (I) as described above, $R^2$ is selected from H, -CN, $C_{1-4}$ alkyl, $C_{1-4}$ haloalkyl, $C_{1-4}$ alkoxy, and halogen.

In some embodiments, $R^2$ is H.

In some embodiments, $R^2$ is halogen, e.g., $R^2$ is selected from F, Cl, Br, and I.

In some embodiments, $R^2$ is $C_{1-4}$ alkyl or $C_{1-4}$ haloalkyl, optionally e.g., methyl, ethyl, propyl, isopropyl, butyl and isobutyl optionally substituted with one or more halogen atoms (e.g., fluorine, chlorine, bromine, iodine).

In some embodiments, $R^2$ is a $C_{1-4}$ alkoxy, e.g., methoxy, ethoxy, propoxy, isopropoxy, butoxyl and isobutoxy.

In some embodiments, $R^2$ is -CN.

It should be understood that any of the above embodiments of $R^2$ may be combined with any of the embodiments of $R^1$, $R^3$, $R^5$, $R^6$, X, L and n as described above and below in arbitrary combination.

In some embodiments, $R^1$ and $R^2$ may be the same. For example, $R^1$ and $R^2$ both are halogen, such as Cl or

F; further, for example, $R^1$ and $R^2$ both are H. In a preferred embodiment, $R^1$ and $R^2$ both are F. In a preferred embodiment, $R^1$ and $R^2$ both are H.

In other embodiments, $R^1$ and $R^2$ may be different.

In formula (I) as described above, $R^3$ is selected from H, $C_{1-4}$ alkyl, $C_{1-4}$ haloalkyl, $C_{1-4}$ alkoxy, $C_{3-6}$ alicyclic group, and 4-6 membered heteroalicyclic group.

In some embodiments, $R^3$ is H.

In some embodiments, $R^3$ is $C_{1-3}$ alkyl or $C_{1-3}$ haloalkyl, e.g., $R^3$ is selected from methyl, ethyl, propyl, and isopropyl optionally substituted with one or more halogen atoms (e.g., fluorine, chlorine, bromine, iodine).

In some embodiments, $R^3$ is a $C_{1-3}$ alkoxy, e.g., $R^3$ is selected from methoxy, ethoxy, propoxy, and isopropoxy.

In some embodiments, $R^3$ is $C_{3-6}$ alicyclic group, e.g., $R^3$ is selected from cyclopropyl, cyclobutyl, cyclopentyl, cyclohexyl, cyclohexenyl, cyclohexadienyl, cyclopentenyl, and the like.

In some embodiments, $R^3$ is 4-6 membered heteroalicyclic group in which the hetero atom may be selected from O, S and N. For example, $R^3$ is selected from oxetanyl, thietanyl, azetidinyl, tetrahydrofuranyl, tetrahydrothienyl, pyrrolidinyl, tetrahydropyranyl, tetrahydrothiopyranyl, piperidinyl, morpholinyl, and piperazinyl.

In some embodiments, $R^3$ is methyl.

It should be understood that any of the above embodiments of $R^3$ may be combined with any of the embodiments of $R^1$, $R^2$, $R^5$, $R^6$, X, L and n as described above and below in arbitrary combination.

There may be 1, 2, or 3 $R^6$(s) present in formula (I) as described above where each $R^6$ is each independently selected from H, halogen, -CN, -OH, $-NO_2$, $-NR^7R^8$, $C_{1-4}$ alkyl, $C_{1-4}$ haloalkyl, $C_{1-4}$ alkoxy, $C_{3-6}$ alicyclic group, and 4-6 membered heteroalicyclic group with the hetero atom selected from O, S and N in which $R^7$ and $R^8$ are each independently selected from: H, $C_{1-6}$ alkyl, $C_{1-4}$ haloalkyl, $C_{1-4}$ alkoxy, or $R^7$, $R^8$ and N atom attached thereto together form a 3-6-membered ring, e.g. those rings at which $R^7$ and $R^8$ both are $(CH_2)_n$ (n=1, 2, 3, 4, 5, etc.) or $R^7$, $R^8$ and the N atom(s) attached thereto together form a 5- or 6-membered N-containing aromatic ring.

In some preferred embodiments, $R^6$ is H.

In some embodiments, $R^6$ is halogen, e.g., $R^6$ is selected from F, Cl, Br, and I.

In some embodiments, $R^6$ is -CN, -OH or $-NO_2$.

In some embodiments, $R^6$ is $C_{1-3}$ alkyl or $C_{1-3}$ haloalkyl, e.g., $R^6$ is selected from methyl, ethyl, propyl, and isopropyl optionally substituted with one or more halogen atoms (e.g., fluorine, chlorine, bromine, iodine).

In some embodiments, $R^6$ is $C_{1-3}$ alkoxy, e.g., $R^6$ is selected from methoxy, ethoxy, propoxy, and isopropoxy.

In some embodiments, $R^6$ is $C_{3-6}$ alicyclic group, e.g., $R^6$ is selected from cyclopropyl, cyclobutyl, cyclopentyl, cyclohexyl, and cyclohexenyl.

In some embodiments, $R^6$ is 4-6 membered heteroalicyclic group in which the hetero atom may be selected from O, S and N, e.g., $R^6$ is selected from oxetanyl, thietanyl, azetidinyl, tetrahydrofuranyl, tetrahydrothienyl, pyrrolidinyl, tetrahydropyranyl, tetrahydrothiopyranyl, piperidinyl, morpholinyl, and piperazinyl.

In some embodiments, $R^6$ is $-NR^7R^8$, wherein $R^7$ and $R^8$ are each independently selected from: H, $C_{1-3}$ alkyl (e.g., methyl, ethyl, propyl, isopropyl, etc.), $C_{1-3}$ haloalkyl (e.g., methyl, ethyl, propyl, isopropyl substituted with one or more halogen atoms selected from fluorine, chlorine, bromine, iodine), $C_{1-3}$ alkoxy (e.g., methoxy, ethoxy, propoxy, isopropoxy), or $R^7$, $R^8$ and N atom attached thereto together form a 3-6-membered ring, e.g. those rings at which $R^7$ and $R^8$ both are $(CH_2)_n$ (n=1, 2, 3, 4, 5, etc.) or $R^7$, $R^8$ and the N atom(s) attached thereto together form a 5- or 6-membered N-containing aromatic ring, such as pyrrole, pyridine, pyrimidine, imidazole, pyrazole, pyrrolidine, hexahydropyridine, etc.

In some embodiments, $R^6$ is dimethylamino, diethylamino, methylethylamino.

In some preferred embodiments, one $R^6$ is present.

It should be understood that any of the above embodiments of $R^6$ may be combined with any of the embodiments of $R^1$, $R^2$, $R^3$, $R^5$, X, L and n as described above and below in arbitrary combination.

In formula (I) as described above, X is a bond, O, S, or $(NR^4)$, wherein $R^4$ is selected from: H, $C_{1-4}$ alkyl, $C_{1-4}$ haloalkyl, $C_{1-4}$ alkoxy, $C_{3-6}$ alicyclic group, and 4-6 membered heteroalicyclic group in which the hetero atom may be selected from O, S and N.

In some embodiments, X is a bond (i.e., X is not present, and the groups on either side of X are directly connected).

In some embodiments, X is -O-.

In some embodiments, X is -S-.

In some embodiments, X is imino, i.e., -(NH)-.

In some embodiments, X is $-(N(CH_3))-$.

In some embodiments, X is selected from a bond, -O- and -(NH)-.

It should be understood that any of the above embodiments of X can be combined with any of the embodiments of $R^1$, $R^2$, $R^3$, $R^5$, $R^6$, L and n as described above and below in arbitrary combination.

In formula (I) as described above, n is 1, 2, or 3.

In some embodiments, n is 1.

In some embodiments, n is 2.

In some embodiments, n is 3.

It should be understood that any of the above embodiments of n may be combined with any of the embodiments of $R^1$, $R^2$, $R^3$, $R^5$, $R^6$, L, and X as described above and below in arbitrary combination.

In formula (I) as described above, L is (C=O), (O=S=O), $CR^aR^b$ or a bond in which $R^a$ and $R^b$ are each indepdnently selected from H, $C_{1-4}$ alkyl, $C_{1-4}$ haloalkyl, $C_{1-4}$ alkoxy, $C_{3-6}$ alicyclic group, and 4-6 membered heteroalicyclic group in which the hetero atom may be selected from O, S and N, or $R^a$, $R^b$, and the carbon atom(s) attached thereto together form a 3-6-membered ring, for example a 3-6-membered saturated alicyclic ring at which $R^a$ and $R^b$ both are $(CH_2)_n$ (n=1, 2, 3, 4, 5, etc.) or $R^a$, $R^b$ and the carbon atom(s) attached thereto together form a 3-6-membered unsaturated alicyclic ringe or a 6-membered aromatic ring,

In some embodiments, L is -(C=O)-.

In some embodiments, L is -(O=S=O)-.

In some embodiments, L is a bond, (i.e., X is not present, and $R^5$ and N are directly connected).

In some embodiments, L is -($CR^aR^b$)-, wherein $R^a$ and $R^b$ are each independently selected from: H, $C_{1-4}$ alkyl (e.g., methyl, ethyl, propyl, isopropyl, n-butyl, isobutyl, tert-butyl, etc.), $C_{1-4}$ haloalkyl (e.g., methyl, ethyl, propyl, isopropyl, n-butyl, isobutyl, tert-butyl, etc., substituted with one or more halogen atoms selected from fluorine, chlorine, bromine, and iodine), $C_{1-4}$ alkoxy (e.g., methoxy, ethoxy, propoxy, isopropoxy, n-butoxy, isobutoxy, tert-butoxy, etc.), $C_{3-6}$ alicyclic (e.g. cyclopropyl, cyclobutyl, cyclopentyl, cyclohexyl, cyclohexenyl, cyclohexadienyl, cyclopentenyl, etc.), and 4-6-membered heteroalicyclic ring (e.g. oxetanyl, thietidinyl,yangza azetidinyl, tetrahydrofuranyl, tetrahydrothiophenyl, pyrrolidyl, tetrahydropyranyl, tetrahydothiopyranyl, piperidinyl, morpholinyl, piperazinyl, and the like), or $R^a$, $R^b$, and the carbon atom(s) attached thereto together form a 3- to 6-membered ring (preferably a 3- to 6-membered alicyclic ring). For example, L may be $-CH_2-$, $-C(CH_3)_2-$, $-CH(CH_3)-$, cyclopropylidene, cyclobutylidene, cyclopentylidene, cyclohexylidene, phenylidene, and the like.

It should be understood that any of the above embodiments of L can be combined with any of the embodiments of $R^1$, $R^2$, $R^3$, $R^5$, $R^6$, n and X as described above and below in arbitrary combiantion.

In formula (I) as described above, $R^5$ can be any substituent commonly used in organic chemistry, which is not particularly limited.

In some embodiments, $R^5$ is $C_{1-6}$ alkyl. For example, $R^5$ is selected from methyl, ethyl, propyl, isopropyl, butyl, isobutyl, sec-butyl, tert-butyl, pentyl, 2-pentyl, 3-pentyl, isopentyl, neopentyl, (R)-2-methylbutyl, (S)-2-methylbutyl, 3-methylbutyl, 2,3-dimethylpropyl, 2,3-dimethylbutyl, hexyl.

In some embodiments, $R^5$ is $C_{3-7}$ alicyclic group. For example, $R^5$ is selected from cyclopropyl, cyclobutyl, cyclopentyl, cyclohexyl, cyclohexenyl, cycloheptyl, cyclohexadienyl, cyclopentenyl, cycloheptenyl.

In some embodiments, $R^5$ is 3-10 membered heteroalicyclic group. For example, $R^5$ is selected from azetidinyl, thietidinyl, dihydrofuranyl, dihydrothienyl, tetrahydrothienyl, tetrahydrofuranyl, tetrahydrotriazinyl, tetrahydropyrazolyl, tetrahydrooxazinyl, tetrahydropyrimidinyl, octahydrobenzofuranyl, octahydrobenzimidazolyl, octahydrobenzothiazolyl, imidazolidinyl, pyrrolidinyl, piperidinyl, piperazinyl, oxazolidinyl, thiazolidinyl, pyrazolidinyl, thiomorpholinyl, tetrahydropyranyl, tetrahydrothiopyranyl, tetrahydrothiazinyl, tetrahydrothiadiazinyl, tetrahydrooxazolyl, morpholinyl, oxetanyl, tetrahydrodiazinyl, oxazinyl, oxathiadiazinyl, quinuclidinyl, benzodipyranyl, isobenzodipyranyl, dihydrobenzodioxinyl, benzodioxopentenyl, benzoxazinyl, dihydroindolyl, dihydrobenzofuranyl, tetrahydroquinolinyl, isochromanyl, dihydro-1H-isoindolyl, oxepanyl, thiepanyl, and azepanyl.

In some embodiments, $R^5$ is H, halogen, -OH, $-NO_2$, -CN, $-SF_5$, or -SH.

In some embodiments, $R^5$ is selected from -S-$C_{1-4}$ alkyl, $C_{1-6}$ alkyl, $C_{1-6}$ haloalkyl, $C_{1-6}$ alkoxy, $C_{1-6}$ haloalkoxy, $C_{2-8}$ alkenyl, $C_{2-8}$ alkynyl.

In some embodiments, $R^5$ is selected from $C_{5-8}$ aryl (e.g., phenyl), 5-10-membered heteroaryl (e.g., furanyl, thienyl, pyrrolyl, thiazolyl, pyrazolyl, imidazolyl, pyridinyl, pyranyl, pyridazinyl, pyrimidinyl, pyrazinyl, etc.), -$C_{1-4}$ alkyl-($C_{5-8}$ aryl), -$C_{1-4}$ alkyl- (5-10-membered heteroaryl).

In some embodiments, $R^5$ is selected from $-N(R^{10})(R^{11})$, $-N(R^{10})(C(=O)R^{11})$, - $N(R^{10})(C(=O)-OR^{11})$, $-N(R^{12})(C(=O)-N(R^{10})(R^{11}))$, $-C(=O)-N(R^{10})(R^{11})$, -C(=O)-R $^{12}$, - $C(=O)-OR^{12}$, $-OC(=O)R^{12}$, $-N(R^{10})(S(=O)_2R^{11})$, $-S(=O)_2-N(R^{10})(R^{11})$, $-SR^{12}$, and $-OR^{12}$, wherein $R^{10}$, $R^{11}$, and $R^{12}$ at each occurrence are each independently selected from: H, $C_{1-6}$ alkyl, $C_{1-6}$ haloalkyl, $C_{3-7}$ alicyclic group, 3-10-membered heteroalicyclic group, $C_{5-8}$ aryl, 5-7-membered heteroaryl, $C_{7-11}$ bicyclic aryl, 7-11-membered bicyclic heteroaryl, -$C_{1-4}$ alkyl-($C_{3-7}$ alicyclic group), -$C_{1-4}$ alkyl- (3-10-membered heteroalicyclic group), -$C_{1-4}$ alkyl-($C_{6-12}$ bicyclic alicyclic group), -$C_{1-4}$ alkyl- (6-12-membered bicyclic heteroalicyclic group), -$C_{1-4}$ alkyl-($C_{8-15}$ tricyclic alicyclic group), -$C_{1-4}$ alkyl-(8-15-membered tricyclic heteroalicyclic group), -$C_{1-4}$ alkyl-($C_{5-8}$ aryl), and -$C_{1-4}$ alkyl-(5-10-membered heteroaryl), wherein each substitute within the group is optionally substituted with 0, 1, 2, 3, or 4 substituents each independently selected from the following groups: halogen, -OH, $-NH_2$, $-NH(CH_3)$, $-N(CH_3)_2$, -CN, $-NO_2$, $-SF_5$, -SH, -S-$C_{1-4}$ alkyl, oxo, $C_{1-4}$ alkyl, $C_{2-6}$ alkenyl, $C_{2-6}$ alkynyl, $C_{3-7}$ alicyclic group, 3-10-membered heteroalicyclic group, $C_{5-8}$ aryl, 5-7-membered heteroaryl, $C_{7-11}$ bicyclic aryl, 7-11-membered bicyclic heteroaryl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, $-C(=O)-NH_2$, -C(=O)-N($C_{1-4}$

alkyl)$_2$, $C_{1-4}$ haloalkyl, $C_{1-4}$ alkoxy, and $C_{1-4}$ haloalkoxy; or $R^{10}$, $R^{11}$, and the atom(s) attached thereto together form a 3-14-membered ring (for example those rings at which $R^{10}$ and $R^{11}$ both are $(CH_2)_n$ (n=1, 2, 3, 4, 5, 6, 7, 8 etc.) or $R^{10}$, $R^{11}$ and the atom(s) attached thereto together form a 5-14-membered aromatic ring).

Any exemplary groups enumerated above for $R^5$ are to be understood as being optionally substituted; i.e., where appropriate, any of the groups given above for $R^5$ may be optionally substituted with 0, 1, 2, 3 or 4 $R^{5a}$(s), and $R^{5a}$ is independently selected from halogen, -OH, $-NO_2$, -CN, $-SF_5$, -SH, -S-$C_{1-4}$ alkyl, oxo, $C_{1-4}$ alkyl, $C_{1-4}$ haloalkyl, $C_{1-4}$ alkoxy, $C_{1-4}$ haloalkoxy, $C_{2-8}$ alkenyl, $C_{2-8}$ alkynyl, $C_{3-7}$ alicyclic group, 3-10-membered heteroalicyclic group, $C_{5-8}$ aryl, 5-7-membered heteroaryl, -N($R^{13}$)($R^{14}$), -N($R^{13}$)(C(=O)$R^{14}$), -N($R^{13}$)(C(=O)-O$R^{14}$), -N($R^{15}$)(C(=O)-N($R^{13}$)($R^{14}$)), -C(=O)-N($R^{13}$)($R^{14}$), -C(=O)-$R^{15}$, - C(=O)-O$R^{15}$, -OC(=O)$R^{15}$, -N($R^{13}$)(S(=O)$_2R^{14}$), -S(=O)$_2$-N($R^{13}$)($R^{14}$), -S$R^{15}$, and -O$R^{15}$, where $R^{13}$, $R^{14}$, and $R^{15}$ at each occurrence are each independently selected from: H, $C_{1-6}$ alkyl, $C_{1-6}$ haloalkyl, $C_{3-7}$ alicyclic group, 3-10-membered heteroalicyclic group, $C_{5-8}$ aryl, 5-7-membered heteroaryl, $C_{7-11}$ bicyclic aryl, 7-11-membered bicyclic heteroaryl, -$C_{1-4}$ alkyl- ($C_{3-7}$ alicyclic group), -$C_{1-4}$ alkyl- (3-10-membered heteroalicylic group), -$C_{1-4}$ alkyl-($C_{6-12}$ bicyclic alicyclic group), -$C_{1-4}$ alkyl- (6-12-membered bicyclic heteroalicyclic group), -$C_{1-4}$ alkyl-($C_{8-15}$ tricyclic alicylic group), -$C_{1-4}$ alkyl- (8-15-membered tricyclic heteroalicyclic group), -$C_{1-4}$ alkyl-($C_{5-8}$ aryl), and -$C_{1-4}$ alkyl- (5-10-membered heteroaryl), wherein each substituent within the group is optionally substituted with 0, 1, 2, 3 or 4 substituents each independently selected from the group consisting of: halogen, -OH, $-NH_2$, $-NH(CH_3)$, - $N(CH_3)_2$, -CN, $-NO_2$, $-SF_5$, -SH, -S-$C_{1-4}$ alkyl, oxo, $C_{1-4}$ alkyl, $C_{2-6}$ alkenyl, $C_{2-6}$ alkynyl, $C_{3-7}$ alicyclic group, 3-10-membered alicyclic group, $C_{5-8}$ aryl, 5-7-membered heteroaryl, $C_{7-11}$ bicyclic aryl, 7-11-membered bicyclic heteroaryl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, -C(=O)-$NH_2$, -C(=O)-N($C_{1-4}$ alkyl)$_2$, $C_{1-4}$ haloalkyl, $C_{1-4}$ alkoxy, and $C_{1-4}$ haloalkoxy; or $R^{13}$, $R^{14}$, and the atom(s) attached thereto together form a 3-14-membered ring (for example those rings at which $R^{13}$ and $R^{14}$ both are $(CH_2)_n$ (n=1, 2, 3, 4, 5, 6, 7, 8 etc.) or $R^{13}$, $R^{14}$ and the atom(s) attached thereto together form a 5-14-membered aromatic ring).

In some preferred embodiments, $R^5$ is selected from methyl, ethyl, propyl, isopropyl, and cyclobutyl groups. The methyl, ethyl, propyl, isopropyl or cyclobutyl group may optionally be substituted with 0, 1 or 2 substituents each independently selected from a group consisting of: methyl, ethyl, cyclopentyl, cyclopropyl, -CN, -OH, morpholinyl, piperidinyl, and these substituents may further optionally be substituted with 0, 1 or 2 substituents each independently selected from a group consisting of: methyl, ethyl, cyclopentyl, cyclopropyl, -CN, and -OH. For example, in some preferred embodiments, $R^5$ is selected from (1-hydroxycyclopropyl)ethyl, 3-hydroxycyclobutyl, 2-cyanoethyl, 2-hydroxyethyl, 2-cyano-1-cyclopentyl ethyl, 1-cyanopropane, 2-morpholinoethyl, ethyl, and (1-methylpiperidin-4-yl)methyl.

In some preferred embodiments, $R^5$ is selected from halogen, such as F.

In some preferred embodiments, $R^5$ is selected from piperazinyl, morpholinyl, pyrrolidinyl, piperidinyl and azetidinyl, the piperazinyl, pyrrolidinyl, piperidinyl and azetidinyl being optionally substituted with 0, 1 or 2 substituents each independently selected from a group consisting of: methyl, ethyl, cyclopentyl, cyclopropyl, -CN, and -OH. In some preferred embodiments, $R^5$ is selected from methyl, ethyl, propyl, isopropyl, and cyclobutyl groups, in which said methyl, ethyl, propyl, isopropyl or cyclobutyl groups may optionally be substituted with 0, 1 or 2 substituents each independently selected from a group consisting of: methyl, ethyl, cyclopentyl, cyclopropyl, -CN, -OH, morpholinyl, and piperidinyl, and these substituents are further optionally substituted with 0, 1 or 2 substituents each independently selected from a group consisting of: methyl, ethyl, cyclopentyl, cyclopropyl, -CN, and -OH.

In some preferred embodiments, $R^5$ is selected from piperazinyl, morpholinyl, pyrrolidinyl, piperidinyl, and azetidinyl. For example, in some preferred embodiments, $R^5$ is selected from 3,5-dimethylpiperazinyl, morpholinyl, 3-hydroxypyrrolidinyl, 4-methylpiperazinyl, 4-ethylpiperazinyl, 4-hydroxypiperidinyl, 1-methylpiperidinyl, 1-ethylpiperidin-4-yl, 1-methylazetidin-3-yl.

In some preferred embodiments, $R^5$ is selected from H, methyl, ethyl, n-propyl, isopropyl, butyl, methoxy, ethoxy, hydroxy, cyclopropyl, cyclobutyl, cyclopentyl, cyclohexyl, morpholinomethyl, hydroxycyclobutyl, hydroxycyclohexyl, cyanoethoxy, cyanomethyl, pyridin-3-yl, 1-methyl-1H-pyrazol-4-yl, 1-methyl-1H-pyrazol-3-yl, 4-methylpiperazin-1-yl, 1-methylpiperidin-4-yl, morpholinyl, pyrrolidin-3-yl, 3-hydroxypyrrolidin-1-yl, and 3-cyanopyrrolidin-1-yl.

It should be understood that any of the above embodiments of $R^5$ may be combined with any of the embodiments of $R^1$, $R^2$, $R^3$, $R^6$, X, L and n as described above and below in arbitrary combination.

In some preferred embodiments, the present application also relates to compounds shown in formula (Ia):

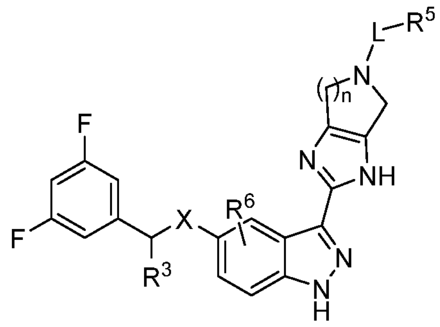

(Ia)

wherein X is -O- or -(NH)-; L, n, $R^3$, $R^5$, $R^6$ have the same definitions as well as preferred options as those in the compound of formula (I).

In some preferred embodiments, the present application also relates to compounds shown in formula (Ib):

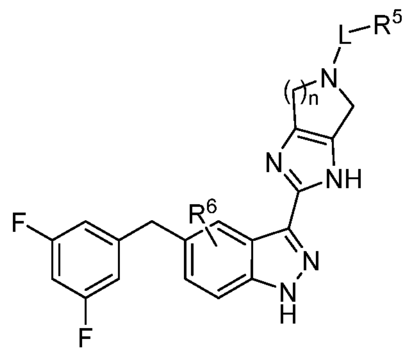

(Ib)

wherein L, n, $R^5$, $R^6$ have the same definitions as well as preferred options as those in the compound of formula (I)..

In some preferred embodiments, the present application also relates to compounds shown in formula (Ic):

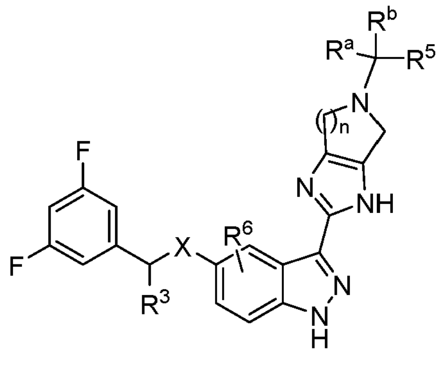

(Ic)

wherein X is -O- or -(NH)-; n, $R^3$, $R^5$, $R^6$, $R^a$ and $R^b$ have the same definitions as well as preferred options as those in the compound of formula (I).

In some preferred embodiments, the present application also relates to compounds shown in formula (IIa):

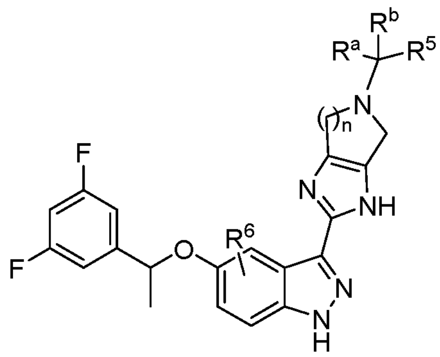

(IIa)

wherein n, $R^3$, $R^5$, $R^6$, $R^a$ and $R^b$ have the same definitions as well as preferred options as those in the compound of formula (I).

In some preferred embodiments, the present application also relates to compounds shown in formula (IIb):

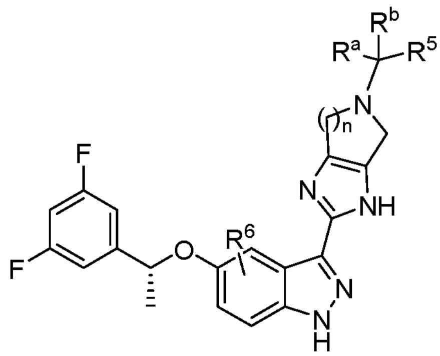

(IIb)

wherein n, $R^3$, $R^5$, $R^6$, $R^a$ and $R^b$ have the same definitions as well as preferred options as those in the compound of formula (I).

In some more preferred embodiments, the compounds of the present application are selected from the specific compounds shown in the examples of the present application.

The compounds according to the present application can be synthesized via conventional organic synthesis methods according to their specific structures by those skilled in the art. For example, compounds of formula (I) can be prepared by the method shown in synthetic route (I) or the synthetic route (II) below.

Synthetic route (I)

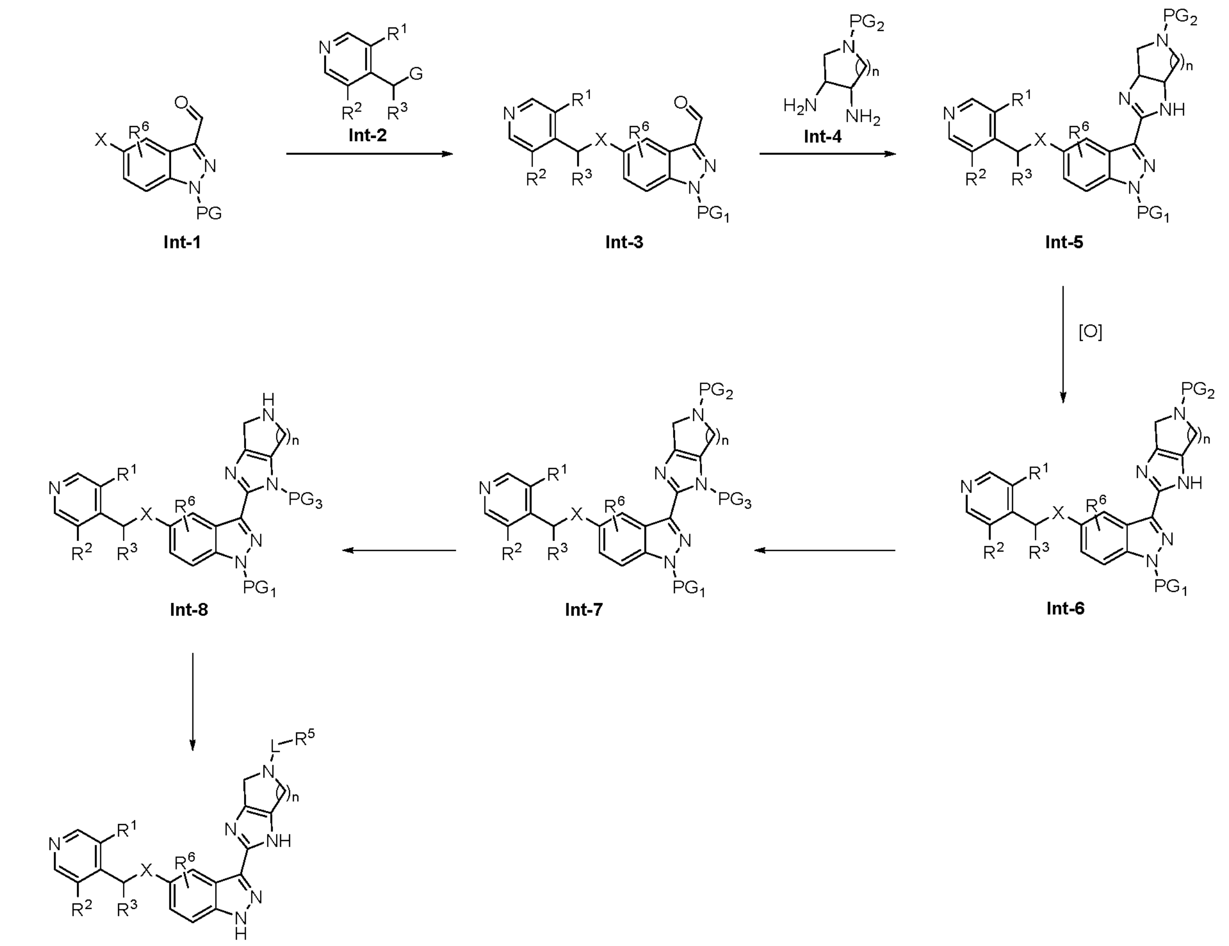


Int-1 Int-2 Int-3 Int-4 Int-5 Int-6 Int-7 Int-8

(I)

For example, the compound of formula (I) may be prepared by the method as shown in the above synthetic route (I). G in an intermediate Int-2 is selected from halogen, hydroxyl, methylsulfonyl (OMs), p-toluenesulfonyl (OTs), and the like. $PG_1$, $PG_2$, and $PG_3$ are an amino protecting group such as tetrahydropyran (THP), benzyl (Bn), p-methoxybenzyl (PMB), SEM, Boc and the like. When G is halogen, OMs, or OTs, the SN2 coupling reaction between intermediates Int-1 and Int-2 under a basic condition can produce an intermediate Int-3. When G is OH, the intermediate Int-3 can be obtained by the Mitsunobu reaction between Int-1 and Int-2. The condensation reaction between intermediate Int-3 and intermediate Int-4 produces a compound Int-5. Under oxidizing conditions (such as, but not limited to, Swern oxidation or IBX oxidation), the Int-5 is converted to an intermediate Int-6. Via a protecting group conversion, the intermediate Int-6 can be converted to an Int-8. Under typical reaction conditions for generating amide (such as, but not limited to, in the presence of DIPEA/HATU), or reaction conditions for generating sulfonamide, or reaction conditions for generating urea, or SN2 coupling reaction conditions, or reaction conditions for generating carbamate, the Int-8 can be reacted with a suitable starting material and then the protecting group is removed to give a target compound of formula (I).

Synthetic route (II)

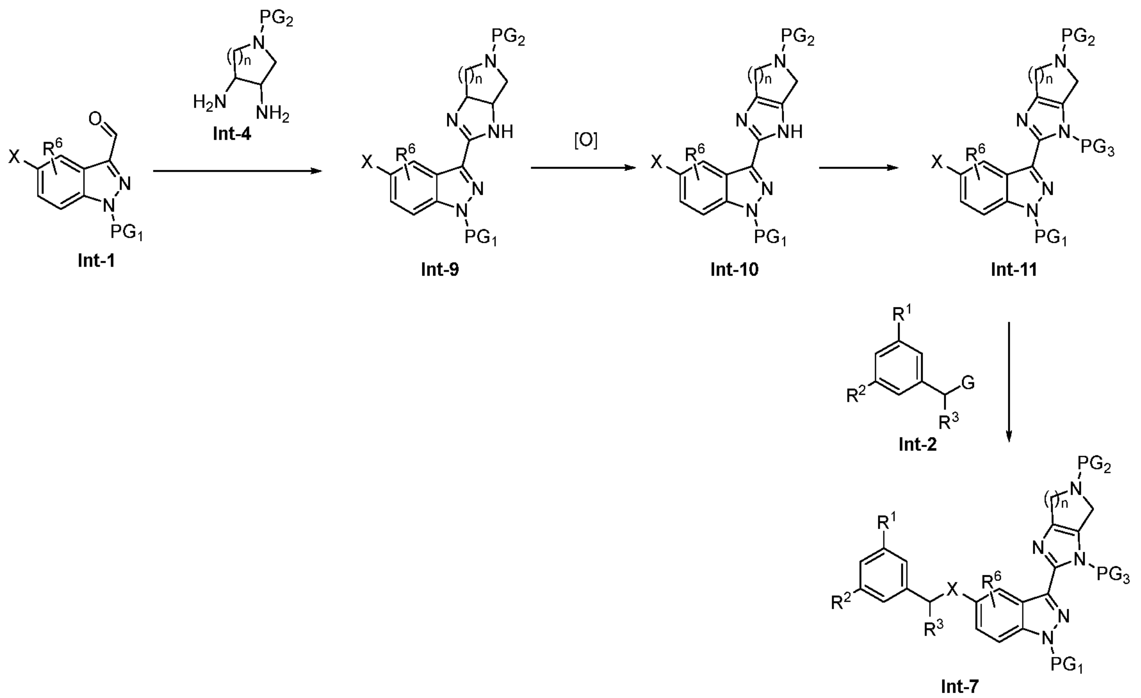


Alternatively, the compound Int-7 can be prepared by the method shown in synthetic route (II) above. The condensation reaction of intermediate Int-1 with intermediate Int-4 produces compound Int-9. Under oxidizing conditions (e.g., but not limited to, Swern oxidation or IBX oxidation), Int-9 is converted to intermediate Int-10. Upon conversion of the protecting group, intermediate Int-6 can be converted to Int-11. PG1, PG2, and PG3 are amino protecting groups, such as but not limited to tetrahydropyran (THP), benzyl (Bn), p-methoxybenzyl (PMB), SEM, Boc, and the like. G in the intermediate Int-2 is selected from halogen, hydroxyl, methylsulfonyl (OMs), p-toluenesulfonyl (OTs), and the like. When G is halogen, OMs, or OTs, the intermediate Int-7 can be generated by SN2 coupling reaction between intermediate Int-11 and Int-2 under alkaline conditions.When G is OH, the intermediate Int-7 can be obtained by Mitsunobu reaction between Int-11 and Int-2.

In addition, a person skilled in the art can refer to the synthetic routes of specific compounds shown in Examples of the present application and make appropriate adjustments to the raw materials and reaction conditions to obtain synthesis methods of other compounds.

The compounds of the present application have been shown to possess TRK kinase inhibitory activity that can effectively inhibit TRKA, TRKB and/or TRKC, and have comparable or better inhibitory activity or selectivity than that of Larotrectinib or Entrectinib. Thus, more options and possibilities for the development of broad-spectrum anticancer drugs, pain relievers, anti-inflammatory drugs and the like are provided.

In a second aspect, the present application provides a pharmaceutical composition comprising a compound according to the present application, or an isotopically labeled compound thereof, or an optical isomer thereof, a geometric isomer thereof, a tautomer thereof or a pharmaceutically acceptable salt thereof. as described above, and one or more pharmaceutically acceptable carriers, adjuvants, or excipients.

The pharmaceutical compositions according to the present application can be prepared in a manner well known in the pharmaceutical field and can be administered by a variety of routes, depending on whether topical or systemic treatment is desired and depending on the site to be treated. Administration can be topical (including ophthalmic and to mucous membranes, including intranasal, vaginal, and rectal delivery), pulmonary (e.g., by inhalation or by blowing in a powder or aerosol, including through aerosol dispensers; intratracheal, intranasal, epidermal, and transdermal), ocular, transoral, or parenteral. Methods for ocular delivery may include topical administration (eye drops), subconjunctival, periocular or intravitreal injection or introduction via a balloon catheter or ophthalmic insert surgically placed in the conjunctival sac. Parenteral administration includes intravenous, intra-arterial, subcutaneous, intraperitoneal, or intramuscular injection or infusion; or intracranial (e.g., intrathecal or intracerebroventricular) administration. Parenteral administration can be in the form of a single push dose, or can be, for example, via a continuous perfusion pump. Pharmaceutical compositions and formulations for topical administration may include transdermal patches, ointments, lotions, creams, gels, drops, suppositories, sprays, liquids, and powders.

If a solid carrier is used, the dosage may be tableted, or placed in a hard gelatin capsule in powder or pellet form, or in the form of a troche or lozenge. The solid carrier may contain conventional excipients such as binding agents,

fillers, tableting lubricants, disintegrants, wetting agents and the like. The tablet may, if desired, be film coated by conventional techniques. If a liquid carrier is employed, the dosage may be in the form of a syrup, emulsion, paste, soft gelatin capsule, sterile vehicle for injection, an aqueous or non-aqueous liquid suspension, or may be a dry product for reconstitution with water or other suitable vehicle before use. Liquid preparations may contain conventional additives such as suspending agents, emulsifying agents, wetting agents, non-aqueous vehicle (including edible oils), preservatives, as well as flavoring and/or coloring agents. For parenteral administration, a vehicle normally will comprise sterile water, at least in large part, although saline solutions, glucose solutions and like may be utilized. Injectable suspensions also may be used, in which case conventional suspending agents may be employed. Conventional preservatives, buffering agents and the like also may be added to the parenteral dosage forms. The medicament are prepared by conventional techniques appropriate to the desired preparation containing appropriate amounts of the active ingredient, that is, the compound according to the invention.

Medicament dosage suitable for parenteral injection may comprise physiologically acceptable sterile aqueous or nonaqueous solutions, dispersions, suspensions or emulsions, and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), suitable mixtures thereof, vegetable oils (such as olive oil), and injectable organic esters such as ethyl oleate.

The medicament may also contain excipients such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride, and the like. Prolonged absorption of the injectable pharmaceutical form can be brought about by the use of agents delaying absorption, for example, aluminum monostearate and gelatin.

Solid dosage forms for oral administration include capsules, tablets, pills, powders, and granules. In such solid dosage forms, the active compound is admixed with at least one inert customary excipient (or carrier) such as sodium citrate or dicalcium phosphate or (a) fillers or extenders, as for example, starches, lactose, sucrose, glucose, mannitol, and silicic acid; (b) binders, as for example, carboxymethylcellulose, alignates, gelatin, polyvinylpyrrolidone, sucrose, and acacia; (c) humectants, as for example, glycerol; (d) disintegrating agents, as for example, agar-agar, calcium carbonate, potato or tapioca starch, alginic acid, certain complex silicates, and sodium carbonate; (e) solution retarders, as for example paraffin; (f) absorption accelerators, as for example, quaternary ammonium compounds; (g) wetting agents, as for example, cetyl alcohol and glycerol monostearate; (h) adsorbents, as for example, kaolin and bentonite; and (i) lubricants, as for example, talc, calcium stearate, magnesium stearate, solid polyethylene glycols, sodium lauryl sulfate, or mixtures thereof.

Solid compositions of a similar type may also be employed as fillers in soft and hard-filled gelatin capsules using such excipients as lactose or milk sugar as well as high molecular weight polyethyleneglycols, and the like.

Solid dosage forms such as tablets, dragees, capsules, pills, and granules can be prepared with coatings and shells, such as enteric coatings and others well-known in the art. They may contain opacifying agents, and can also be of such composition that they release the active compound or compounds in a certain part of the intestinal tract in a delayed manner. Examples of embedding compositions which can be used are polymeric substances and waxes. The active compounds can also be in micro-encapsulated form, if appropriate, with one or more of the above-mentioned excipients.

Liquid dosage forms for oral administration include pharmaceutically acceptable emulsions, solutions, suspensions, syrups, and elixirs. In addition to the active compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil and sesame oil, glycerol, tetrahydrofurfuryl alcohol, polyethyleneglycols and fatty acid esters of sorbitan or mixtures of these substances, and the like.

Besides such inert diluents, the composition can also include adjuvants, such as wetting agents, emulsifying and suspending agents, sweetening, flavoring, and perfuming agents.

Suspensions, in addition to the active compounds, may contain suspending agents, as for example, ethoxylatedisostearyl alcohols, polyoxyethylene sorbitol and sorbitan esters, microcrystalline cellulose, meta-aluminum hydroxide, bentonite, agar-agar and tragacanth, or mixtures of these substances, and the like.

Dosage forms for topical administration of a compound of the invention include paste, powders, sprays, and inhalants. The active component is admixed under sterile conditions with a physiologically acceptable carrier and any preservatives, buffers, or propellants as may be required.

The amount of the compound according to the present application in the pharmaceutical composition and dosage form can be appropriately determined by those skilled in the art as needed. For example, the compound according to the present application can be present in the pharmaceutical composition or dosage form in a therapeutically effective amount.

In a third aspect, the present application provides use of the compounds according to the present application, or an isotopically labeled compound thereof, or an optical isomer thereof, a geometric isomer thereof, a tautomer thereof or a pharmaceutically acceptable salt thereof, as described above, or the pharmaceutical composition as described above, in the preparation of drugs for the treatment of diseases or conditions associated with TRK kinases or NTRK genes.

The present application also discloses a method of treating diseases or conditions associated with TRK kinases or NTRK genes, said method comprising administering to a patient in need thereof a therapeutically effective amount of a compound according to the present application, or an isotopically labeled compound thereof, or an optical isomer thereof, a geometric isomer thereof, a tautomer thereof or a pharmaceutically acceptable salt thereof, as described above, or the pharmaceutical composition as described above. Said patient is preferably a mammal, more preferably a human patient. The route of administration may be oral, topical (including, but not limited to, topical application, spraying, and the like), parenteral (including subcutaneous, intramuscular, cortical and intravenous) administration, bronchial administration, or nasal administration, etc.

Said disease or condition associated with TRK kinases or NTRK genes is, for example, diseases caused by dysregulation of TRK kinase activity and/or diseases associated with NTRK gene fusions, such as, but not limited to: cancer, pain, inflammation, neurodegenerative diseases, and cell proliferation diseases. The compounds of the present application may be used, for example, for inhibiting proliferation, metastasis, and the like of cancer cells, for relieving pain, for anti-inflammation, for treating or alleviating neurodegenerative diseases, and the like.

In some embodiments, said disease or condition associated with TRK kinases or NTRK genes is a cancer.

Exemplary cancers include bladder cancer, breast cancer, cervical cancer, colorectal cancer, small bowel cancer, colon cancer, rectal cancer, anal cancer, endometrial cancer, head and neck cancer (e.g., cancers of larynx, laryngopharynx, nasopharynx, oropharynx, lips, and mouth), kidney cancer, liver cancer (e.g., hepatocellular carcinoma, bile duct cell carcinoma), lung cancer (e.g., adenocarcinoma, small cell lung cancer, and non-small cell lung cancer, small cell cancer and non-small cell cancer, bronchial cancer, bronchial adenoma, pleural pneumoblastoma), ovarian cancer, prostate cancer, testicular cancer, uterine cancer, esophageal cancer, gallbladder cancer, pancreatic cancer (e.g., exocrine pancreatic cancer), thyroid cancer, parathyroid cancer, skin cancer (e.g., squamous cell carcinoma, Kaposi's sarcoma, Merkel cell skin cancer), and brain cancer (e.g., stellate cell tumor, neural tube embryonal cell tumor, ventricular meningioma, neuroectodermal tumor, pineal gland tumor).

Additional exemplary cancers include hematopoietic malignancies such as leukemia or lymphoma, multiple myeloma, chronic lymphocytic lymphoma, adult T-cell leukemia, B-cell lymphoma, cutaneous T-cell lymphoma, acute myelogenous leukemia, Hodgkin or non-Hodgkin lymphoma, myeloproliferative neoplasms (e.g., true erythroblastosis, primary thrombocythemia, and primary myelofibrosis ), Waldenstrom's macroglobulinemia, hairy cell lymphoma, chronic myelogenous lymphoma, acute lymphoblastic lymphoma, AIDS-related lymphoma, and Burkitt's lymphoma.

Additional exemplary cancers include eye tumors, glioblastoma, melanoma, rhabdomyosarcoma, lymphosarcoma, and osteosarcoma.

In some preferred embodiments, said diseases or conditions associated with TRK kinases or NTRK genes are selected from hepatocellular carcinoma, breast cancer, bladder cancer, colorectal cancer, melanoma, mesothelioma, lung cancer, prostate cancer, membrane adenocarcinoma, pancreatic cancer, esophageal cancer, stomach cancer, lymphoma, leukemia, nasopharyngeal cancer, testicular cancer, thyroid cancer, squamous cell carcinoma, glioblastoma, neuroblastoma, uterine cancer, and rhabdomyosarcoma.

In some preferred embodiments, said disease or condition associated with the TRK kinases or NTRK genes is a solid tumor, such as melanoma, breast tumor, and neuroblastoma, glioblastoma, Ewing's sarcoma, retinoblastoma, and the like.

In other embodiments, said disease or condition associated with the TRK kinases or NTRK genes is a cell proliferation disease such as, but not limited to, benign prostatic hyperplasia, familial adenomatosis, polyposis, neurofibromatosis, psoriasis, atherosclerosis, and disorders associated with vascular smooth cell proliferation and neoplastic endothelial formation such as restenosis after angioplasty or surgery, pulmonary fibrosis, arthritis, glomerulonephritis, retinopathy (including diabetic and neonatal retinopathy and age-related macular degeneration), graft vascular disease (e.g., which may occur after vascular or organ transplantation), acromegaly and conditions secondary to acromegaly.

In yet other embodiments, said disease or condition associated with the TRK kinases or NTRK genes is an inflammatory condition, including, but not limited to, (1) metaplastic inflammation; (2) exudative inflammation (plasmacytosis, fibrillitis, septic inflammation, hemorrhagic inflammation, necrotizing inflammation, cicatricial inflammation); (3) hyperproliferative inflammation; and (4) idiopathic inflammation (tuberculosis, syphilis, leprosy, lymphomalacia, and the like).

In other embodiments, said disease or condition associated with the TRK kinases or NTRK genes is pain, including but not limited to inflammatory pain, arthritis pain, complex regional pain syndrome, lumbar coccygeal pain, musculoskeletal pain, neuropathic pain, chronic pain, cancer-associated pain, acute pain, postoperative pain, and the like.

In other embodiments, said disease or condition associated with the TRK kinases or NTRK genes is a neurodegenerative disease, including but not limited to Alzheimer's disease and Parkinson's disease.

The "therapeutically effective amount" of the compounds of the present application for the treatment of the aforementioned diseases may be reasonably determined by an experienced physician or researcher based on the patient's condition, physical condition, severity of disease, route of administration, and other factors.
The present application is further described and illustrated below in connection with specific examples.

**Examples**

The following examples set forth herein are for illustrative purposes only, to exemplify aspects of the invention and the manner in which they are to be carried out, and are not intended to limit in any way the scope of protection as claimed.
Unless otherwise stated, all raw materials and reagents were obtained from commercial sources. The instruments and equipment used in the synthesis experiments and product analysis are all conventional instruments and equipment normally used in organic synthesis. All unspecified reaction conditions and test conditions are conventional reaction conditions and test conditions commonly used in the field and can be obtained with reference to the relevant technical literature or instrument manuals, etc.

**Example 1: N-(1-(1-(3,5-difluorophenyl)ethyl)-3-(1,4,5,6-tetrahydropyrrolo[3,4-dlimidazol-2-yl)-1H-indazol-5-amine**

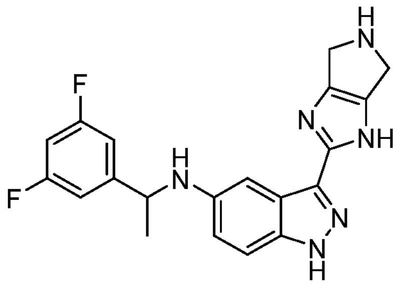

1

**Synthetic route for compound 1:**

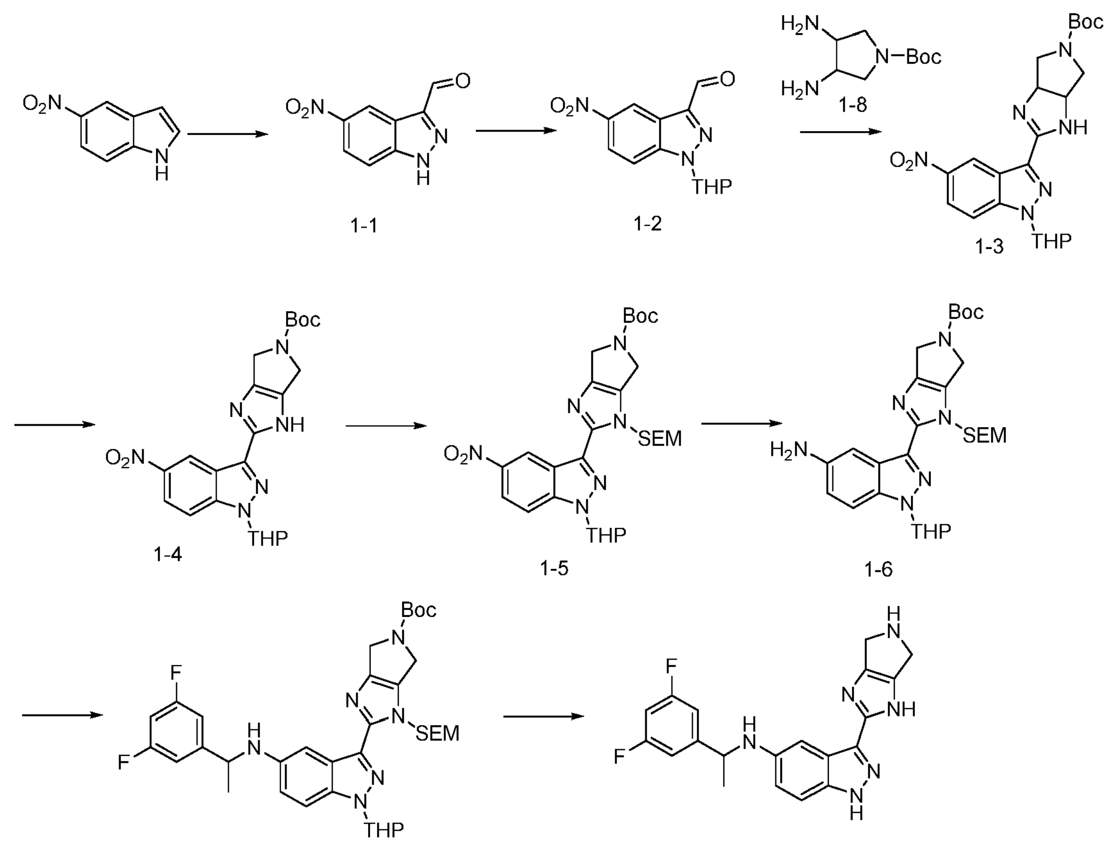

1-1 1-2 1-3 1-8 1-4 1-5 1-6 1-7 1

**Synthetic route for intermediate 1-8:**

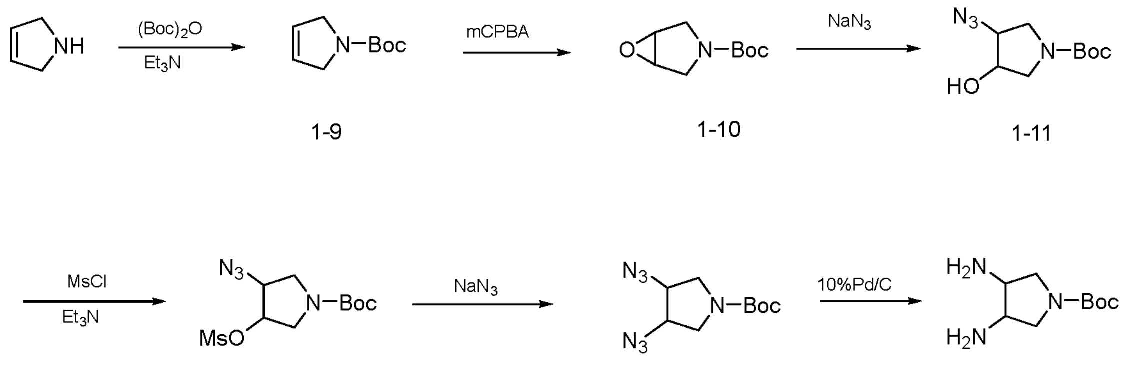


1-9 1-10 1-11 1-12 1-13 1-8

**Synthesis Method:**

**Synthesis of intermediate 1-1: 5-nitro-1H-indazole-3-formaldehyde**

Sodium nitrite (1.7 g, 24.70 mmol) was dissolved in 9 ml of DMF and 5 ml of water, and then cooled to 0°C, to which 3N HCl (7.2 ml, 21.61 mmol) was added slowly dropwise. It was allowed to react for 10 min after the dropwise addition. To the reaction solution a solution of 5-nitro-1H-indole (501 mg, 3.10 mmol) in DMF (6 ml) was added dropwise at 0°C. After the addition was completed, the mixture was heasted to 80°C and it was allowed to react overnight. The reaction mixture was extracted with ethyl acetate 3 times, and the resulting organic phases were combined, washed with water 3 times, saturated brine, dried over anhydrous sodium sulfate, concentrated, and purified on silica gel column

to give 370 mg of Intermediate 1-1 in a yield of 62.6%.

$^1$H NMR (400 MHz, DMSO) δ 14.71 (brs, 1H), 10.26 (s, 1H), 8.95 (d, J = 8.0 Hz, 1H), 8.33 (dd, J = 8.0 Hz, J = 12.0 Hz, 1H), 7.93 (d, J = 16.0 Hz, 1H).

**Synthesis of Intermediate 1-2: 5-nitro-1-(tetrahydro-2H-pyran-2-yl)-1H-indazole-3-formaldehyde**

Intermediate 1-1 (370 mg, 1.94 mmol) was dissolved in 30 ml DCM, to which p-toluenesulfonic acid (333 mg, 1.94 mmol) was added and the resulting mixture was stirred for 2 min. To the reaction solution, 3,4-dihydro-2H-pyran (326 mg, 3.87 mmol) was added and it was allowed to react for 2 h at room temperature. Water was added to the reaction solution, the mixture was extracted with DCM for 2 times, and the resulting organic phases were combined, washed with saturated sodium bicarbonate solution and saturated saline, dried over anhydrous sodium sulfate, concentrated, and purified on a silica gel column to give 380 mg of Intermediates 1-2 in a yield of 71.3 %.

$^1$H NMR (400 MHz, CDCl3) δ10.30 (s, 1H), 9.26 (d, J = 4.0 Hz, 1H), 8.39-8.36 (m, 1H), 7.82 (d, J = 8.0 Hz, 1H), 5.93-5.90 (m, 1H), 4.04-3.99 (m, 1H), 3.86-3.80 (m, 1H), 2.60-2.80 (m, 1H), 2.60-3.80 (m, 1H), 2.60-3.80 (m, 1H), 2.60-3.80 (m, 1H). 1H), 2.60-2.52 (m, 1H), 2.23-2.20 (m, 2H), 1.84-1.78 (m, 3H).

**Synthesis of intermediates 1-9: tert-butyl 2,5-dihydro-1H-pyrrole-1carboxylate**

3-Pyrroline (10.0 g, 0.15 mol) was dissolved in 400 ml of dichloromethane and triethylamine (40.6 ml, 0.29 mol), and then cooled to 0 °C, to which $(Boc)_2O$ (37.9 g, 0.17 mol) was slowly added and it was allowed to react overnight at room temperature. Water was added, the mixture was extracted with dichloromethane twice, and the resulting organic phases were combined, washed three times with water, saturated saline water, dried over anhydrous sodium sulfate, concentrated, purified on silica gel column to give intermediates 1-9 in a yield of 91.0%.

**Synthesis of Intermediate 1-10: tert-butyl 6-oxa-3-azabicyclo[3.1.0]hexane-3-carboxylate**

Intermediate 1-9 (24.5 g, 0.15 mol) was dissolved in 450 ml of dichloromethane, and then cooled to 0°C, to which m-chloroperoxybenzoic acid (37.5 g, 0.22 mol) was slowly added in batches and it was allowed to react overnight at room temperature. Saturated sodium thiosulfate (40 ml) was added with stirring for 30 minutes. The resulting aqueous phase was extracted with dichloromethane for 2 times, washed with saturated potassium carbonate solution, water and saturated brine, dried over anhydrous sodium sulfate, concentrated, and purified on silica gel column to give Intermediates 1-10 in a yield of 84.9%.

$^1$H NMR (400 MHz, CDCl3) δ 3.85 (d, J = 12.0 Hz, 1H), 3.77 (d, J = 12.0 Hz, 1H), 3.69-3.67 (m, 2H), 3.36-3.30 (m, 2H), 1.45 (s, 9H).

**Synthesis of Intermediate 1-11: tert-butyl 3-azido-4-hydroxypyrrolidinyl-1-carboxylate**

Intermediate 1-10 (20.8 g, 0.12 mol) was dissolved in 150 ml of 1,4-dioxane and 50 ml of water, and sodium azide (24.0 g, 0.37 mol) was added. The reaction mixture was heated to 106°C for 18 hrs and then cooled to room temperature. 100 ml of saturated brine was added, the mixture was extracted with dichloromethane (250 ml x 4), and the resulting organic phases were combined, washed with saturated brine, dried over anhydrous sodium sulfate and concentrated to give intermediates 1-11 in a yield of 100%. $^1$H NMR (400 MHz, CDCl3) δ 4.27-4.24 (m, 1H), 3.94 (s, 1H), 3.73-3.59 (m, 2H), 3.41-3.36 (m, 2H), 1.47 (s, 9H).

**Synthesis of Intermediate 1-12: tert-butyl 3-azido-4-((methylsulfonyl)oxy)pyrrolidinyl-1-carboxylate**

Intermediate 1-11 (28.0 g, 0.12 mol) was dissolved in 350 ml of dichloromethane and triethylamine (37.3 g, 0.37 mol), and then cooled to 0°C. Methylsulfonyl chloride (16.9 g, 0.15 mol) was added slowly dropwise, and it was allowed to react for 2 h at room temperature after the dropwise addition. The reaction was quenched by the addition of water, the mixture was extracted with dichloromethane for 2 times, and the resulting organic phases were combined, washed with saturated sodium bicarbonate solution, water and saturated brine, dried over anhydrous sodium sulfate and concentrated to give Intermediate 1-12 in a yield of 98.0%.

**Synthesis of Intermediate 1-13: tert-butyl 3,4-diazido-pyrrolidinyl-1-carboxylate**

Intermediate 1-12 (36.9 g, 0.12 mol) was dissolved in 250 ml of DMF, to which sodium azide (23.5 g, 0.36 mol) was added. The reaction mixture was heated to 90°C and it was allowed to react for 2 days. The resulting mixture was cooled to room temperature, and 750 ml of water was added. The mixture was extracted with methyl tert-butyl ether

(400 ml * 4), and the resulting organic phases were combined, washed with saturated brine, dired over anhydrous sodium sulfate, purified on silica gel column to give Intermediate 1-13 in a yield of 62.2%.

**Synthesis of Intermediate 1-8: tert-butyl 3,4-diaminopyrrolidinyl-1-carboxylate**

Intermediate 1-13 (18.9 g, 0.08 mol) was dissolved in 200 ml of methanol, to which 10% Pd/C was added and the atmosphere was replaced with hydrogen gas three times. It was allowed to react for 2 days. The resulting mixture was filtered, and concentrated to obtain Intermediate 1-8 in a yield of 78%. $^1$H NMR (400 MHz, CDCl3) $\delta$ 3.51-3.49 (m, 2H), 3.40-3.36 (m, 2H), 3.21-3.36 (m), 3.21-3.36 (m, 2H), 3.21-3.36 (m, 2H). , 2H), 3.21-3.11 (m, 2H), 1.47 (s, 9H).

**Synthesis of Intermediate 1-3: tert-butyl 2-(5-nitro-1-(tetrahydro-2H-pyran-2-yl)-1H-indazol-3-yl)-3a,4,6,6a-tetrahydropyrrolo[3,4-d]imidazole-5(1H)-carboxylate**

Intermediate 1-2 (300 mg, 1.09 mmol) was dissolved in 20 ml of tert-butanol, to which tert-butyl 3,4-diaminopyrrolidine-1-carboxylate (219 mg, 1.09 mmol), iodine (415 mg, 1.63 mmol), and potassium carbonate (452 mg, 3.27 mmol) were alled, and the mixture was heated to 70°C for 3 hours. After the reaction was completed, it was reduced to room temperature. The reaction was quenched by adding 5% aqueous sodium thiosulfate, the mixture was extracted with EA, and the resulting organic phases were washed with saturated brine, dried over anhydrous sodium sulfate, concentrated, and purified on silica gel column, to give 410 mg of Intermediates 1-3 in a yield of 82.4 %.

**Synthesis of Intermediate 1-4: tert-butyl 2-(5-nitro-1-(tetrahydro-2H-pyran-2-yl)-1H-indazol-3-yl)-4,6-dihydropyrrolo[3,4-d]imidazole-5(1H)-carboxylate**

Intermediate 1-3 (400 mg, 0.88 mmol) was dissolved in 5 ml of DMSO, to which IBX (491 mg, 1.75 mmol) was added and it was allowed to react at 50°C for 3 hours. After the reaction was completed, it was reduced to room temperature, and the reaction was quenched by adding water. The mixture was extracted with EA, and the resulting organic phase was washed with saturated brine, dried over anhydrous sodium sulfate, concentrated, and purified on silica gel column to give 270 mg of Intermediates 1-4 in a yield of 67.8 %.

**Synthesis of Intermediate 1-5: tert-butyl 2-(5-nitro-1-(tetrahydro-2H-pyran-2-yl)-1H-indazol-3-yl)-1- ((2-(trimethylsilyl)ethoxy)methyl)-4,6-dihydropyrrolo [3,4-d]imidazole-5(1H)-carboxylate**

Intermediate 1-4 (270 mg, 0.59 mmol) was dissolved in 25 ml THF, and then cooled down to 0oC, to which NaH (60%) (35.6 mg, 0.89 mmol) was added, and the mixture was stirred for 10 mim, and then SEMCl (149 mg, 0.89 mmol) was added. After the addition was completed, it was raised to room temperature and stirred. After the reaction was completed, the reaction was quenched with water, the mixture was extracted with EA, and the resulting organic phase was washed with saturated brine, dried over anhydrous sodium sulfate, concentrated, and purified on silica gel column to give 209 mg of intermediates 1-5 in a yield of 60.2 %.

$^1$H NMR (400 MHz, CDCl3) $\delta$ 9.51 (d, J = 8.0 Hz, 1H), 8.33 (d, J = 8.0 Hz, 1H), 7.69 (d, J = 8.0 Hz, 1H), 5.99-5.94 (m, 1H), 5.90-5.87 (m, 1H), 5.81-5.79 (m, 1H), 4.63-4.52 (m, 4H), 4.00-3.98 (m, 1H), 3.79- 3.77 (m, 1H), 3.62-3.56 (m, 2H), 2.55-2.52 (m, 1H), 2.20-2.17 (m, 2H), 1.79-1.76 (m, 3H), 1.54 (s, 9H), 0.95-0.89 (m, 2H), -0.08-0.89 (m, 2H), -0.09-0.09 (m, 2H), -0.09-0.09 (m, 2H) 2H), -0.08 (s, 9H).

**Synthesis of Intermediates 1-6: tert-butyl 2-(5-amino-1-(tetrahydro-2H-pyran-2-yl)-1H-indazol-3-yl)-1-((2-(trimethylsilyl)ethoxy)methyl)-4,6-dihydropyrrolo [3,4-d]imidazole-5(1H)-carboxylate**

Intermediates 1-5 (200 mg, 0.34 mmol) were dissolved in 60 ml methanol, to which 10% Pd/C (20 mg) was added, and the atmosphere was replaced with hydrogen 3 times. It was allowed to react at room temperature for 3 hours. After completion of the reaction, the reaction mixture was filtered and concentrated to give 173 mg of Intermediate 1-6 in a yield of 91.2 %.

**Synthesis of Intermediate 1-7: tert-butyl 2-(5-((1-(3,5-difluorophenyl) ethyl)amino)-1-(tetrahydro-2H-pyran-2-yl)-1H-indazol-3-yl)-1-((2-(trimethylsilyl) ethoxy)methyl)-4,6-dihydropyrrolo[3,4-d]imidazole-5(1H)-carboxylate**

Intermediates 1-6 (20.0 mg, 0.04 mmol) and 3,5-difluoroacetophenone (6.8 mg, 0.04 mmol) were dissolved in 5 ml of toluene, to which 1 drop of glacial acetic acid was added. It was allowed to react at 80°C overnight. The reaction mixture was cooled to room temperature and sodium triacetylborohydride (9.9 mg, 0.05 mmol) was added. It was allowed to react at room temperature for 3 h. The reaction was quenched by addition of water, the mixture was extracted with

EA and the resulting organic phase was washed with saturated brine, concentrated and purified to give 9 mg of intermediates 1-7 in a yield of 35.9 %.

**Synthesis of compound 1: N-(1-(1-(3,5-difluorophenyl)ethyl)-3-(1,4,5,6-tetrahydropyrrolo[3,4-d]imidazol-2-yl)-1H-indazol-5-amine**

Intermediates 1-7 (10.0 mg, 0.05 mmol) were dissolved in 2 ml of methanol, to which 1 ml of concentrated hydrochloric acid was added, and it was allowed to react at 50°C for 5 h. The reaction mixture was concentrated, dissolved in 5 ml of methanol, to which 0.5 ml of ammonia was added, and then the resulting mixture was concentrated, and purified by preparative plate to give the final product of 2.1 mg in a yield of 38.3 %.

$^1$H NMR (400 MHz, CD3OD) $\delta$7.33 (d, J = 8.0 Hz, 1H), 7.18 (d, J = 4.0 Hz, 1H), 7.10-7.06 (m, 2H), 6.98 (dd, J = 4.0 Hz, J = 8.0 Hz, 1H), 6.74-6.69 (m, 1H), 4.70-4.65 (m, 1H), 4.18 (m, 1H), 4.70-4.65 (m, 1H). 1H), 4.18 (s, 4H), 1.55 (d, J = 8.0 Hz, 3H).

**Example 2: N-(3,5-difluorobenzyl)-3-(5-(piperidin-4-ylmethyl)-1,4,5,6-tetrahydropyrrolo[3,4-d]imidazol-2-yl)-1H-indazol-5-amine**

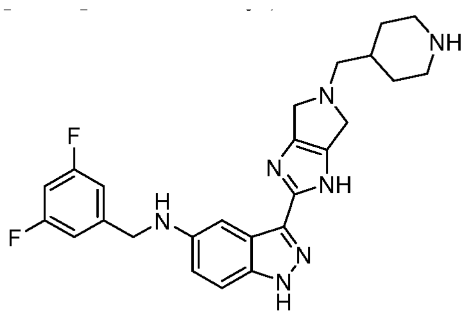

2

**Synthetic route for compound 2:**

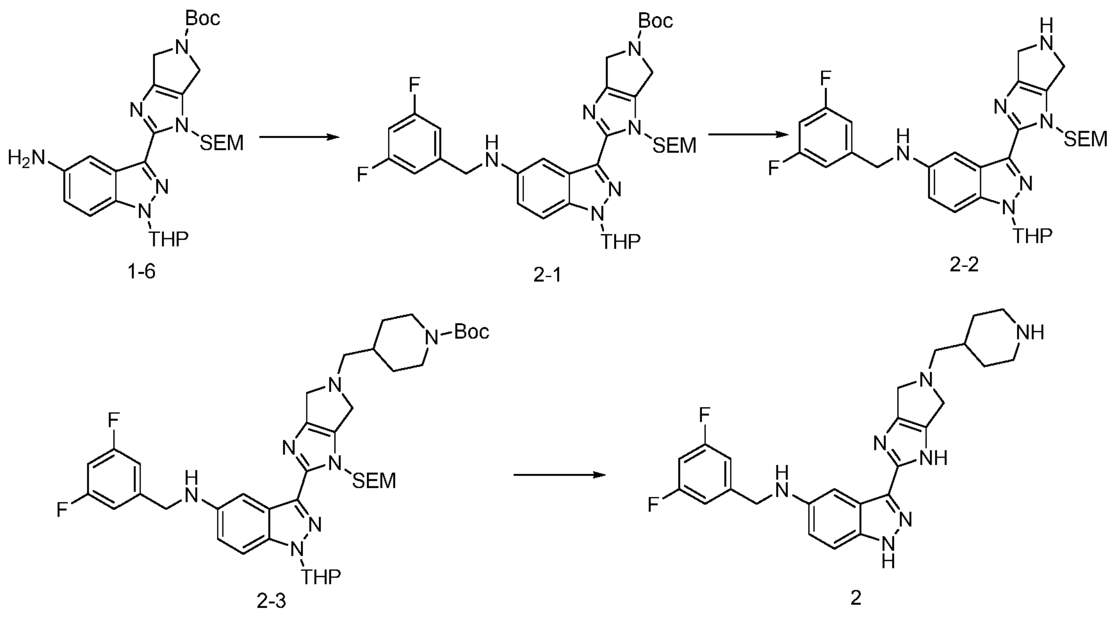

1-6

2-1

2-2

2-3

2

**Synthesis Method:**

**Synthesis of Intermediate 2-1: tert-butyl 2-(5-((3,5-difluorobenzyl)amino)-1-(tetrahydro-2H-pyran-2-yl)-1H-indazol-3-yl)-1-((2-(trimethylsilyl)ethoxy)methyl)-4,6-dihydropyrrolo[3,4-d]imidazole-5(1H)-carboxylate**

Intermediates 1-6 (80.0 mg, 0.14 mmol) and 3,5-difluorobenzaldehyde (24.6 mg, 0.17 mmol) were dissolved in 5 ml of toluene, to which 1 drop of glacial acetic acid was added, and it was allowed to react at 80°C overnight. The reaction mixture was cooled to room temperature, to which sodium triacetylborohydride (45.9 mg, 0.22 mmol) was allowed. It was allowed to react for 3 hr at room temperature. The reaction was quenched with water, the mixture was extracted with EA, and the resulting organic phase was washed with saturated saline, concentrated and purified to give 79 mg of Intermediate 2-1 in a yield of 80.5 %.

**Synthesis of Intermediate 2-2: N-(3,5-difluorobenzyl)-1-(tetrahydro-2H-pyran-2-yl)-3-(1-((2-(trimethylsilyl)ethoxy)methyl)-1,4,5,6-tetrahydropyrrolo[3,4-d]imidazol-2-yl)-1H-indazol-5-amine**

Intermediate 2-1 (79.0 mg, 0.12 mmol) was dissolved in 20 ml DCM, to which $ZnBr_2$ (105.0 mg, 0.46 mmol) was added and the mixture was stirred at room temperature. After the reaction was completed, it was quenched with water, the mixture was extracted with DCM, and the organic phase was washed with saturated brine, dried over anhydrous sodium sulfate, and concentrated to give 60.2 mg of Intermediate 2-2 in a yield of 89.4 %.

**Synthesis of Intermediate 2-3: tert-butyl 4-((2-(5-((3,5-difluorobenzyl) amino)-1-(tetrahydro-2H-pyran-2-yl)-1H-indazol-3-yl)-1-((2-(trimethylsilyl) ethoxy)methyl)-4,6-dihydropyrrolo[3,4-d]imidazol-5(1H)-yl)methyl) piperidine-1-carboxylate**

Intermediate 2-2 (67.5 mg, 0.12 mmol) and tert-butyl 4-formylpiperidine-1-carboxylate (29.7 mg, 0.14 mmol) were dissolved in 10 ml of DCM, and the mixture was stirred at room temperature for 10 min, and then triacetylsodium borohydride (37.0 mg, 0.17 mmol) was added. It was allowed to react at room temperature for 3 h. The reaction was quenched by addition of water, the mixture was extracted with DCM, and the resulting organic phase was washed with saturated saline, concentrated and purified to give 24.9 mg of intermediate 2-3 in a yield of 27.5 %.

**Synthesis of compound 2: N-(3,5-difluorobenzyl)-3-(5-(piperidin-4-ylmethyl)-1,4,5,6-tetrahydropyrrolo[3,4-d]imidazol-2-yl)-1H-indazol-5-amine**

Intermediate 2-3 (24.9 mg, 0.03 mmol) was dissolved in 2 ml of methanol, to which 1 ml of concentrated hydrochloric acid was added, and it was allowed to react at 50°C for 5 h. The reaction mixture was concentrated, dissolved in 5 ml of methanol, to which 0.5 ml of ammonia was added, and then the resulting mixture was concentrated, and purified by preparative plate to give the final product 5.1 mg in a yield of 34.4 %.

$^1H$ NMR (400 MHz, CD3OD) δ7.37 (d, J = 8.0 Hz, 1H), 7.30 (d, J = 4.0 Hz, 1H), 7.08 (dd, J = 4.0 Hz, J = 16.0 Hz, 2H), 7.01 (dd, J = 4.0 Hz, J = 8.0 Hz, 1H), 6.80-6.75 (m, 1H) , 4.46 (s, 2H), 3.90 (s, 4H), 3.19-3.15 (m, 2H), 2.78-2.72 (m, 4H), 2.06-2.04 (m, 1H), 1.96-1.91 (m, 2H), 1.81-1.78 (m, 1H), 1.62-1.57 (m, 1H).

**Example 3: 5-(3,5-difluorobenzyl)-3-(5-(methylsulfonyl)-1,4,5,6-tetrahydropyrrolo[3,4-d]imidazol-2-yl)-1H-indazole**

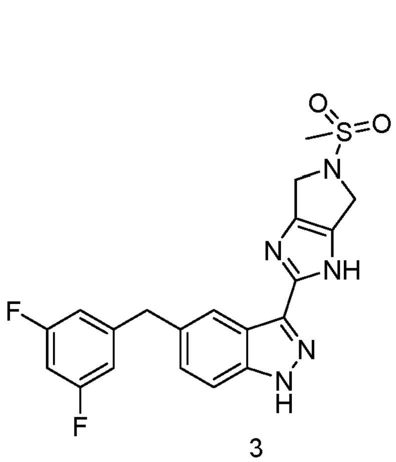

3

**Synthetic route for compound 3:**

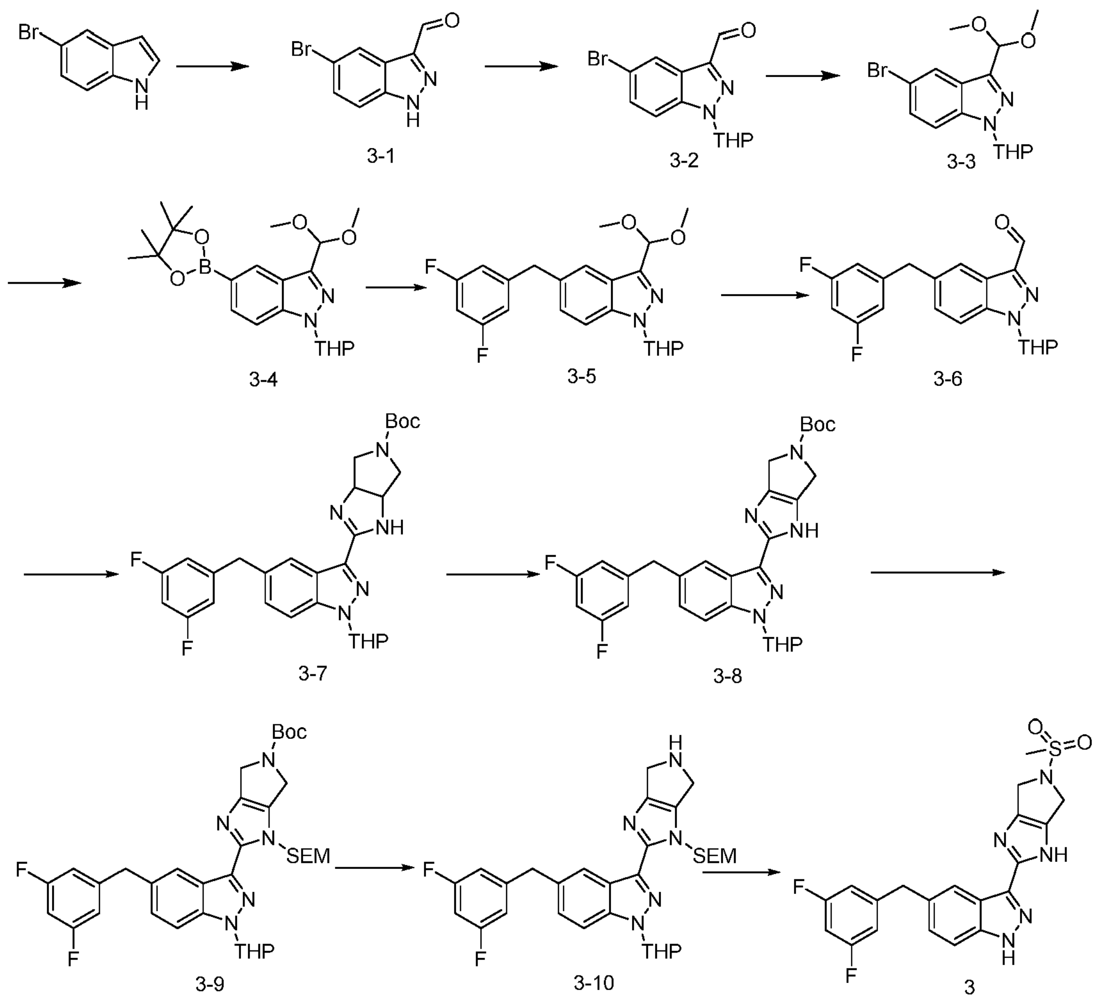

**Synthesis Method:**

**Synthesis of intermediate 3-1: 5-bromo-1H-indazole-3-formaldehyde**

Sodium nitrite (1.41 g, 20.4 mmol) was dissolved in 10 ml of water, to which 10 ml of DMF was added, and 3M HCl (2.29 ml, 6.89 mmol) was added dropwise at 0°C. The mixture was stirred for 10 min, to which a solution of 5-bromoindole (500 mg, 2.55 mmol) in DMF (10 ml) was added, and it was allowed to react for 3 h at room temperature. Water was added to the reaction solution, the mixture was extracted with ethyl acetate for 2 times, and the organic phases were combined, washed with saturated brine, dried over anhydrous sodium sulfate, concentrated and purified on silica gel column to give Intermediate 3-1 410 mg in a yield of 71.4%.

$^1$H NMR (400 MHz, CDCl3) δ 10.28 (s, 1H), 8.53 (d, J = 1.1 Hz,1H), 7.61 (dd, J = 8.9, 1.8 Hz, 1H), 7.49 (d, J = 8.8, 1H).

**Synthesis of Intermediate 3-2: 5-bromo-1-(tetrahydro-2H-pyran-2-yl)-1H-indazole-3-carbaldehyde**

Intermediate 3-1 (300 mg, 1.33 mmol) was dissolved in 20 ml of DCM, to which p-toluenesulfonic acid (279 mg, 1.46 mmol) was added and the mixtuew was stirred for 2 min. To the reaction solution was added a solution of 3,4-dihydro-2H-pyran (168 mg, 2.0 mmol) in DCM (3 ml), and it was allowed to react for 1 h at room temperature. Water was added to the reaction solution, the mixture was extracted with DCM for 2 times, and the resulting organic phases were combined, washed with saturated sodium bicarbonate solution and saturated saline, dried over anhydrous sodium sulfate, concentrated, and purified by silica gel column to give 300 mg of Intermediate 3-2 in a yield of 72.8%.

**Synthesis of Intermediate 3-3: 5-bromo-3-(dimethoxymethyl)-1-(tetrahydro-2H-pyran-2-yl)-1H-indazole**

Intermediate 3-2 (50.0 mg, 0.16 mmol), trimethyl orthoformate (20.6 mg, 0.19 mmol) and p-toluenesulfonic acid (2.8 mg, 0.02 mmol) were dissolved in 5 ml methanol and it was allowed to react at room temperature for 2 h. The reaction was quenched by adding water, the mixture was extracted with EA, and the resulting orgianic phase was washed with saturated brine, dried over anhydrous sodium sulfate, and concentrated and purified to give 23 mg of intermediate 3-3 in a yield of 40.0%.

1H NMR (400 MHz, CDCl3) δ 8.13 (d, J = 4.0 Hz, 1H), 7.52-7.46 (m, 2H), 5.74 (s, 1H), 5.72-5.69 (m, 1H), 4.08-4.04 (m, 1H), 3.78-3.72 (m, 1H), 3.47 (s, 6H), 2.52-2.48 (m, 1H), 2.15-2.04 (m, 2H), 1.80-1.71 (m, 3H).

**Synthesis of Intermediate 3-4: 3-(dimethoxymethyl)-1-(tetrahydro-2H-pyran-2-yl)-5-(4,4,5,5-tetramethyl-1,3,2-dioxaborolan-2-yl)-1H-indazole**

Intermediate 3-3 (20.0 mg, 0.06 mmol), pinacolborate (28.6 mg, 0.11 mmol), Pd(dppf)Cl2 (4.1 mg, 0.006 mmol) and potassium acetate (16.6 mg, 0.17 mmol) were dissolved in 10 ml of 1,4-dioxane, the atmosphere was replaced with nitrogen three times and it was allowed to react at 100°C overnight. The reaction was quenched with water, the mixture was extracted with EA, and the organic phase was washed with saturated brine, dried over anhydrous sodium sulfate, concentrated by thin layer chromatography, to obtain 15 mg of Intermediate 3-4 in a yield of 66.2%.

$^1$H NMR (400 MHz, $CDCl_3$) δ 8.45 (s, 1H), 7.82 (dd, J = 4.0 Hz, J = 8.0 Hz, 1H), 7.58 (dd, J = 8.0, 4.0 Hz, 1H), 5.81 (s, 1H), 5.76 (dd, J = 8.0, 4.0 Hz, 1H), 4.11-4.07 (m, 1H), 3.78-3.07 (m, 1H). 1H), 3.78-3.75 (m, 1H), 3.48 (s, 6H), 2.60-2.51 (m, 1H), 2.16-2.03 (m, 2H), 1.79-1.60 (m, 3H), 1.38 (s, 12H).

**Synthesis of Intermediate 3-5: 5-(3,5-difluorobenzyl)-3-(dimethoxymethyl)-1-(tetrahydro-2H-pyran-2-yl)-1H-indazole**

Intermediate 3-4 (175 mg, 0.44 mmol), 3,5-difluorobenzyl bromide (180 mg, 0.87 mmol), Pd(PPh3)4 (50.2 mg, 0.04 mmol), and sodium carbonate (138 mg, 1.31 mmol) were dissolved in 10 ml of tetrahydrofuran and 2 ml of water, the atmosphere was replaced with nitrogen 3 times, and it was allowed to react at 65°C. The reaction was quenched with water, the mixture was extracted with EA, and the resulting organic phase was washed with saturated brine, dried over anhydrous sodium sulfate, and concentrated by column chromatography to give 148 mg of Intermediate 3-5 in a yield of 84.5%.

**Synthesis of Intermediate 3-6: 5-(3,5-difluorobenzyl)-1-(tetrahydro-2H-pyran-2-yl)-1H-indazole-3-formaldehyde**

Intermediate 3-5 (50 mg, 0.12 mmol), and p-toluenesulfonic acid (47.3 mg, 0.25 mmol) were dissolved in 10 ml of acetonitrile and it was allowed to react at room temperature. The reaction was quenched with water, the mixture was extracted with EA, and the resulting organic phase was washed with saturated brine, dried over anhydrous sodium sulfate, and concentrated by column chromatography to give 27 mg of Intermediate 3-6 in a yield of 61.0%.

$^1$H NMR (400 MHz, $CDCl_3$) δ 10.27 (s, 1H), 8.18 (s, 1H), 7.64 (d, J = 8.4 Hz, 1H), 7.31-7.28 (m, 1H), 6.74-6.63 (m, 3H), 5.84 (dd, J = 8.9, 2.9 Hz, 1H), 4.11 (s, 2H), 4.06-3.99 (m, 2H), 4.06-3.99 (m, 2H). 4.06-3.99 (m, 1H), 3.83-3.77 (m, 1H), 2.63-2.55 (m, 1H), 2.26-2.14 (m, 2H), 1.85-1.74 (m, 3H).

**Synthesis of Intermediate 3-7: tert-butyl 2-(5-(3,5-difluorobenzyl)-1-(tetrahydro-2H-pyran-2-yl)-1H-indazol-3-yl)-3a,4,6,6a-tetrahydropyrrolo[3,4-d]imidazole-5(1H)-carboxylate**

Intermediate 3-6 (50 mg, 0.14 mmol) was dissolved in 10 ml of tert-butanol, to which tert-butyl 3,4-diaminopyrrolidine-1-carboxylate (28.2 mg, 0.14 mmol), iodine (53.4 mg, 0.21 mmol), potassium carbonate (58.2 mg, 0.42 mmol) were added, and it was allowed to react at 70°C 3 hours. After the reaction was completed, it was reduced to room temperature, the reaction was quenched by adding 5% aqueous sodium thiosulfate, the mixture was extracted with EA, and the resulting organic phase was washed with saturated brine, dried over anhydrous sodium sulfate, concentrated, and purified on silica gel column, to give 68 mg of Intermediate 3-7 in a yield of 90.2%.

**Synthesis of Intermediate 3-8: tert-butyl 2-(5-(3,5-difluorobenzyl)-1-(tetrahydro-2H-pyran-2-yl)-1H-indazol-3-yl)-4,6-dihydropyrrolo[3,4-d] imidazole-5(1H)-carboxylate**

Intermediate 3-7 (65 mg, 0.12 mmol) was dissolved in 10 ml of DMSO, to which IBX (67.7 mg, 0.24 mmol) was added and it was allowed to react at 50°C for 3 hours. After the reaction was completed, it was reduced to room temperature. The reaction was quenched by adding water, the mixture was extracted with EA, and the resulting organic

phase was washed with saturated brine, dried over anhydrous sodium sulfate, concentrated, and purified on a silica gel column to give 34 mg of Intermediate 3-8 in a yield of 52.5%.

$^1$H NMR (400 MHz, $CDCl_3$) δ 8.33 (s, 1H), 7.53 (d, J = 8.8 Hz, 1H), 7.24 (dd, J = 8.8, 1.7 Hz, 1H), 6.74-6.71 (m, 1H), 6.67-6.60 (m, 2H), 5.72 (dd, J = 9.6, 2.6 Hz, 1H), 5.72 (dd, J = 9.6, 2.6 Hz, 1H), 4.60-4.54 (m, 1H). 4.60-4.54 (m, 4H), 4.13 (s, 2H), 4.10-4.06 (m, 1H), 3.81-3.75 (m, 1H), 2.62-2.54 (m, 1H), 2.16-2.06 (m, 2H), 1.85-1.70 (m, 3H), 1.55 (s, 9H).

**Synthesis of Intermediate 3-9: tert-butyl 2-(5-(3,5-difluorobenzyl)-1-(tetrahydro-2H-pyran-2-yl)-1H-indazol-3-yl)-1-((2-(trimethylsilyl)ethoxy)methyl)-4,6-dihydropyrrolo[3,4-d]imidazole-5(1H)-carboxylate**

Intermediate 3-8 (19.8 mg, 0.04 mmol) was dissolved in 5 ml of THF, and then cooled down to 0°C, to which NaH (60%) (1.9 mg, 0.05 mmol) was added, and the mixture was stirred for 15 mim, to which SEMCl (6.7 mg, 0.04 mmol) was added. After the addition was completed, the reaction mixture was heated to room temperature with stirring. After the reaction was completed, it was quenched with water, the mixture was extracted with EA, and the resulting organic phase was washed with saturated brine, dried over anhydrous sodium sulfate, concentrated, and purified on silica gel column to obtain 18.1 mg of Intermediate 3-9 in a yield of 73.4%.

**Synthesis of Intermediate 3-10: 5-(3,5-difluorobenzyl)-1-(tetrahydro-2H-pyran-2-yl)-3-(1- ((2-(trimethylsilyl)ethoxy)methyl)-1,4,5,6-tetrahydropyrrolo[3,4-d] imidazol-2-yl)-1H-indazole**

Intermediate 3-9 (182 mg, 0.27 mmol) was dissolved in 20 ml DCM, to which $ZnBr_2$ (24 mg, 1.10 mmol) was added and the mixture was stirred at room temperature. After the reaction was completed, it was quenched with water, the mixture was extracted with DCM, and the resulting organic phase was washed with saturated brine, dried over anhydrous sodium sulfate, and concentrated to give 140 mg of Intermediate 3-10 in a yield of 90.3 %.

**Synthesis of compound 3: 5-(3,5-difluorobenzyl)-3-(5-(methylsulfonyl)-1,4,5,6-tetrahydropyrrolo[3,4-d]imidazol-2-yl)-1H-indazole**

Intermediate 3-10 (30.0 mg, 0.05 mmol) was dissolved in 5 ml DCM, to which TEA (8.0 mg, 0.08 mmol), and methanesulfonyl chloride (7.3 mg, 0.06 mmol) were added. After the reaction was completed, it was quenched with water, the mixture was extracted with DCM, and the resulting organic phases were combined, washed with saturated saline and concentrated. The concentrate was dissolved in 4 ml of methanol, to which 2 ml of concentrated hydrochloric acid was added, and it was allowed to react at 50°C for 3 h. The reaction mixture was concentrated. The concentrate was dissolved in 5 ml of methanol, to which 0.5 ml of ammonia was added, and the resulting mixture was concentrated, and purified by preparative plate to give the final product of 5.6 mg in a yield of 24.6 %.

$^1$H NMR (400 MHz, $CD_3OD$) δ 8.18 (s, 1H), 7.53 (d, J = 8.4 Hz, 1H), 7.32 (dd, J = 8.7, 4.0 Hz, 1H), 6.91-6.85 (m, 2H), 6.78-6.73 (m, 1H), 4.66-4.52 (m, 4H), 3.65 (s, 2H), 3.01 (s, 2H), 3.01 (m, 2H). 2H), 3.01 (s, 3H).

**Example 4: 5-(1-(3,5-difluorophenyl)ethoxy)-3-(5-(1-methylpiperidin-4-yl)-1,4,5,6-tetrahydropyrrolo[3,4-d]imidazol-2-yl)-1H-indazole**

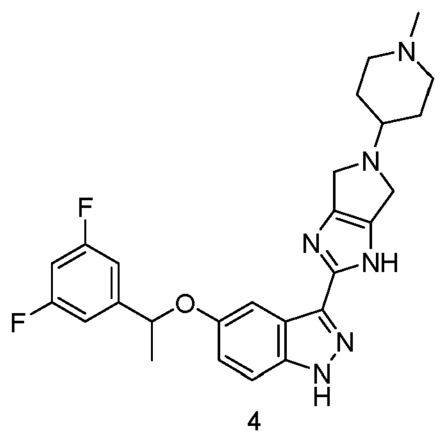

4

**Synthetic route for compound 4:**

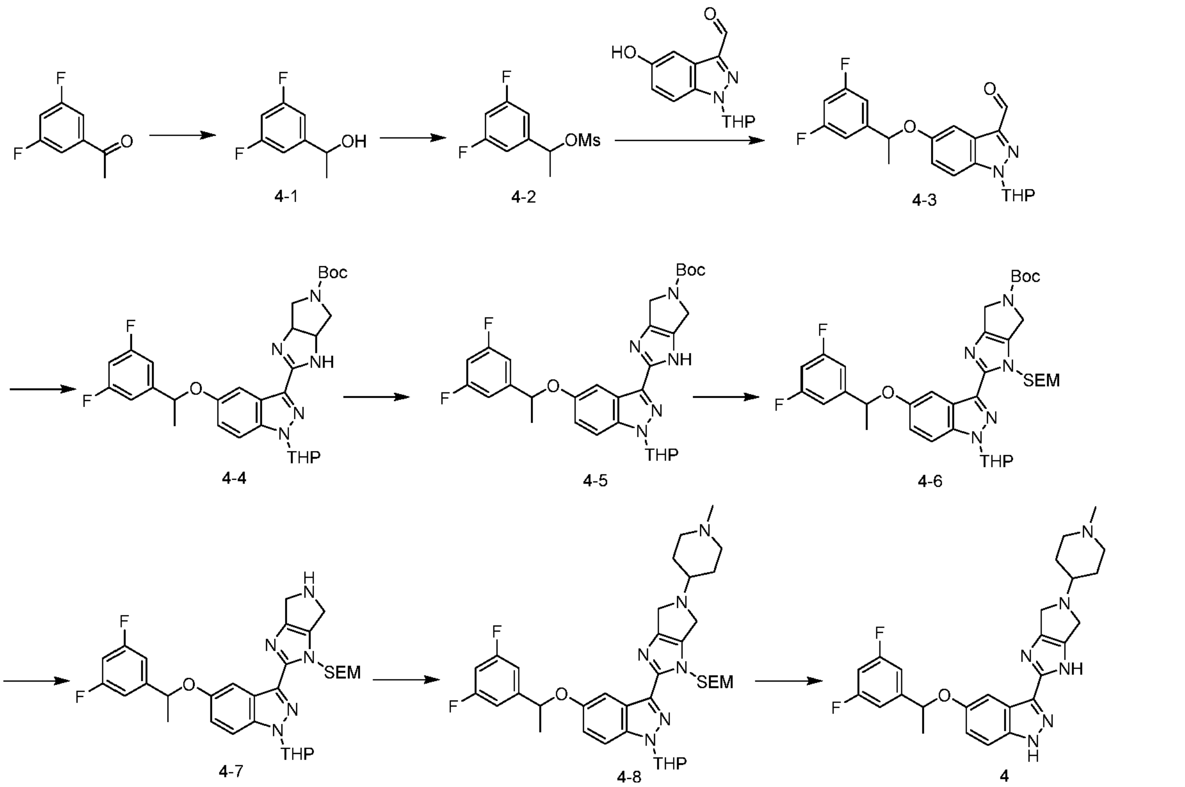

**Synthesis Method:**

**Synthesis of intermediate 4-1: 1-(3,5-difluorophenyl)ethanol**

3,5-Difluoroacetophenone (2.1 g, 13.50 mmol) was dissolved in 20 ml of methanol, to which sodium cyanoborohydride (1.3 g, 20.21 mmol) was added batchwise at 0oC and after addition the reaction mixture was transferred to room temperature and stirred for 1 h. After the reaction was completed, water was added to the reaction solution, the mixture was extracted with ethyl acetate 2 times, and the resulting organic phases were combined, washed with saturated brine, dried over anhydrous sodium sulfate, and concentrated to obtain 2.13 g of the crude intermediate 4-1 in a yield of 100.1 %.

**Synthesis of Intermediate 4-2: Ethyl 1-(3,5-difluorophenyl) methanesulfonate**

Intermediate 4-1 (2.13 g, 13.50 mmol) was dissolved in 20 ml of dichloromethane, to which triethylamine (4.1 g, 40.41 mmol) was added, and then methanesulfonyl chloride (1.9 g, 16.20 mmol) was added dropwise at 0°C. After the addition, the reaction mixture was transferred to room temperature and stirred for 1 hour. After the reaction was completed, water was added to the reaction solution, the mixture was extracted with ethyl acetate 2 times, and the resulting organic phases were combined, washed with saturated brine, dried over anhydrous sodium sulfate, concentrated, and purified on silica gel column, to give 3.2 g of crude intermediate 4-2 in a yield of 100 %.

**Synthesis of Intermediate 4-3: 5-(1-(3,5-difluorophenyl)ethoxy)-1-(tetrahydro-2H-pyran-2-yl)-1H-indazole-3-formaldehyde**

Intermediate 4-2 (2.2 g, 9.30 mmol) and 5-hydroxy-1-(tetrahydro-2H-pyran-2-yl)-1H-indazole-3-formaldehyde (2.3 g, 18.61 mmol) were dissolved in 20 ml of DMF, to which cesium carbonate (6.1 g, 0.82 mmol) was added, and it was allowed to react at 60°C for 2 hours. Water was added to the reaction solution, the mixture was extracted with ethyl acetate twice, and the resulting organic phases were combined, washed with saturated brine, dried over anhydrous sodium sulfate, concentrated and purified on silica gel column to obtain 3.1 g of Intermediate 4-3 in a yield of 86.2 %.

$^1$H NMR (400 MHz, $CDCl_3$) δ 10.19 (s, 1H), 7.72-7.45 (m, 2H), 7.19-7.16 (m, 1H), 7.00-6.92 (m, 2H), 6.73-6.67

(m, 1H), 5.81-5.77 (m, 1H), 5.43 (q, J = 6.3 Hz, 1H) , 4.03-3.98 (m, 1H), 3.81-3.75 (m, 1H), 2.56-2.49 (m, 1H), 2.26-2.03 (m, 2H), 1.82-1.71 (m, 3H), 1.67 (d, J = 6.3 Hz, 3H).

**Synthesis of Intermediate 4-4: tert-butyl 2-(5-(1-(3,5-difluorophenyl)ethoxy)-1-(tetrahydro-2H-pyran-2-yl)-1H-indazol-3-yl)-3a,4,6,6a-tetrahydropyrrolo[3,4-d]imidazole-5(1H)-carboxylate**

Intermediate 4-3 (3.1 g, 8.01 mmol) was dissolved in 30 ml of tert-butanol, to which tert-butyl 3,4-diaminopyrrolidine-1-carboxylate (1.6 g, 8.01 mmol), iodine (3.1 g, 12.11 mmol), and potassium carbonate (3.3 g, 24.02 mmol) were added, and it was allowed to react 70°C for 3 hours. After the reaction was completed, the temperature was lowered to room temperature, the reaction was quenched by adding 5% aqueous sodium thiosulfate. The mixture was extracted with EA, and the resulting organic phase was washed with saturated brine, dried over anhydrous sodium sulfate, concentrated, and purified on silica gel column, to give 3.6 g of Intermediate 4-4 in a yield of 79.1 %.
$^1$H NMR (400 MHz, $CDCl_3$) δ 7.74 (s, 1H), 7.49 (dd, J = 9.1 Hz, 3.2 Hz, 1H), 7.11 (dd, J = 9.1 Hz, 2.4 Hz, 1H), 7.00 (d, J = 7.3 Hz, 2H), 6.69 (t, J = 9.0 Hz, 1H), 5.70-5.67 (m, 1H), 5.49-5.44 (m, 1H), 5.00-4.80 (m, 1H), 4.52-4.31 (m, 1H), 4.03-3.99 (m, 1H), 3.77-3.66 (m, 5H), 2.56-2.47 (m, 1H), 2.18-2.10 (m, 1H), 2.06-2.02 (m , 1H), 1.79-1.72 (m, 3H), 1.66-1.64 (m, 3H), 1.45 (s, 9H).

**Synthesis of Intermediate 4-5: tert-butyl 2-(5-(1-(3,5-difluorophenyl)ethoxy)-1-(tetrahydro-2H-pyran-2-yl)-1H-indazol-3-yl)-4,6-dihydropyrrolo[3,4-d]imidazole-5(1H)-carboxylate**

Intermediate 4-4 (1.3 g, 2.31 mmol) was dissolved in 10 ml of DMSO, to which IBX (1.3 g, 4.60 mmol) was added and it was allowed to react at 50°C for 3 hours. After the reaction was completed, it was reduced to room temperature, and the reaction was quenched by adding water. The mixture was extracted with EA, and the resulting organic phase was washed with saturated brine, dried over anhydrous sodium sulfate, concentrated, and purified on silica gel column, to give 560 mg of Intermediate 4-5 in a yield of 43.2 %.

**Synthesis of Intermediate 4-6: tert-butyl 2-(5-(1-(3,5-difluorophenyl) ethoxy)-1-(tetrahydro-2H-pyran-2-yl)-1H-indazol-3-yl)-1-((2-(trimethylsilyl)ethoxy) methyl)-4,6-dihydropyrrolo [3,4-d] imidazole-5(1H)-carboxylate**

Intermediate 4-5 (790 mg, 1.40 mmol) was dissolved in 10 ml THF, and cooled down to 0°C, to which NaH (60%) (96.4 mg, 4.20 mmol) was added, and the mixture was stirred for 15 mim, to which SEMCl (280 mg, 1.68 mmol) was added. After the addition was completed, the mixture was raised to room temperature and stirred. After the reaction was completed, it was quenched with water, the mixture was extracted with EA, and the organic phase was washed with saturated brine, dried over anhydrous sodium sulfate, concentrated, and purified on silica gel column, to give 810 mg of Intermediate 4-6 in a yield of 83.3 %.

**Synthesis of Intermediate 4-7: 5-(1-(3,5-difluorophenyl)ethoxy)-1-(tetrahydro-2H-pyran-2-yl)-3-(1-((2-(trimethylsilyl)ethoxy)methyl)-1,4,5,6-tetrahydropyrrolo [3,4-d] imidazol-2-yl)-1H-indazole**

Intermediate 4-6 (810.0 mg, 1.20 mmol) was dissolved in 20 ml DCM, to which $ZnBr_2$ (1.1 g, 4.70 mmol) was added and the mixture was stirred at room temperature. After the reaction was completed, it was quenched with water, the mixture was extracted with DCM, and the resulting organic phase was washed with saturated brine, dried over anhydrous sodium sulfate, and concentrated to give 660 mg of Intermediate 4-7 in a yield of 95.2 %.

**Synthesis of compound 4: 5-(1-(3,5-difluorophenyl)ethoxy)3-(5-(1-methylpiperidin-4-yl)-1,4,5,6-tetrahydropyrrolo[3,4-d]imidazol-2-yl)-1H-indazole**

Intermediate 4-7 (15.0 mg, 0.03 mmol) and 1-methylpiperidin-4-one (4.3 mg, 0.04 mmol), were dissolved in 5 ml of DCM with stirring at room temperature for 10 min. Sodium triacetoxyborohydride (8.01 mg, 0.04 mmol) was added, and the mixture was stirred at room temperature for 3 hours. After completion of the reaction, it was quenched with water, the mixture was extracted with DCM, and the resulting organic phase was washed with saturated saline, concentrated and purified on silica gel plate. The purified product obtained was dissolved in 2 ml of methanol, to which 1 ml of concentrated hydrochloric acid was added, and it was allowed to react at 50°C for 5 h. The product was concentrated, and the concentrate was dissolved in 5 ml of methanol, to which 0.5 ml of ammonia was added, and the mixture was concentrated, and purified by preparative plate to give the final product of 1.9 mg in a two-step overall yield of 15.8 %.
1H NMR (400 MHz, CD3OD) δ7.67 (d, J = 4.0 Hz, 1H), 7.50 (d, J = 8.0 Hz, 1H), 7.19 (dd, J = 4.0 Hz, J = 8.0 Hz, 1H), 7.10 (dd, J = 4.0 Hz, J = 8.0 Hz, 2H), 6.84-6.79 (m, 1H), 5.61-5.56 (m, 1H), 4.36 (s, 4H), 3.71-3.59 (m, 2H), 3.19-3.15 (m, 3H), 2.93 (s, 3H), 2.48-2.47 (m, 2H), 2.06-2.02 (m, 2H), 1.67 (d, J = 8.0 Hz, 3H).

**Example 5: 2-(5-(1-(3,5-difluorophenyl)ethoxyl-1H-indazol-3-yl)-N,N-dimethyl-4,6-dihydropyrrolo[3,4-d]imidazole-5(1H) formamide**

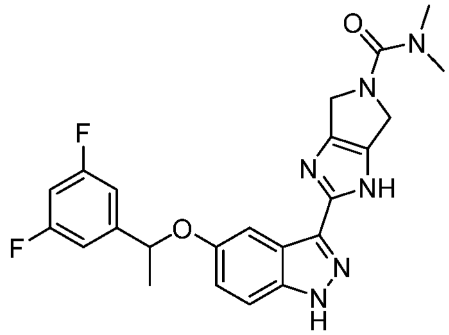

5

**Synthetic route for compound 5:**

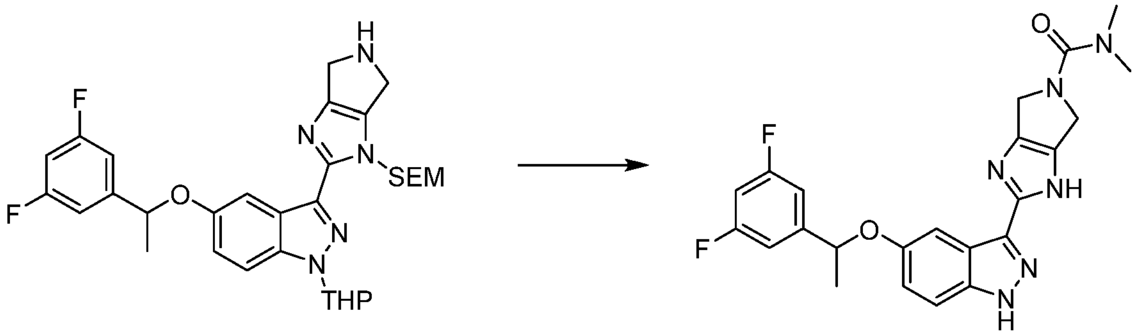

4-7 5

**Synthesis Method:**

**Synthesis of compound 5: 2-(5-(1-(3,5-difluorophenyl)ethoxy)-1H-indazol-3-yl)-N,N-dimethyl-4,6-dihydropyrrolo[3,4-d]imidazole-5(1H) formamide**

Intermediate 4-7 (30.0 mg, 0.05 mmol) was dissolved in 10 ml of dichloromethane, to which triphosgene (15.3 mg, 0.05 mmol) was added, and the system was cooled down to 0°C. Triethylamine (10.4 mg, 0.10 mmol) was added dropwise, and it was allowed to react for 0.5h. To the system dimethylamine hydrochloride (5.05 mg, 0.06 mmol) was added, and the mixture was warmed to room temperature. After completion of the reaction, it was quenched with water, the mixture was extracted with DCM, and the resulting organic phases were combined, washed with saturated saline and concentrated. The concentrate was dissolved in 4 ml of methanol, to which 2 ml of concentrated hydrochloric acid was added, and it was allowed to react at 50°C for 5 h. The reaction mixture was concentrated, and the concentrate was dissolved in 5 ml of methanol, to which 0.5 ml of ammonia was added, and then the resulting mixture was further concentrated, and purified by preparative plate to give the final product of 4.8 mg in a yield of 20.4%.

$^1H$ NMR (400 MHz, $CD_3OD$) δ7.69 (d, J = 2.3 Hz, 1H), 7.46 (d, J = 9.0 Hz, 1H), 7.17 (dd, J = 9.0, 2.4 Hz, 1H), 7.13-7.08 (m, 2H), 6.84-6.78 (m, 1H), 5.58 (q, J = 6.4 Hz, 1H), 4.71-4.08 (m, 2H). 1H), 4.71-4.63 (m, 4H), 2.99 (s, 6H), 1.66 (d, J = 6.4 Hz, 3H).

**Example 6: 2-(Dimethylamino)ethyl 2-(5-(1-(3,5-difluorophenyl)ethoxyl-1H-indazol-3-yl)-4,6-dihydropyrrolo[3,4-d]imidazole-5(1H)-carboxylate**

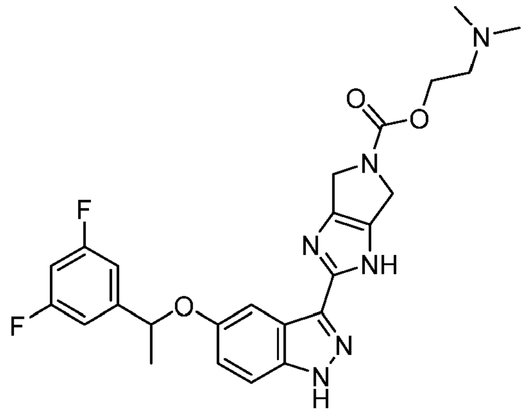

6

**Synthetic route for compound 6:**

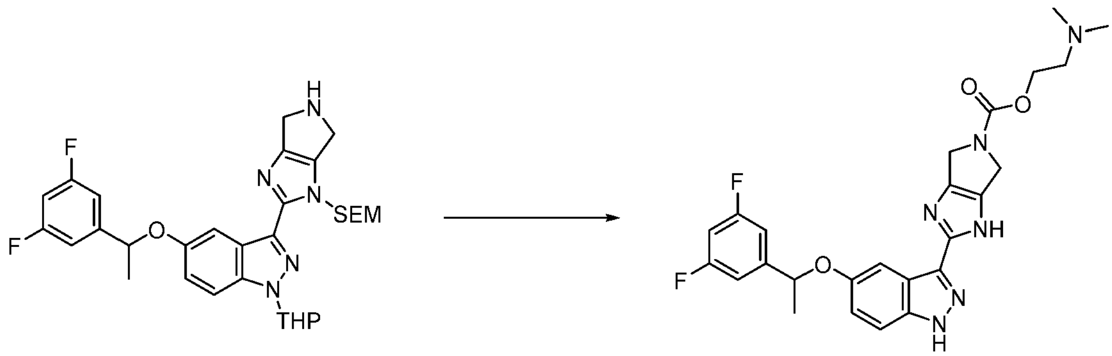

4-7

6

**Synthesis Method:**

**Synthesis of compound 6: 2-(dimethylamino)ethyl 2-(5-(1-(3,5-difluorophenyl)ethoxy)-1H-indazol-3-yl)-4,6-dihydropyrrolo[3,4-d]imidazole-5(1H)-carboxylate**

Intermediate 4-7 (50.0 mg, 0.08 mmol) was dissolved in THF, and cooled to 0oC, to which triphosgene (25.1 mg, 0.08 mmol) was added, and it was allowed to react for 5 min. Triethylamine (84.2 mg, 0.80 mmol) was added slowly, and it was allowed to react at room temperature for 0.5 h. To the reaction solution was added 2-(dimethylamino)ethan-1-ol (38.1 mg , 0.42 mmol) and DMAP (5.0 mg, 0.04 mmol), and the reaction solution was heated to 60°C and it was allowed to reactfor 1.5 h. Water was added to the reaction solution, the mixture was extracted with EA 2 times, and the resulting organic phases were combined, washed with saturated saline, dried over anhydrous sodium sulfate, concentrated, and purified by silica gel plate. The purified concentrate was dissolved in 2 ml of methanol, to which 1 ml of concentrated hydrochloric acid was added and it was allowed to react for 6 h at 50°C. The mixture was concentrated, and the concentrate was dissolved in 3 ml of methanol, to which 0.5 ml of ammonia was added, and the mixture was concentrated, purified on silica gel plate to give 15 mg of the final product in a yield of 35.0 %.

$^1$H NMR (400 MHz, $CD_3OD$) δ 7.68 (d, J = 4.0 Hz, 1H), 7.46 (d, J = 8.0 Hz, 1H), 7.20-7.14 (m, 1H), 7.14-7.08 (m, 2H), 6.85-6.78 (m, 1H), 5.58 (q, J = 8.0 Hz, 1H), 4.72-4.50 (m, 4H), 4.35 (t, J = 8.0 Hz, 2H), 2.82 -2.74 (m, 2H), 2.41 (s, 6H), 1.66 (d, J = 8.0 Hz, 3H).

**Example 7: (2-(5-(1-(3,5-difluorophenyl)ethoxy)-1H-indazol-3-yl)-4,6-dihydropyrrolo[3,4-d]imidazol-5(1H)-yl)(1-methylpiperidin-4-yl)ketone**

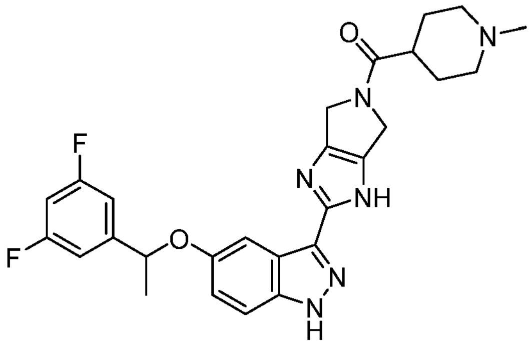

7

**Synthetic route for compound 7**

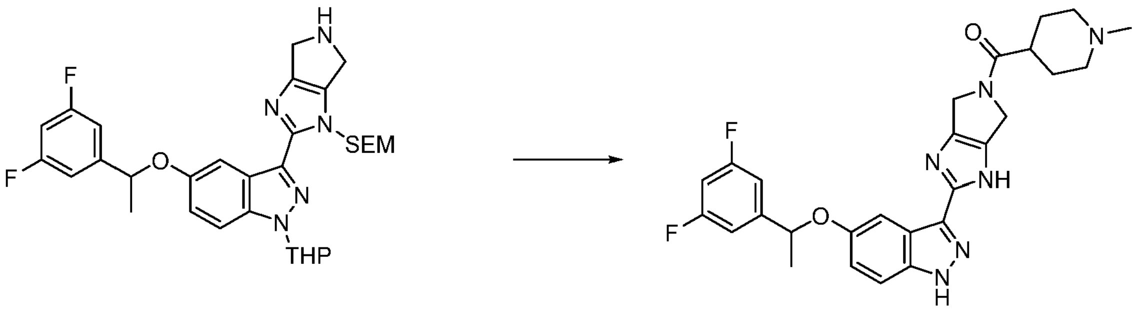

4-7

7

**Synthesis Method:**

**Synthesis of compound 7: (2-(5-(1-(3,5-difluorophenyl)ethoxy)-1H-indazol-3-yl)-4,6-dihydropyrrolo[3,4-d]imidazol-5(1H)-yl)(1-methylpiperidin-4-yl)ketone**

At room temperature, Intermediate 4-7 (30.0 mg, 0.05 mmol), HATU (29.1 mg, 0.10 mmol), N,N-diisopropylethylamine (13.2 mg, 0.10 mmol) and 1-methylpiperidine-4-carboxylic acid (14.0 mg, 0.10 mmol) were dissolved in 20 ml of DCM and it was allowed to react with stirring for 2 h. The reaction was quenched with water. The mixture was extracted with DCM twice, and the resulting organic phases were combined, washed with saturated brine, dried over anhydrous sodium sulfate and concentrated. The obtained product was dissolved in 2 ml methanol, to which 1 ml of concentrated hydrochloric acid was added, and it was allowed to react at 50°C for 2 h. The reaction mixture was concentrated, and the concentrate was dissolved in 3 ml methanol, to which 1 ml of ammonia was added, and the resulting mixture was further concentrated, and purified by preparative plate to give the final product of 8 mg, in a yield of 43.7%.

$^1H$ NMR (400 MHz, $CD_3OD$) δ 7.67 (d, J = 1.8 Hz, 1H), 7.45 (d, J = 9.0 Hz, 1H), 7.21-7.03 (m, 3H), 6.78 (t, J = 9.1 Hz, 1H), 5.55 (q, J = 6.2 Hz, 1H), 4.80-4.59 (m, 4H). 3.17-3.14 (m, 2H), 2.75-2.65 (m, 1H), 2.48 (s, 3H), 2.46-2.41 (m, 2H), 1.95-1.90 (m, 4H), 1.63 (d, J = 6.4 Hz, 3H).

**Example 8: 2-(5-(1-(3,5-difluorophenyl)ethoxy)-1H-indazol-3-yl)-5-(1-methylpiperidin-4-yl)-4,5,6,7-tetrahydro-3H-imidazo[4,5-c]pyridine**

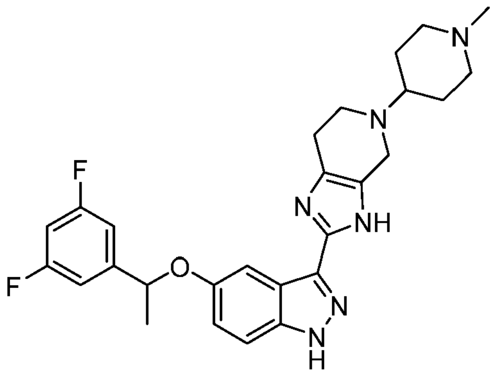

8

**Synthetic route for compound 8:**

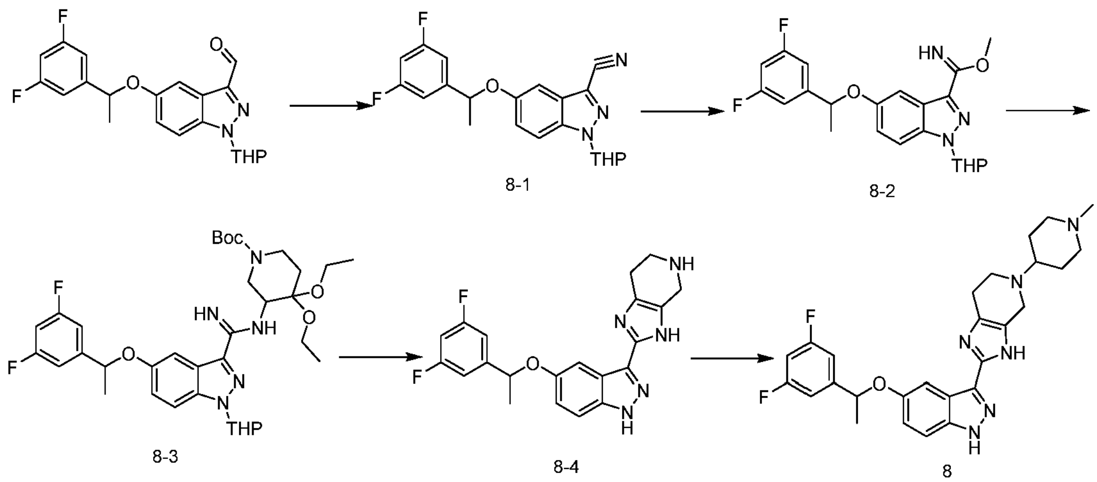

8-1 8-2

8-3 8-4 8

**Synthesis Method:**

**Synthesis of Intermediate 8-1: 5-(1-(3,5-difluorophenyl)-4-yl)ethoxy)-1-(tetrahydro-2H-pyran-2-yl)-1H-indazole-3-carbonitrile**

5-(1-(3,5-difluorophenyl)ethoxy)-1-(tetrahydro-2H-pyran-2-yl)-1H-indazole-3-formaldehyde (200 mg, 0.52 mmol) and triethylamine (157 mg, 1.55 mmol) were dissolved in 10 ml of acetonitrile, to which hydroxylamine hydrochloride (53.9 mg, 0.78 mmol) was added, and it was allowed to react at 60°C for 3 hours. The reaction solution was cooled to room temperature, to which triethylamine (419 mg, 4.14 mmol) and acetic anhydride (211 mg, 2.07 mmol) were added and it was allowed to react at 80°C for 16 hours. Water was added to the reaction solution, the mixture was extracted with dichloromethane twice, and the organic phases were combined, washed with saturated brine, dried over anhydrous sodium sulfate, concentrated and purified on silica gel column to give 145 mg of Intermediate 8-1 in a yield of 73.1 %.

$^1$H NMR (400 MHz, $CDCl_3$) δ 7.64-7.60 (m, 1H), 7.19-7.17 (m, 1H), 6.98-6.97 (m, 1H), 6.93-6.89 (m, 2H), 6.73-6.68 (m, 1H), 5.77-5.73 (m, 1H), 5.34 (q, J = 6.4 Hz , 1H), 3.94-3.88 (m, 1H), 3.76-3.71 (m, 1H), 2.49-2.41 (m, 1H), 2.14-2.06 (m, 2H), 1.77-1.68 (m, 3H),1.65 (d, J = 6.4 Hz, 3H).

**Synthesis of Intermediate 8-2: 5-(1-(3,5-difluorophenyl)ethoxy)-1-(tetrahydro-2H-pyran-2-yl)-1H-indazole-3-carbamate**

Intermediate 8-1 (135 mg, 0.35 mmol) was dissolved in 10 ml of methanol, to the reaction solution was added sodium methanol (57.1 mg, 1.06 mmol), and it was allowed to react at room temperature for 16 h. The reaction solution was concentrated to remove the methanol, and water was added to the concentrate, which was then extracted with ethyl

acetate two times, and the resulting organic phases were combined, washed with saturated brine, dried over anhydrous sodium sulfate and concentrated to give 120 mg of crude Intermediate 8-2, in a yield of 82.0 %.

**Synthesis of Intermediate 8-3: tert-butyl 3-(5-(1-(3,5-difluorophenyl)ethoxy)-1-(tetrahydro-2H-pyran-2-yl)-1H-indazole-3-amidino)-4,4-diethoxypiperidine-1-carboxylate**

Intermediate 8-2 (110 mg, 0.26 mmol) and tert-butyl 3-amino-4,4-diethoxypiperidine-1-carboxylate (91.6 mg, 0.32 mmol) were dissolved in 10 ml of ethanol, to which acetic acid (31.8 mg, 0.53 mmol) was added and it was allowed to react at 50°C for 16 h. The reaction solution was concentrated to remove the ethanol, and the residue was dissolved in an appropriate amount of toluene and was concentrated to dryness, thereby obtaining 208 mg of crude Intermediate 8-3.

**Synthesis of Intermediate 8-4: 2-(5-(1-(3,5-difluorophenyl)ethoxy)-1H-indazol-3-yl)-4,5,6,7-tetrahydro-3H-imidazo[4,5-c]pyridine**

Intermediate 8-3 (200 mg, 0.22 mmol) was dissolved in 10 ml of ethanol, to which 5 ml of concentrated hydrochloric acid was added, and it was allowed to react at 40°C for 16 h. The reaction mixture was concentrated, and the residue was dissolved in 5 ml of methanol, to which 0.5 ml of ammonia was added, and then the mixture was further concentrated, and purified by silica gel column to give 78 mg of Intermediate 8-4, in a yield of 66.2 %.

**Synthesis of compound 8: 2-(5-(1-(3,5-difluorophenyl)ethoxy)-1H-indazol-3-yl)-5-(1-methylpiperidin-4-yl)-4,5,6,7-tetrahydro-3H-imidazo [4,5-c] pyridine**

Intermediate 8-4 (15.0 mg, 0.04 mmol) was dissolved in 5 ml of NMP, to which 0.1 ml of acetic acid was added, the mixture was stirred at 55°C for 10 min, and then cooled to room temperature, and 1-methylpiperidin-4-one (4.3 mg, 0.04 mmol) was added. It was allowed to react at room temperature for 16 hr. Sodium triacetoxyborohydride (16.0 mg, 0.08 mmol) was added and it was allowed to react for 2 h at room temperature. pH was adjusted by adding 0.5 ml of ammonia, water was added to the mixture, and the resulting mixture was extracted with ethyl acetate twice, the resulting organic phases were combined, washed with saturated brine, dried over anhydrous sodium sulfate, concentrated, and purified by preparative plate to give a final product of 4.2 mg in a yield of 22.5 %.

$^1H$ NMR (400 MHz, $CD_3OD$) $\delta$7.68 (d, J = 2.4 Hz, 1H), 7.45 (d, J = 9.0 Hz, 1H), 7.16 (dd, J = 9.0, 2.4 Hz, 1H), 7.12-7.10 (m, 2H), 6.83-6.79 (m, 1H), 5.58 (q, J = 6.3 Hz, 1H), 3.80 (q, J = 6.3 Hz, 1H), 3.80 (q, J = 6.3 Hz, 1H), 4.2 mg (m, 2H). 1H), 3.80 (s, 2H), 3.17-3.10 (m, 2H), 3.04-3.01 (m, 2H), 2.84-2.80 (m, 2H), 2.73-2.70 (m, 1H), 2.43 (s, 3H), 2.36-2.19 (m, 2H), 2.10-2.03 (m, 2H), 1.84- 1.75 (m, 2H), 1.65 (d, J = 6.3 Hz, 3H).

**Example 9: (S)-5-(1-(3,5-difluorophenyl)ethoxyl-3-(5-(((1-methyl piperidin-4-yl)methyl)-1,4,5,6-tetrahydropyrrolo[3,4-d]imidazol-2-yl)-1H-indazole**

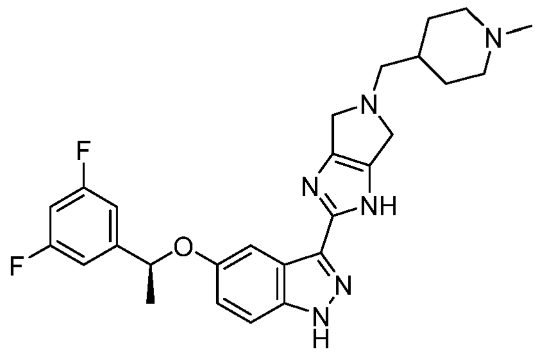

9

**Synthetic route for compound 9:**

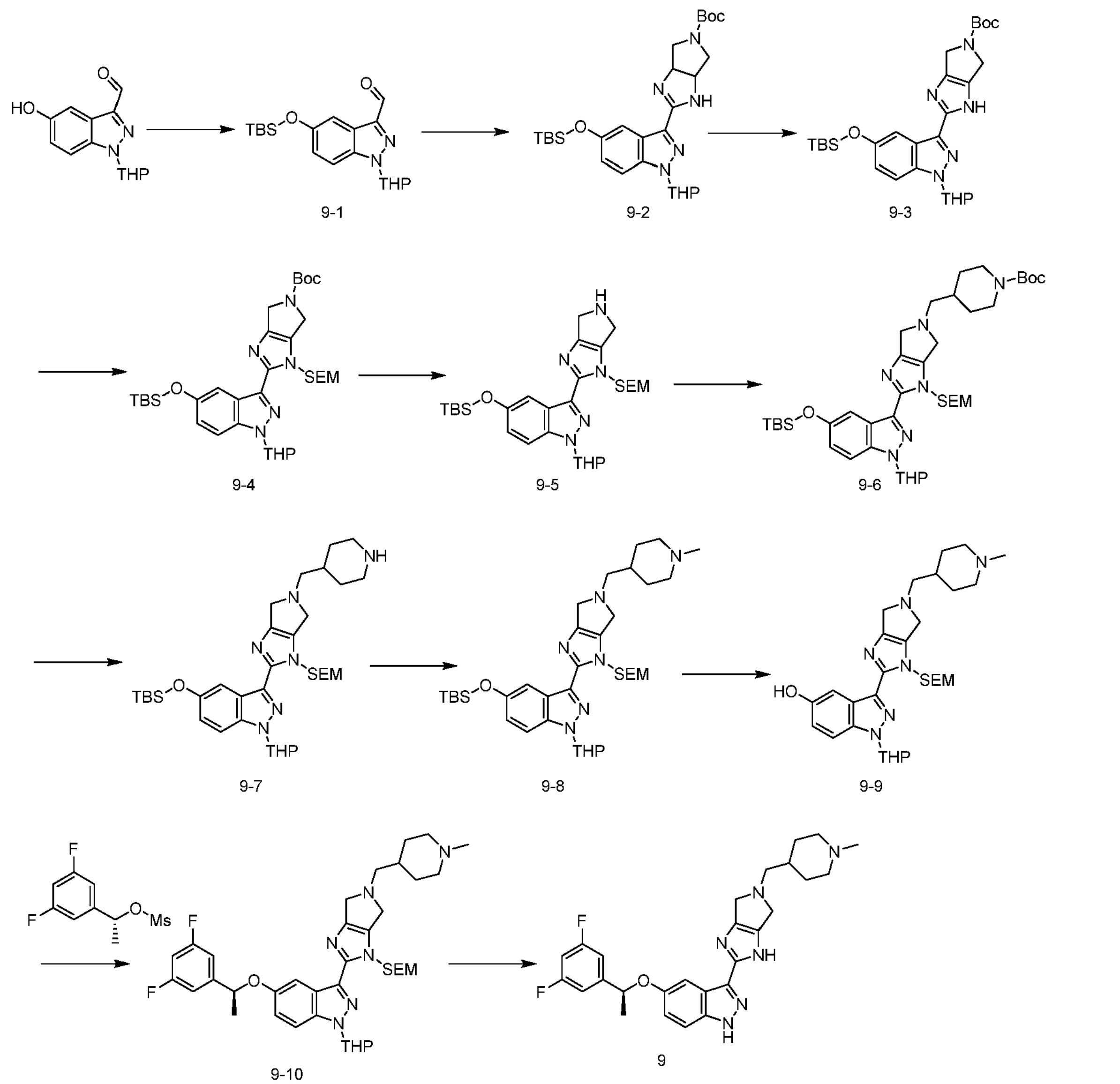

9-1 9-2 9-3 9-4 9-5 9-6 9-7 9-8 9-9 9-10 9

**Synthesis Method:**

**Synthesis of Intermediate 9-1: 5-((tert-butyldimethylsilyl)oxy)-1-(tetrahydro-2H-pyran-2-yl)-1H-indazole-3-carbaldehyde**

5-Hydroxy-1-(tetrahydro-2H-pyran-2-yl)-1H-indazole-3-formaldehyde (427 mg, 1.73 mmol) and imidazole (177 mg, 2.60 mmol) were dissolved in 20 ml of dichloromethane, to which TBSCl (0.33 ml, 1.91 mmol) was added dropwise at 0°C and after the addition, the mixture was transferred to room temperature and stirred for 1 hour so as to allow the reaction. Water was added to the reaction solution, the mixture was extracted with dichloromethane 2 times, and the resulting organic phases were combined, washed with saturated brine, dried over anhydrous sodium sulfate, and concentrated to obtain 540 mg of crude intermediate 9-1, in a yield of 86.4 %.

**Synthesis of Intermediate 9-2: tert-butyl 2-(5-((tert-butyldimethylsilyl) oxy)-1-(tetrahydro-2H-pyran-2-yl)-1H-indazol-3-yl)-3a,4,6,6a-tetrahydropyrrolo[3,4-d]imidazole-5(1H)-carboxylate**

Intermediate 9-1 (540 mg, 1.49 mmol) was dissolved in 20 ml of tert-butanol, to which tert-butyl 3,4-diaminopyrrolidine-1-carboxylate (362 mg, 1.79 mmol), iodine (570 mg, 2.25 mmol), and potassium carbonate (621 mg, 4.49 mmol) were added, and it was allowed to react at 70°C for 3 hours. After the reaction was completed, the temperature was lowered to room temperature, and the reaction was quenched by adding 5% aqueous sodium thiosulfate. The mixture was extracted with EA, and the resulting organic phase was washed with saturated brine, dried over anhydrous sodium

sulfate, concentrated, and purified by silica gel column, to give 530 mg of Intermediate 9-2 in a yield of 65.3 %.

**Synthesis of Intermediate 9-3: tert-butyl 2-(5-((tert-butyldimethyl silyl)oxy)-1-(tetrahydro-2H-pyran-2-yl)-1H-indazol-3-yl)-4,6-dihydropyrrolo[3,4-d]imidazole-5(1H)-carboxylate**

Intermediate 9-2 (530 mg, 0.99 mmol) was dissolved in 10 ml of DMSO, to which IBX (548 mg, 1.96 mmol) was added and it was allowed to react at 50°C for 3 hours. After the reaction was completed, it was reduced to room temperature, the reaction was quenched by adding water, the mixture was extracted with EA, and the resulting organic phase was washed with saturated brine, dried over anhydrous sodium sulfate, concentrated, and purified on silica gel column, to give 362 mg of Intermediate 9-3 in a yield of 68.4 %.

**Synthesis of Intermediate 9-4: tert-butyl 2-(5-((tert-butyldimethylsilyl) oxy)-1-(tetrahydro-2H-pyran-2-yl)-1H-indazol-3-yl)-1-((2-(trimethylsilyl)ethoxy) methyl)-4,6-dihydropyrrolo [3,4-d] imidazole-5(1H)-carboxylate**

Intermediate 9-3 (362 mg, 0.67 mmol) was dissolved in 10 ml of THF, and was cooled to 0°C, to which NaH (60% w/w, 32.1 mg, 1.33 mmol) was added and the mixture was stirred for 15 min. SEMCl (0.14 ml, 0.80 mmol) was added. After the addition was completed, the mixture was warmed to room temperature and stirred, and after the reaction was completed, it was quenched with water. The mixture was extracted with EA, and the resulting organic phase was washed with saturated brine, dried over anhydrous sodium sulfate, concentrated, and purified on silica gel column, to give 365 mg of Intermediate 9-4 in a yield of 81.5 %.

**Synthesis of Intermediate 9-5: 5-((tert-butyldimethylsilyl)oxy)-1-(tetrahydro-2H-pyran-2-yl)-3-(1-((2-(trimethylsilyl)ethoxy)methyl)-1,4,5,6-tetrahydropyrrolo [3,4-d] imidazol-2-yl)-1H-indazole**

Intermediate 9-4 (180 mg, 0.27 mmol) was dissolved in 10 ml DCM, to which $ZnBr_2$ (303 mg, 1.34 mmol) was added and the mixture was stirred at room temperature. After the reaction was completed, it was quenched with water, the mixture was extracted with DCM, and the resulting organic phase was washed with saturated brine, dried over anhydrous sodium sulfate, and concentrated to give 153 mg of Intermediate 9-5 in 99.9 % yield.

**Synthesis of Intermediate 9-6: tert-butyl 4-((2-(5-((tert-butyldimethyl silyl)oxy)-1-(tetrahydro-2H-pyran-2-yl)-1H-indazol-3-yl)-1-((2- (trimethylsilyl) ethoxy) methyl)-4,6-dihydropyrrolo[3,4-d]imidazol-5(1H)-yl)methyl)piperidine-1-carboxylate**

Intermediate 9-5 (153 mg, 0.27 mmol) and tert-butyl 4-formylpiperidine-1-carboxylate (68.7 mg, 0.32 mmol) were dissolved in 10 ml of dichloromethane and the mixture was stirred at room temperature for 1 h. Sodium cyanoborohydride (25.3 mg, 0.40 mmol) was added and the mixture was stirred for 1 h. After completion of the reaction, the reaction was quenched by adding water, the mixture was extracted with dichloromethane, and the resulting organic phase was washed with saturated saline, dried over anhydrous sodium sulfate, concentrated, and purified on silica gel column, to give 165 mg of Intermediate 9-6 in a yield of 80.1 %.

**Synthesis of Intermediate 9-7: 5-((tert-butyldimethylsilyl)oxy)-3-(5-(piperidin-4-ylmethyl)-1-((2-(trimethylsilyl)ethoxy)methyl)-1,4,5,6-tetrahydropyrrolo [3,4-d]imidazol-2-yl)-1-(tetrahydro-2H-pyran-2-yl)-1H-indazole**

Intermediate 9-6 (165 mg, 0.22 mmol) was dissolved in 10 ml DCM, to which $ZnBr_2$ (248 mg, 1.10 mmol) was added and the mixture was stirred at room temperature. After the reaction was completed, it was quenched with water, the mixture was extracted with DCM, and the resulting organic phase was washed with saturated brine, dried over anhydrous sodium sulfate, and concentrated to give 130 mg of Intermediate 9-7 in a yield of 90.6 %.

**Synthesis of compound 9-8: 5-((tert-butyldimethylsilyl)oxy)-3-(5-(((1-methylpiperidin-4-yl)methyl)-1-((2-(trimethylsilyl)ethoxy)methyl)-1,4,5,6 - tetrahydropyrrolo[3,4-d]imidazol-2-yl)-1-(tetrahydro-2H-pyran-2-yl)-1H-indazole**

Intermediate 9-7 (130 mg, 0.19 mmol) and 30% aqueous formaldehyde (17.5 mg, 0.58 mmol) were dissolved in 5 ml DCM and the mixture was stirred for 1 h. Sodium triacetylborohydride (80.5 mg, 0.38 mmol) was added to the solution. After the reaction was completed, it was quenched with water, the mixture was extracted with DCM, and the resulting organic phases were combined, washed with saturated saline, concentrated, and purified on silica gel column to give 75.5 mg of Intermediate 9-8 in a yield of 56.9 %.

**Synthesis of compound 9-9: 3-(5-((1-methylpiperidin-4-yl)methyl)-1-((2-(trimethylsilyl)ethoxy)methyl)-1,4,5,6-tetrahydropyrrolo [3,4-d] imidazol-2-yl)-1-(tetrahydro-2H-pyran-2-yl)-1H-indazol-5-ol**

Intermediate 9-8 (40.0 mg, 0.06 mmol) was dissolved in 5 ml of tetrahydrofuran and was cooled to 0°C. To the solution was added a solution of TBAF (0.12 ml, 0.12 mmol) in tetrahydrofuran solution (1M), the mixture was stirred for 30 minutes, and the reaction was completed. It was quenched by adding water, the mixture was extracted with ethyl acetate, and the resulting organic phases were combined, washed with saturated brine, and concentrated to give 30.1 mg of crude intermediate 9-9 in a yield of 90.1 %.

**Synthesis of Intermediate 9-10: 5-((S)-1-(3,5-difluorophenyl)ethoxy)-3-(5-(((1-methylpiperidin-4-yl)methyl)-1-((2-(trimethylsilyl)ethoxy)methyl)-1,4,5,6-tetrahydropyrrolo[3,4-d]imidazol-2-yl)-1-(tetrahydro-2H-pyran-2-yl)-1H-indazole**

Intermediate 9-9 (30.1 mg, 0.05 mmol) and ethyl (R)-1-(3,5-difluorophenyl)methanesulfonate (12.5 mg, 0.05 mmol) were dissolved in 10 ml of DMF, to which cesium carbonate (35.8 mg, 0.11 mmol) was added, and it was allowed to react at 60°C for 2 hrs. Water was added to the reaction solution, the mixture was extracted with ethyl acetate 2 times, and the organic phases were combined, washed with saturated brine, dried over anhydrous sodium sulfate, concentrated, and purified by silica gel column to obtain 19.2 mg of Intermediates 9-10 in a yield of 51.3 %.

**Synthesis of compound 9: (S)-5-(1-(3,5-difluorophenyl)ethoxy)-3-(5-(((1-methylpiperidin-4-yl)methyl)-1,4,5,6-tetrahydropyrrolo[3,4 -d]imidazol-2-yl)-1H-indazole**

Intermediate 9-10 (19.2 mg, 0.02 mmol) was dissolved in 4 ml of methanol, to which 2 ml of concentrated hydrochloric acid was added, and it was allowed to react at 50°C for 5 h. The reaction mixture was concentrated, and the residue was dissolved in 5 ml of methanol, to which 0.5 ml of ammonia was added, and the resulting mixture was further concentrated, and purified by preparative plate to give the final product of 6.2 mg in a yield of 46.3 %.

1H NMR (400 MHz, CD3OD) δ 7.64 (s, 1H), 7.46 (d, J = 9.0 Hz, 1H), 7.15 (dd, J = 9.1, 2.4 Hz, 1H), 7.10-7.05 (m, 2H), 6.81-6.76 (m, 1H), 5.55 (q, J = 6.4 Hz, 1H), 4.38 ( s, 4H), 3.56 (d, J = 12.4 Hz, 2H), 3.27-3.20 (m, 2H), 3.16-3.06 (m, 2H), 2.89 (s, 3H), 2.21-2.18 (m, 3H), 1.76-1.62 (m, 5H).

**Example 10: 1-Methylpiperidin-4-yl-2-(5-(1-(3,5-difluorophenyl)ethoxyl-1H-indazol-3-yl)-4,6-dihydropyrrolo[3,4-dlimidazole-5(1H)-carboxylate**

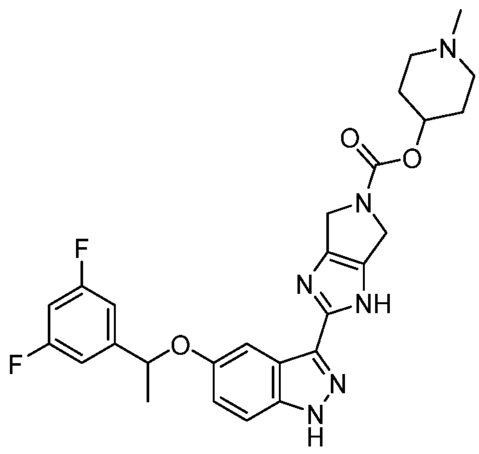

10

**Route of synthesis for compound 10:**

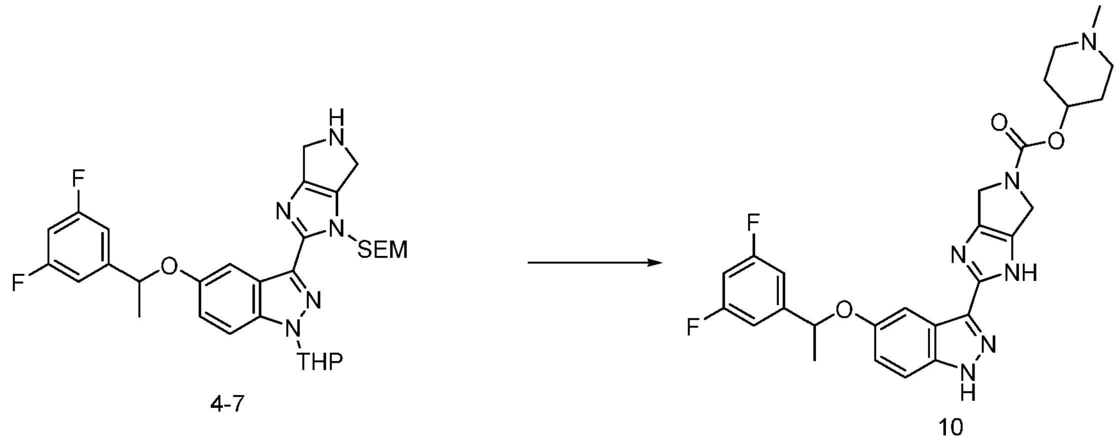

4-7

10

**Synthesis Method:**

**Synthesis of compound 10: 1-methylpiperidin-4-yl-2-(5-(1-(3,5-difluorophenyl)ethoxy)-1H-indazol-3-yl)-4,6-dihydropyrrolo[3,4-d]imidazole-5(1H)-carboxylate**

1-Methylpiperidin-4-ol (11.0 mg, 0.10 mmol) was dissolved in 10 ml of DCM, and was cooled to 0°C, to which triphosgene (15.0 mg, 0.05 mmol) was added and it was allowed to react for 5 min. Triethylamine (41.2 mg, 0.40 mmol) was added dropwise to the solution and the mixture was stirred for 0.5 h. To the reaction solution, intermediate 4-7 (30.0 mg, 0.05 mmol) was added and it was allowed to react for 16 h. The reaction was quenched with water, the mixture was extracted twice with DCM, and the resulting organic phases were combined, washed with saturated brine, dried over anhydrous sodium sulfate and concentrated. The obtained product was dissolved in 2 ml methanol and 1 ml concentrated hydrochloric acid, and it was allowed to react at 50°C for 2 h. The reaction mixture was concentrated, and the concentrate was dissolved in 3 ml of methanol, to which 1 ml ammonia was added, and the resulting mixture was further concentrated, and purified by preparative plate to give 2.2 mg of the final product, in a yield of 17%.

$_1$H NMR (400 MHz, $CD_3OD$) δ 7.69 (d, J = 2.2 Hz, 1H), 7.47 (d, J = 9.1 Hz, 1H), 7.18-7.09 (m, 3H), 6.83-6.79 (m, 1H), 5.58 (q, J = 6.2 Hz, 1H), 4.87-4.83 (m, 1H), 4.63-4.58 (m, 4H), 2.87-2.79 (m, 2H), 2.62-2.52 (m, 2H), 2.43 (s, 3H), 2.08-2.02 (m, 2H), 1.93-1.84 (m, 2H), 1.66 (d, J = 6.4 Hz, 3H).

**Example 11: N-(1-(3,5-difluorophenyl)ethyl)-3-(5-(piperidin-4-ylmethyl)-1,4,5,6-tetrahydropyrrolo[3,4-dlimidazol-2-yl)-1H-indazol-5-amine**

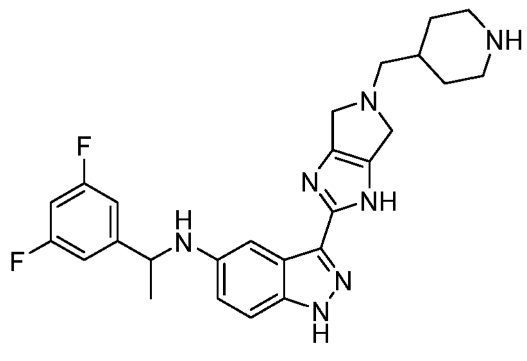

11

Synthetic route for compound 11:

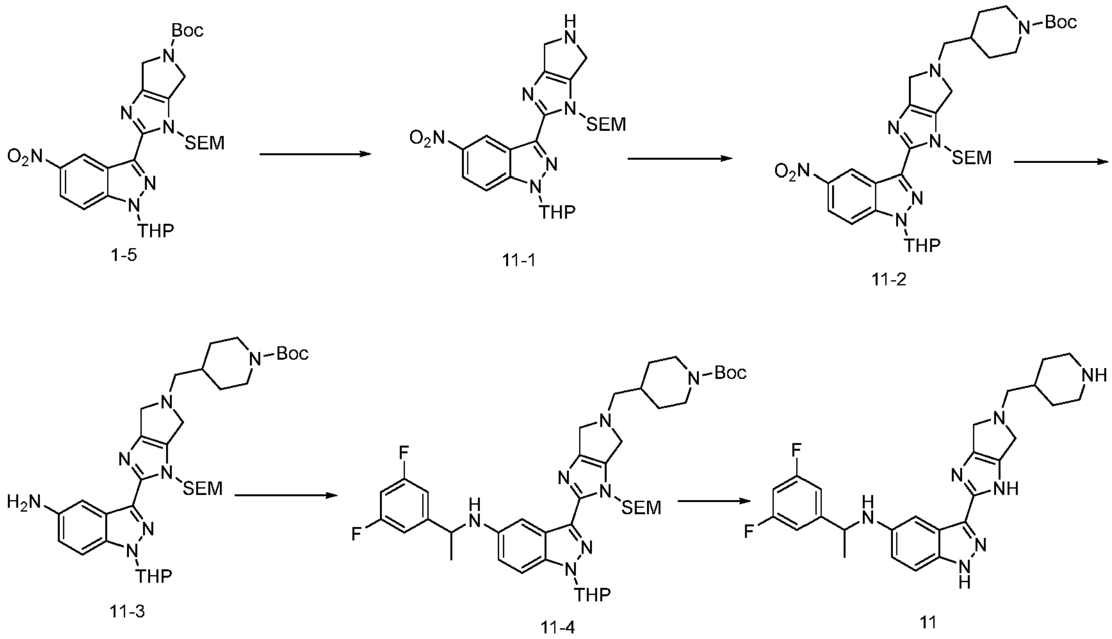

1-5 11-1 11-2 11-3 11-4 11

**Synthesis Method:**

**Synthesis of Intermediate 11-1: 5-nitro-1-(tetrahydro-2H-pyran-2-yl)-3-(1-((2-(trimethylsilyl)ethoxy)methyl)-1,4,5,6-tetrahydropyrrolo[3,4-d ]imidazol-2-yl)-1H-indazole**

Intermediate 1-5 (1.40 g, 2.39 mmol) was dissolved in 10 ml DCM, to which zinc bromide (1.08 g, 4.79 mmol) was added, the atmosphere was replaced with nitrogen and it was allowed to react for 4 h at room temperature. After the reaction was completed, it was quenched with water, the mixture was extracted with DCM, and the resulting organic phase was washed with saturated brine, dried over anhydrous sodium sulfate, concentrated, and purified on silica gel column to obtain 780 mg of Intermediate 11-1 in a yield of 67.21%. LC-MS m/z (ESI) [M+H]+ for $C_{23}H_{33}NeO_4Si$ was calculated as: 484.2; Measured as: 484.2.

**Synthesis of Intermediate 11-2: tert-butyl 4-((2-(5-nitro-1-(tetrahydro-2H-pyran-2-yl)-1H-indazol-3-yl)-1-((2-(trimethylsilyl)ethoxy)methyl)-4,6-dihydropyrrolo[3,4-d]imidazol-5(1H)-yl)methyl)piperidine-1-carboxylate**

Intermediate 11-1 (780 mg, 1.61 mmol) was dissolved in 10 ml of 1,2-dichloroethane, to which tert-butyl 4-formylpiperidine-1-carboxylate (686 mg, 3.22 mmol) was added and the mixture was stirred at room temperature for 2 h. Sodium triacetylborohydride (683 mg, 3.22 mmol) was added and the mixture was stirred at room temperature for 1 h. The reaction was quenched with water, the mixture was extracted with DCM and the resulting organic phase was washed with saturated saline, dried over anhydrous sodium sulfate, concentrated, and purified on silica gel column to obtain 650 mg of Intermediate 11-2 in a yield of 59.01%. LC-MS m/z (ESI) [M+H]+ for $C_{34}H_{52}N_7O_6Si$ was calculated as: 682.4; Measured as: 682.4.

**Synthesis of Intermediate 11-3: tert-butyl 4-((2-(5-amino-1-(tetrahydro-2H-pyran-2-yl)-1H-indazol-3-yl)-1-((2-(trimethylsilyl)ethoxy)methyl)-4,6-dihydropyrrolo[3,4-d]imidazol-5(1H)-yl)methyl)piperidine-1-carboxylate**

Intermediate 11-2 (200 mg, 0.29 mmol) was dissolved in 5 ml of ethyl acetate, to which palladium carbon (10% w/w, 100 mg) was added, the atmosphere was repaced with hydrogen gas, and it was allowed to react at room temperature under the atmosphere of hydrogen gas for 18 h. After 18 h, the reaction mixture was filtered, concentrated, and purified on a silica gel column to give 110 mg of Intermediate 11-3, in a yield of 57.49%. LC-MS m/z (ESI) [M+H]+ for $C_{34}H_{54}N_7O_4Si$ was calculated as: 652.4; Measured as: 652.4.

**Synthesis of Intermediate 11-4: tert-butyl 4-((2-(5-((1-(3,5-difluorophenyl) ethyl)amino)-1-(tetrahydro-2H-pyran-2-yl)-1H-indazol-3-yl)-1-((2-(trimethylsilyl) ethoxy)methyl)-4,6-dihydropyrrolo[3,4-d]imidazol-5(1H)-yl)methyl)piperidine-1-carboxylate**

Intermediate 11-3 (110 mg, 0.17 mmol) was dissolved in 5 ml of toluene, to which 1-(3,5-difluorophenyl)ethan-1-one (52.6 mg, 0.34 mmol) was added, and it was allowed to react at 90 °C for 18 h. The reaction mixture was cooled to 40 °C, to which sodium cyanoborohydride (31.8 mg, 0.51 mmol) was added, and it was allowed to react for 4 h. The reaction was quenched by adding water, the mixture was extracted with EA, and the resulting organic phase was washed with water, dried, concentrated and purified on silica gel column to give 36 mg of intermediate 11-4 in a yied of 27.0%. LC-MS m/z (ESI) [M+H]+ for $C_{42}H_{60}F_2N_7O_4Si$ was calculated as 792.4; measured as 792.4.

**Synthesis of compound 11: N-(1-(3,5-difluorophenyl)ethyl)-3-(5-(piperidin-4-ylmethyl)-1,4,5,6-tetrahydropyrrolo[3,4-d]imidazol-2-yl)-1H-indazol-5-amine**

Intermediate 11-4 (36 mg, 0.05 mmol) was dissolved in 4 ml of methanol, then 2 ml of concentrated hydrochloric acid was added, and it was allowed to react at 50°C for 3 h. The reaction mixture was concentrated, the concentrate was dissolved in 5 ml of methanol, to which 0.5 ml of ammonia was added, and the resulting mixture was furether concentrated, and purified by preparative plate to give 7 mg of final product with a yield of 32.2%. LC-MS m/z (ESI) [M+H]+ for $C_{26}H_{30}F_2N_7$ was calculated as: 478.3; Measured as: 478.3.

**Example 12:**

The compound of Example 12 was prepared according to the synthetic route of Example 11 using suitable starting materials as follows.

| No. | Structure Formula and Name of Compound | LC-MS Theoretical Calculated values (M+1)$^+$ | LC-MS Measured values (M+1)$^+$ |
|---|---|---|---|
| 12 | 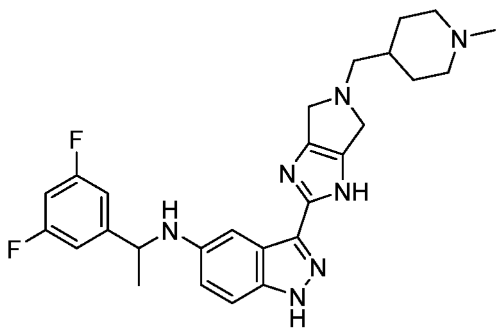 12 N-(1-(1-,3,5-Difluorophenyl)ethyl)-3-(5-(((1-methylpiperidin-4-yl)methyl)-1,4,5,6-tetrahydropyrrolo[3,4-d]imidazol-2-yl)-1H-indazol-5-amine | 492.3 | 492.3 |

**Example 13 and Example 14:**

The compounds of Examples 13 and 14 were prepared according to the synthetic route of Example 13 using suitable starting materials as follows.

| No. | Structure Formula and Name of Compound | $^1$HNMR(400 MHz) |
|---|---|---|
| 13 | 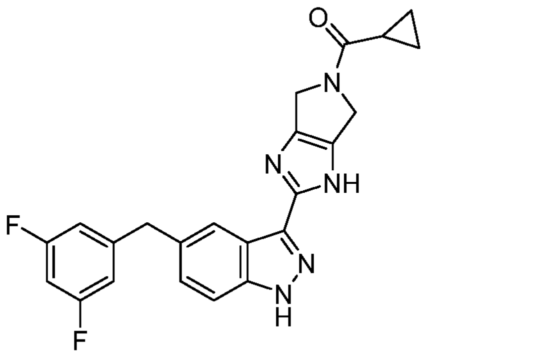 13 Cyclopropyl (2-(5-(3,5-difluorobenzyl) -1H-indazol-3 -yl)-4,6-dihydropyrrolo [3,4-d]imidazol-5(1H)-yl)ketone | (DMSO) δ 13.25 (d, $J$ = 4.0 Hz, 1H), 12.76 (d, $J$ = 24.0 Hz, 1H), 8.23 (d, $J$ = 4.0 Hz, 1H), 7.53 (d, $J$ = 8.0 Hz, 1H), 7.33 (d, $J$ = 12.0 Hz, 1H), 7.06-6.99 (m, 3H), 4.90 (s, 1H), 4.80 (s, 1H), 4.50 (s, 1H), 4.42 (s, 1H), 4.14 (s, 2H), 1.91-1.88 (m, 1H), 0.83-0.80 (m, 4H). |
| 14 | 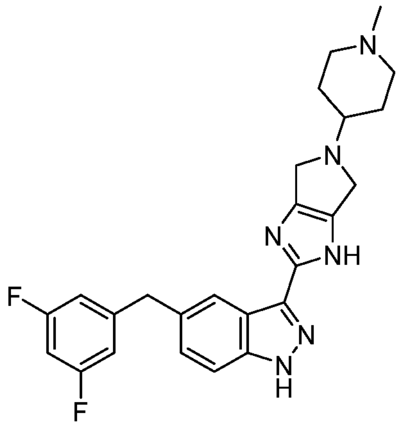 14 5-(3,5-Difluorobenzyl)-3-(5-(1-methyl piperidin-4-yl)-1,4,5,6-tetrahydro pyrrolo[3,4-d]imidazol-2-yl)-1H-indazole | ($CD_3OD$) δ 8.16 (s, 1H), 7.55 (d, $J$ = 8.0 Hz, 1H), 7.34 (d, $J$ = 8.0 Hz, 1H), 6.92-6.87 (m, 2H), 6.81-6.74 (m, 1H), 4.46 (s, 4H), 4.15 (s, 2H), 3.71-3.50 (m, 3H), 3.24-3.15 (m, 2H), 2.94 (s, 3H), 1.64-1.60(m, 4H). |

**Examples 15-28:**

The compounds of Examples 15-14 were prepared according to the synthetic route of Example 4 using suitable starting materials as follows.

| No. | Structure Formula and Name of Compound | $^1$HNMR(400 MHz) |
|---|---|---|
| **15** | 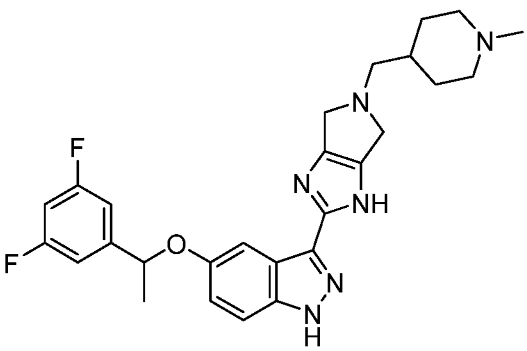 15 5-(1-(3,5-Difluorophenyl)ethoxy)-3-(5-(((1-methylpiperidin-4-yl)methyl)-1,4,5,6-tetrahydropyrrolo[3,4-d]imidazol-2-yl)-1H-indazole | ($CD_3OD$) δ 7.66 (d, $J$ = 4.0 Hz, 1H), 7.47 (d, $J$ = 8.0 Hz, 1H), 7.20-7.15 (m, 1H), 7.13-7.06 (m, 2H), 6.81 (d, $J$ = 2.4 Hz, 1H), 5.61-5.54 (m, 1H), 4.25 (s, 4H), 3.57 (s, 2H), 3.13 (d, $J$ = 8.0 Hz, 4H), 2.91 (s, 3H), 2.25-2.02 (m, 3H), 1.73 -1.52 (m, 5H). |

(continued)

| No. | Structure Formula and Name of Compound | $^{1}$HNMR(400 MHz) |
|---|---|---|
| **16** | 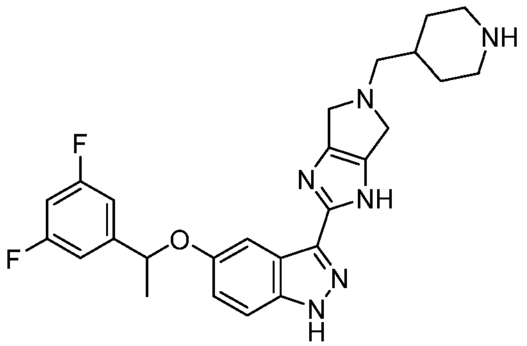<br>16 5-(1-(3,5-Difluorophenyl)ethoxy)-3-(5-(piperidin-4-ylmethyl)-1,4,5,6-tetrahydropyrrolo[3,4-d]imidazol-2-yl)-1H-indazole | ($CD_3OD$)8 7.68 (d, *J* = 4.0 Hz, 1H), 7.48 (d, *J* = 8.0 Hz, 1H), 7.21-7.15 (m, 1H), 7.13-7.07 (m, 2H), 6.85-6.78 (m, 1H), 5.64-5.56 (m, 1H), 4.67 - 4.54 (m, 4H), 4.14 (d, *J* = 8.0 Hz, 2H), 3.51-3.44 (m, 2H), 3.11-3.02 (m, 2H), 2.06 (m, 3H), 1.71-1.62 (m, 5H). |
| **17** | 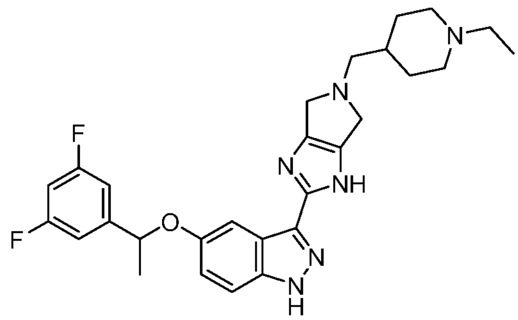<br>17 5-(1-(3,5-Difluorophenyl)ethoxy)-3-(5- (((1-ethylpiperidin-4-yl)methyl)-1,4,5,6-tetrahydropyrrolo[3,4-d]imidazol-2-yl)-1H-indazole | ($CD_3OD$) δ 7.66 (d, *J* = 2.1 Hz, 1H), 7.49 (d, *J* = 9.0 Hz, 1H), 7.18 (dd, *J* = 9.0, 2.3 Hz, 1H), 7.09 (d, *J* = 6.3 Hz, 2H), 6.85 - 6.78 (m, 1H), 5.57 (q, *J* = 6.4 Hz, 1H), 4.60 (s, 4H), 3.70-3.61 (m, 2H), 3.50-3.44 (m, 2H), 3.29-3.21 (m, 2H), 3.18- 3.27 (m, 2H), 2.37-2.24 (m, 3H), 1.81-1.72 (m, 2H), 1.65 (d, *J* = 6.4 Hz, 3H), 1.41 (t, *J* = 7.3 Hz, 3H). |
| **18** | 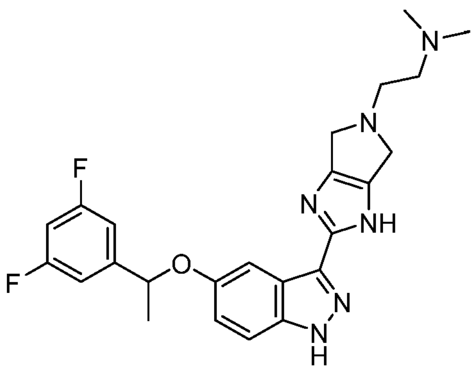<br>18 2-(2-(5-(1-(3,5-Difluorophenyl)ethoxy)-1H-indazol-3-yl)-4,6-dihydropyrrolo[3,4-d] imidazol-5(1H)-yl)-N,N-dimethyl ethan-1-amine | ($CD_3OD$) δ7.67 (d, *J* = 2.2 Hz, 1H), 7.46 (d, *J* = 9.1 Hz, 1H), 7.17 (dd, *J* = 9.1, 2.2 Hz, 1H), 7.13 -7.07 (m, 2H), 6.84-6.78 (m, 1H), 5.58 (q, *J* = 6.4 Hz, 1H), 4.03 (s, 4H), 3.67-3.60 (m, 4H), 2.35 (s, 6H), 1.66 (d, *J* = 6.4 Hz, 3H). |

(continued)

| No. | Structure Formula and Name of Compound | $^1$HNMR(400 MHz) |
|---|---|---|
| **19** | 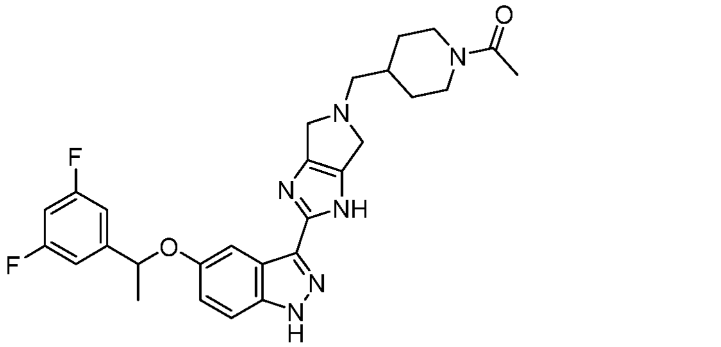 19 1-(4-((2-(5-(1-(3,5-Difluorophenyl) ethoxy)-1H-indazol-3-yl)-4,6-dihydropyrrolo[3,4-d] imidazol-5(1H)-yl)methyl)piperidin-1-yl)ethan-1-one | ($CD_3OD$)8 7.67 (d, *J* = 2.2 Hz, 1H), 7.46 (d, *J* = 9.0 Hz, 1H), 7.22-7.15 (m, 1H), 7.10 (d, *J* = 6.2 Hz, 2H), 6.87-6.76 (m, 1H), 5.59 (q, *J* = 6.3 Hz, 1H), 4.59-4.52 (m, 1H), 4.00-3.97(m, 1H), 3.95 (s, 4H), 3.22-3.14 (m, 1H), 2.81 (d, *J* = 6.7 Hz, 2H), 2.74-2.66 (m, 1H), 2.13 (s, 3H), 1.99-1.88 (m, 3H), 1.65 (d, *J* = 6.4 Hz, 3H), 1.26-1.14(m, 2H). |
| **20** | 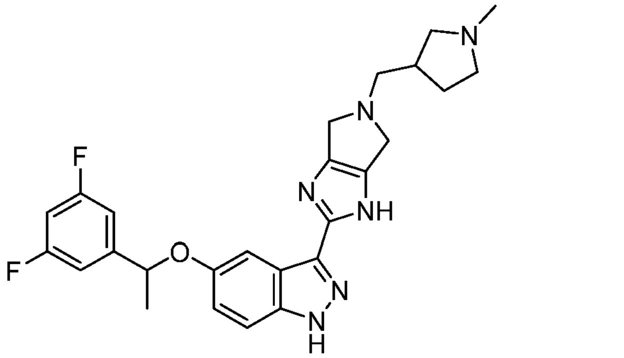 20 5-(1-(3,5-Difluorophenyl)ethoxy)-3-(5-(((1-methylpyrrolidin-3-yl)methyl)-1,4,5,6-tetrahydropyrrolo[3,4-d]imidazol-2-yl)-1H-indazole | ($CD_3OD$) $\delta$ 7.65 (d, *J* = 4 Hz, 1H), 7.49 (d, *J* = 8 Hz, 1H), 7.18 (dd, *J* = 8, 2 Hz, 1H), 7.09-7.11 (m, 2H), 6.79-6.84 (m, 1H), 5.56-5.61 (m, 1H), 4.45 -4.50 (m, 4H), 3.61- 3.84 (m, 2H), 3.49-3.56 (m, 2H), 3.35-3.44 (m, 2H), 2.98-3.09 (m, 4H), 2.42-2.52 (m, 1H), 1.97 -2.07 (m, 1H), 1.65 (d, *J* = 8 Hz, 3H). |
| **21** | 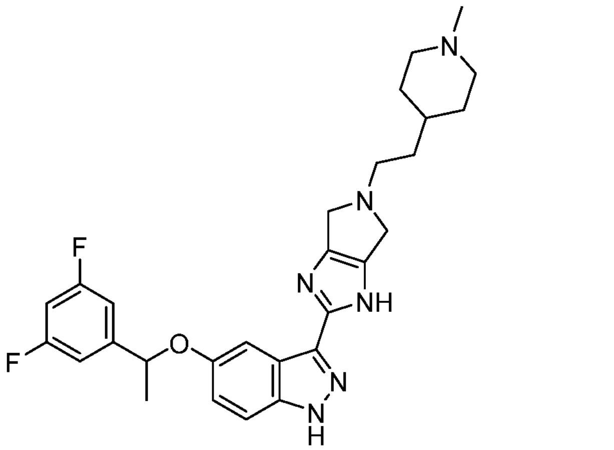 21 5-(1-(3,5-Difluorophenyl)ethoxy)-3-(5-(2-(1-methylpiperidin-4-yl)ethyl)-1,4,5,6-tetrahydropyrrolo[3,4-d]imidazol-2-yl)-1H-indazole | ($CD_3OD$)8 7.65 (d, *J* = 4.0 Hz, 1H), 7.49 (d, *J* = 8.0 Hz, 1H), 7.22-7.16 (m, 1H), 7.13-7.05 (m, 2H), 6.85-6.77 (m, 1H), 5.60-5.53 (m, 1H), 4.64 (s, 4H), 3.66-3.52 (m, 4H), 3.17-3.00 (m, 2H), 2.89 (s, 3H), 2.07 (d, *J* = 12.0 Hz, 2H), 1.89 (s, 3H), 1.65 (m, 5H). |

(continued)

| No. | Structure Formula and Name of Compound | $^1$HNMR(400 MHz) |
|---|---|---|
| 22 | 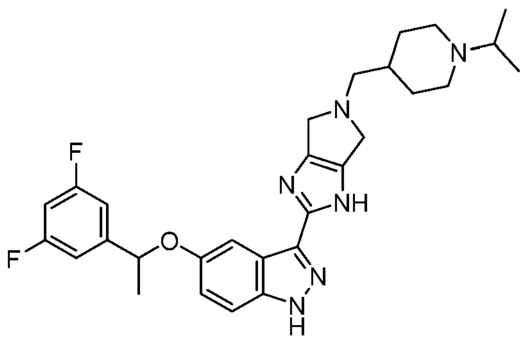 22 5-(1-(3,5-Difluorophenyl)ethoxy)-3-(5-(((1-isopropylpiperidin-4-yl)inethyl)-1,4,5,6-tetrahydropyrrolo[3,4-d]imidazol-2-yl)-1H-indazole | ($CD_3OD$) δ 7.65 (s, 1H), 7.44 (d, *J* = 9.0 Hz, 1H), 7.14 (dd, *J* = 9.0, 2.3 Hz, 1H), 7.10-7.05 (m, 2H), 6.81-6.75 (m, 1H), 5.56 (q, *J* = 6.4 Hz, 1H), 3.92 (s, 4H), 3.48-3.41 (m, 3H), 3.01 (t, *J* = 12.2 Hz, 2H), 2.80 (d, *J* = 7.1 Hz, 2H), 2.16 (d, *J* = 14.1 Hz, 2H), 1.98- 1.85 (m, 1H), 1.63 (d, *J* = 6.4 Hz, 3H), 1.55-1.46 (m, 2H), 1.35 (d, *J* = 6.6 Hz, 6H). |
| **23** | 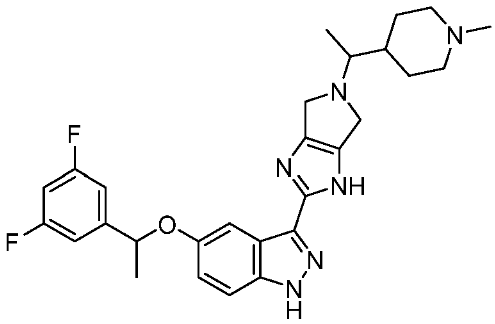 23 5-(1-(3,5-Difluorophenyl)ethoxy)-3-(5- (1-(1-methylpiperidin-4-yl)ethyl)-1,4,5,6-tetrahydropyrrolo[3,4-d]imidazol-2-yl)-1H-indazole | ($CD_3OD$) δ 7.66 (d, *J* = 4 Hz, 1H), 7.49 (d, *J* = 12 Hz, 1H), 7.19 (dd, *J* = 8, 2 Hz, 1H), 7.08-7.11 (m, 2H), 6.79- 6.85 (m, 1H), 5.55-5.60 (m, 1H), 4.40-4.53 (m, 4H), 3.58 -3.65 (m, 2H), 3.42- 3.51 (m, 1H), 3.11-3.18 (m, 2H), 2.92 (s, 3H), 2.19-2.26 (m, 1H), 2.09 -2.16 (m, 2H), 1.72- 1.82 (m, 2H), 1.66 (d, *J* = 8 Hz, 3H), 1.31 (s, 3H). |
| **24** | 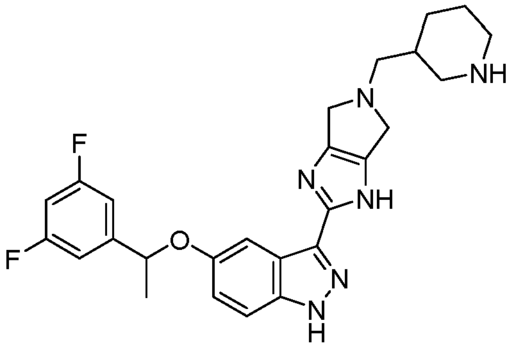 24 5-(1-(3,5-Difluorophenyl)ethoxy)-3-(5-(piperidin-3-ylmethyl)-1,4,5,6-tetrahydropyrrolo[3,4-d]imidazol-2-yl)-1H-indazole | ($CD_3OD$) δ 7.65 (d, *J* = 4 Hz, 1H), 7.45 (d, *J* = 8 Hz, 1H), 7.15 (dd, *J* = 8, 2 Hz, 1H), 7.06-7.10 (m, 2H), 6.76-6.81 (m, 1H), 5.53-5.58 (m, 1H), 4.01 -4.08 (m, 4H), 3.51- 3.67 (m, 1H), 3.38-3.41 (m, 1H), 2.90-2.99 (m, 3H), 2.77 (t, *J* = 12 Hz, 1H), 2.10-2.19 (m, 1H), 1.92-2.05 (m, 3H), 1.75 -1.85 (m, 1H), 1.63 (d, *J* = 8 Hz, 3H). |

(continued)

| No. | Structure Formula and Name of Compound | $^1$HNMR(400 MHz) |
|---|---|---|
| **25** | 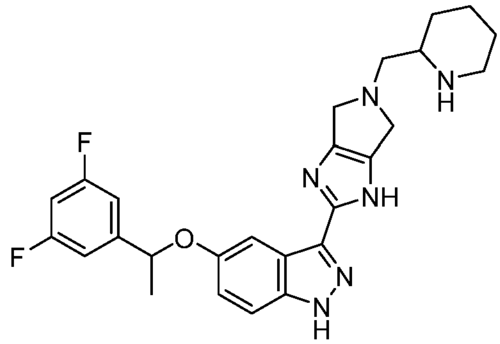 25 5-(1-(3,5-Difluorophenyl)ethoxy)-3-(5- (piperidin-2-ylmethyl)-1,4,5,6-tetrahydro pyrrolo[3,4-d]imidazol-2-yl)-1H-indazole | ($CD_3OD$) δ7.66 (d, *J* = 2.3 Hz, 1H), 7.47 (d, *J* = 9.0 Hz, 1H), 7.18 (dd, *J* = 9.0, 2.3 Hz, 1H), 7.13 -7.08 (m, 2H), 6.84-6.79 (m, 1H), 5.59 (q, *J* = 6.2 Hz, 1H), 4.12-3.96 (m, 4H), 3.42-3.37 (m, 2H), 3.15-3.11 (m, 1H), 3.07- 2.99 (m, 2H), 2.03-1.96 (m, 4H), 1.74-1.67 (m, 1H), 1.65 (d, *J* = 6.2 Hz, 3H), 1.53-1.44 (m, 1H). |
| **26** | 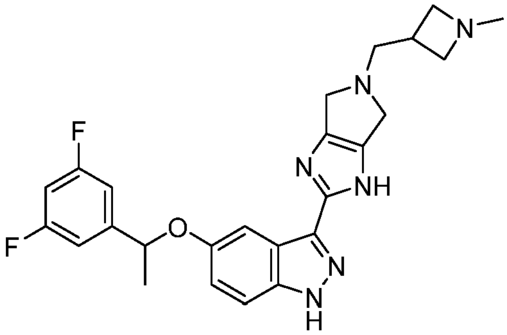 26 5-(1-(3,5-Difluorophenyl)ethoxy)-3-(5- ((1-methylazetidin-3-yl)methyl)-1,4,5,6-tetrahydropyrrolo[3,4-d]imidazol-2-yl)-1H-indazole | ($CD_3OD$) δ 7.64 (d, *J* = 4 Hz, 1H), 7.44 (d, *J* = 8 Hz, 1H), 7.15 (dd, *J* = 8, 2 Hz, 1H), 7.05-7.10 (m, 2H), 6.75-6.81 (m, 1H), 5.53-5.58 (m, 1H), 4.16 -4.24 (m, 2H), 3.89-3.98 (m, 4H), 3.36-3.42 (m, 2H), 3.12-3.17 (m, 3H), 2.90 (s, 3H), 1.63 (d, *J* = 8 Hz, 3H). |
| **27** | 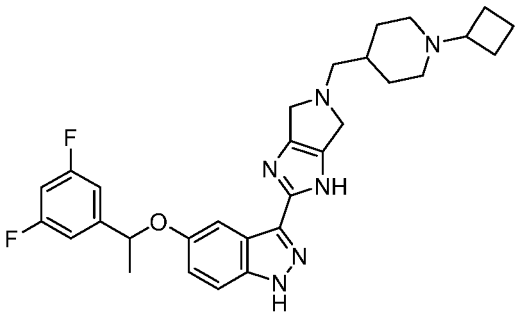 27 3-(5-((1-Cyclobutylpiperidin-4-yl)methyl)-1,4,5,6-tetrahydropyrrolo[3,4-d] imidazol-2-yl)-5-(1-(3,5-difluoro phenyl)ethoxy)-1H-indazole | (DMSO-$d_6$) δ 13.06 (s, 1H), 12.41 (s, 1H), 7.71 (s, 1H), 7.46 (d, *J* = 12.0 Hz, 1H), 7.21 (dd, *J* = 4.0 Hz, *J* = 8.0 Hz, 2H), 7.14-7.11 (m, 2H), 5.56 (q, *J* = 8.0 Hz, 1H), 3.77 -3.70 (m, 4H), 2.79- 2.77 (m, 2H), 2.64-2.62 (m, 2H), 2.03-1.91 (m, 3H), 1.78-1.75 (m, 5H), 1.64-1.62 (m, 2H), 1.59 (d, *J* = 8.0 Hz, 3H), 1.48 -1.46 (m, 2H), 1.13-1.10 (m, 2H). |

(continued)

| No. | Structure Formula and Name of Compound | $^1$HNMR(400 MHz) |
|---|---|---|
| **28** | 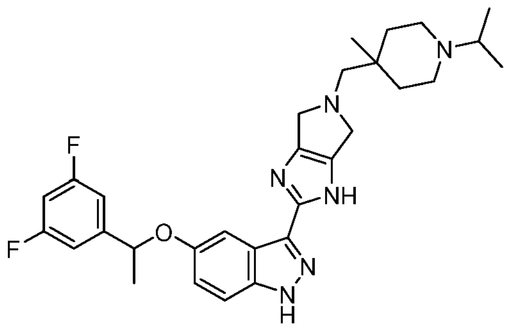 28 5-(1-(3,5-Difluorophenyl)ethoxy)-3-(5- (((1-isopropyl-4-methylpiperidin-4-yl) methyl)-1,4,5,6-tetrahydropyrrolo[3,4-d]imidazol-2-yl)-1H-indazole | ($CD_3OD$) δ 7.67 (d, *J* = 4 Hz, 1H), 7.47 (d, *J* = 8Hz, 1H), 7.17 (dd, *J* = 8, 2 Hz, 1H), 7.09-7.11 (m, 2H), 6.78-6.84 (m, 1H), 5.56-5.61 (m, 1H), 4.10 -4.15 (m, 4H), 3.50-3.55 (m, 1H), 3.38-3.45 (m, 2H), 3.17-3.24 (m, 2H), 2.87-2.92 (m, 2H), 1.93 -2.05 (m, 2H), 1.72-1.79 (m, 2H), 1.65 (d, *J* = 8 Hz, 3H), 1.42 (d, *J* = 8 Hz, 6H), 1.18 (s, 3H). |

**Example 29:**

The compound of Example 29 was prepared according to the synthetic route of Example 5 using suitable starting materials as follows.

| No. | Structure Formula and Name of Compound | $^1$HNMR(400MHz) |
|---|---|---|
| **29** | 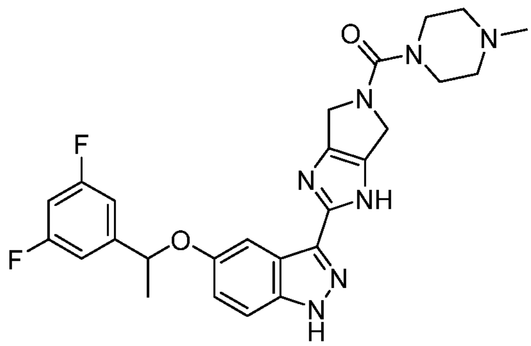 29 (2-(5-(1-(3,5-Difluorophenyl)ethoxy)-1H-indazol-3-yl)-4,6-dihydropyrrolo[3,4-d] imidazol-5(1H)-yl)(4-methylpiperazin-1-yl)ketone | ($CD_3OD$) δ 7.69 (d, *J* = 4 Hz, 1H), 7.47 (d, *J* = 12 Hz, 1H), 7.17 (dd, *J* = 8, 2 Hz, 1H), 7.09 -7.12 (m, 2H), 6.78 -6.84 (m, 1H), 5.56-5.61 (m, 1H), 4.65-4.70 (m, 4H), 3.45-3.48 (m, 4H), 2.63-2.65 (m, 4H), 2.43 (s, 3H), 1.66 (d, *J* = 6.4 Hz, 3H). |

**Examples 30-31:**

The compounds of Examples 30-31 were prepared according to the synthetic route of Example 6 using suitable starting materials as follows.

| No. | Structure Formula and Name of Compound | $^1$HNMR(400 MHz) |
|---|---|---|
| **30** | 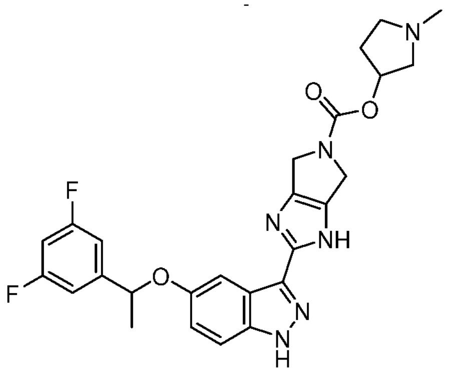 30 1-Methylpyrrolidin-3-yl 2-(5-(1-(3,5-difluorophenyl)ethoxy)-1H-indazol-3-yl)-4,6-dihydropyrrolo[3,4-d]imidazole-5(1H)-carboxylate | ($CD_3OD$) $\delta$ 7.69 (d, *J* = 4 Hz, 1H), 7.46 (d, *J* = 12 Hz, 1H), 7.16 (dd, *J* = 10, 2 Hz, 1H), 7.07-7.11 (m, 2H), 6.77-6.83 (m, 1H), 5.55-5.59 (m, 1H), 5.21-5.26 (m, 1H), 4.51-4.62 (m, 4H), 2.94-3.02 (m, 2H), 2.81-2.85 (m, 1H), 2.49-2.55 (m, 1H), 2.47 (s, 3H), 2.34 - 2.43 (m, 1H), 1.99-2.06 (m, 1H), 1.64 (d, *J* = 8 Hz, 3H). |
| **31** | 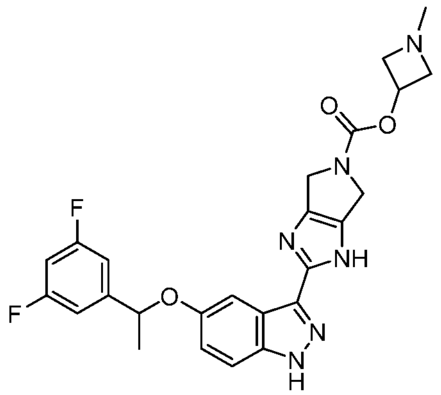 31 1-Methylazetidin-3-yl 2-(5-(1-(3,5-difluorophenyl)ethoxy)-1H-indazol-3-yl)-4,6-dihydropyrrolo[3,4-d]imidazole-5(1H)-carboxylate | ($CD_3OD$) $\delta$7.69 (d, *J* = 4.0 Hz, 1H), 7.47 (d, *J* = 8.0 Hz, 1H), 7.17 (dd, *J* = 4.0 Hz, *J* = 8.0 Hz, 1H), 7.11- 7.09 (m, 2H), 6.83 -6.78 (m, 1H), 5.60-5.55 (m, 1H), 5.18-5.15 (m, 1H), 4.72-4.59 (m, 4H), 3.87-3.78 (m, 2H), 3.01-2.90 (m, 2H), 2.47 (s, 3H), 1.66 (d, *J* = 8.0 Hz, 3H). |

**Example 32:**

The compound of Example 32 was prepared according to the synthetic route of Example 9 using suitable starting materials as follows.

| No. | Structure Formula and Name of Compound | $^1$HNMR(400MHz) |
|---|---|---|
| **32** | 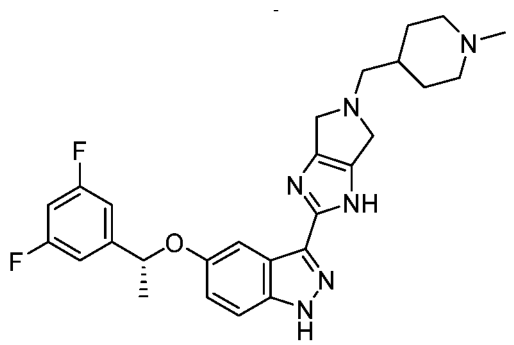 32 (R)-5-(1-(3,5-difluorophenyl)ethoxy)-3-(5-(((1-methylpiperidin-4-yl)methyl)-1,4,5,6-tetrahydropyrrolo[3,4-d]imidazol-2-yl)-1H-indazole | ($CD_3OD$) $\delta$ 7.66 (s, 1H), 7.47 (d, *J* = 9.0 Hz, 1H), 7.17 (dd, *J* = 9.0, 2.3 Hz, 1H), 7.14-7.05 (m, 2H), 6.84-6.78 (m, 1H), 5.58 (q, *J* = 6.3 Hz, 1H), 4.20 (s, 4H), 3.56 (d, *J* = 12.4 Hz, 2H), 3.17-3.08 (m, 4H), 2.91 (s, 3H), 2.21-2.07 (m, 3H), 1.66-1.53 (m, 5H). |

**Example 33:**

The compound of Example 33 was prepared according to the synthetic route of Example 10 using suitable starting materials as follows.

| No. | Structure Formula and Name of Compound | $^1$HNMR(400MHz) |
|---|---|---|
| **33** | 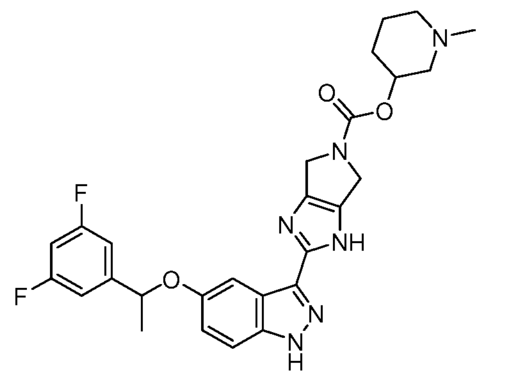 33 1-Methylpiperidin-3-yl 2-(5-(1-(3,5-difluorophenyl)ethoxy)-1H-indazol-3-yl)-4,6-dihydropyrrolo[3,4-d]imidazole-5 (1H)-carboxylate | ($CD_3OD$) δ7.67 (d, *J* = 2.3 Hz, 1H), 7.47 (d, *J* = 9.1 Hz, 1H), 7.17 (dd, *J* = 9.1, 2.3 Hz, 1H), 7.13-7.09 (m, 2H), 6.84-6.78 (m, 1H), 5.57 (q, *J* = 6.4 Hz, 1H), 5.12 -5.02 (m, 2H), 4.76-4.59 (m, 4H), 3.28-3.10 (m, 2H), 3.02-2.94 (m, 2H), 2.76(s, 3H), 2.21 -1.81 (m, 4H), 1.65 (d, *J* = 6.4 Hz, 3H). |

**Examples 34-46:**

The compounds of Examples 34-46 were prepared according to the synthetic route of Example 4 using suitable starting materials as follows.

| No. | Structure Formula and Name of Compound | LC-MS Theoretical Calculated values $(M+1)^+$ | LC-MS Measured values $(M+1)^+$ |
|---|---|---|---|
| 34 | 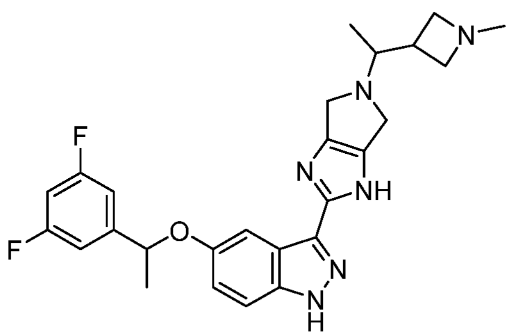 5-(1-(3,5-Difluorophenyl) ethoxy)-3-(5-(1-(1-methylazetidin-3-yl)ethyl)-1,4,5,6-tetrahydropyrrolo[3,4-d] imidazol-2-yl)-1H-indazole | 479.2 | 479.2 |

(continued)

| No. | Structure Formula and Name of Compound | LC-MS Theoretical Calculated values (M+1)+ | LC-MS Measured values (M+1)+ |
|---|---|---|---|
| 35 | 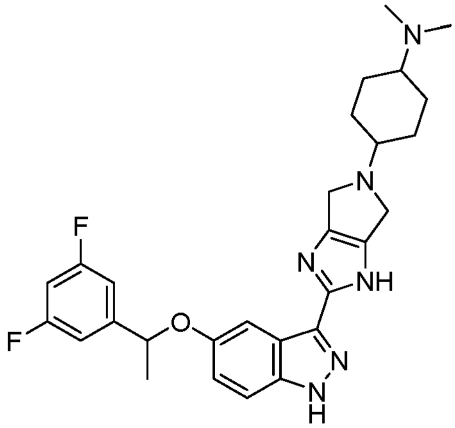 4-(2-(5-(1-(3,5-Difluorophenyl) ethoxy)-1H-indazol-3-yl)-4,6-dihydropyrrolo[3,4-d]imidazol-5(1H)-yl)-N,N-dimethyl cyclohexan-1-amine | 507.3 | 507.2 |
| 36 | 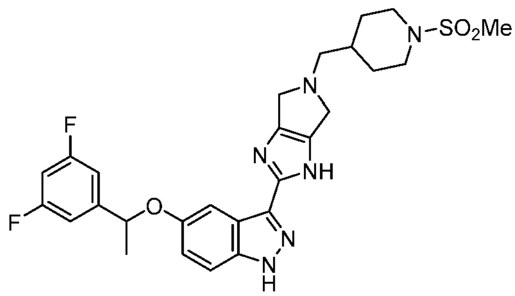 5-(1-(3, 5-Difluorophenyl) ethoxy)-3-(5-(1-(methylsulfonyl)piperidin-4-yl)methyl)-1,4,5,6-tetrahydropyrrolo[3,4-d]imidazol-2-yl)-1H indazole | 557.2 | 557.2 |
| 37 | 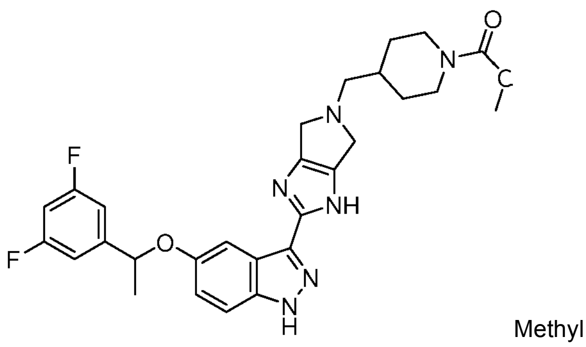 Methyl 4-((2-(5-(1-(3,5-difluorophenyl) ethoxy)-1H-indazol-3-yl)-4,6-dihydro pyrrolo[3,4-d]imidazol-5 (1H)-yl)methyl) piperidine-1-carboxylate | 537.2 | 537.3 |

(continued)

| No. | Structure Formula and Name of Compound | LC-MS Theoretical Calculated values (M+1)+ | LC-MS Measured values (M+1)+ |
|---|---|---|---|
| 38 | 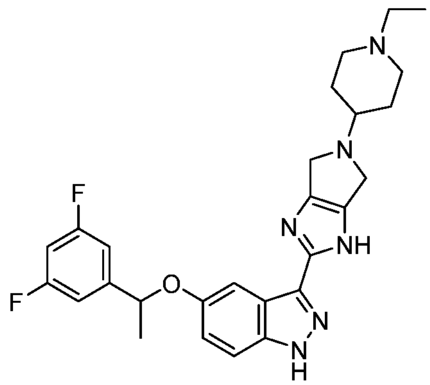 5-(1-(3,5-Difluorophenyl)ethoxy)-3-(5-(1-ethylpiperidin-4-yl)-1,4,5,6-tetrahydro pyrrolo[3,4-d]imidazol-2-yl)-1H indazole | 493.2 | 493.2 |
| 39 | 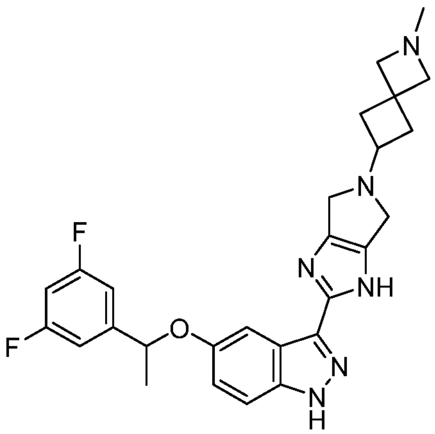 5-(1-(3,5-Difluorophenyl)ethoxy)-3-(5-(2-methyl-2-azaspiro[3.3]heptan-6-yl)-1,4,5,6-tetrahydropyrrolo[3,4-d]imidazol-2-yl)-1H-indazole | 491.2 | 491.2 |
| 40 | 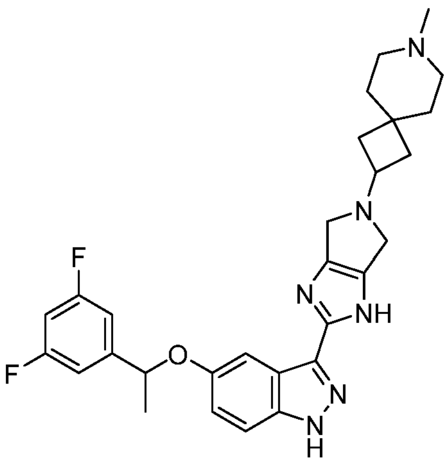 5-(1-(3,5-Difluorophenyl)ethoxy)-3-(5-(7-methyl-7-azaspiro[3.5]nonan-2-yl)-1,4,5,6-tetrahydropyrrolo[3,4-d]imidazol-2-yl)-1H-indazole | 519.3 | 519.2 |
| | | | |

(continued)

| No. | Structure Formula and Name of Compound | LC-MS Theoretical Calculated values $(M+1)^+$ | LC-MS Measured values $(M+1)^+$ |
|---|---|---|---|
| 41 | 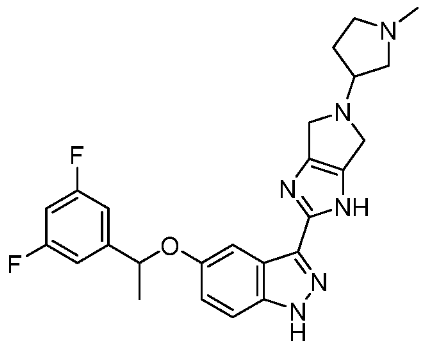 5-(1-(3,5-Difluorophenyl)ethoxy)-3-(5-(1-methylpyrrolidin-3-yl)-1,4,5,6-tetrahydro pyrrolo[3,4-d]imidazol-2-yl)-1H indazole | 465.2 | 465.2 |
| 42 | 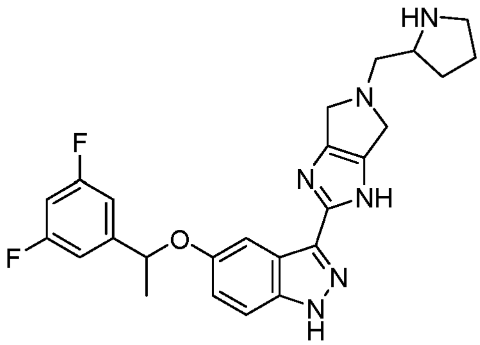 5-(1-(3,5-Difluorophenyl) ethoxy)-3-(5- (pyrrolidin-2-ylmethyl)-1,4,5,6-tetrahydropyrrolo[3,4-d]imidazol-2-yl)-1H-indazole | 465.2 | 465.2 |
| 43 | 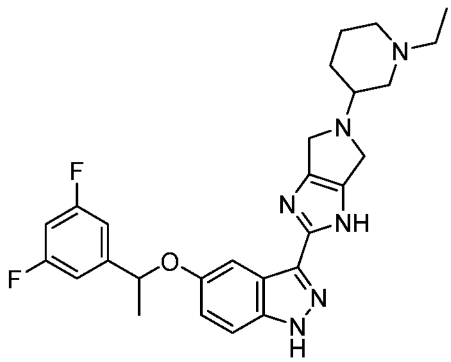 5-(1-(3,5-Difluorophenyl)ethoxy)-3-(5-(1-ethylpiperidin-3-yl)-1,4,5,6-tetrahydro pyrrolo[3,4-d]imidazol-2-yl)-1H indazole | 493.2 | 493.2 |

(continued)

| No. | Structure Formula and Name of Compound | LC-MS Theoretical Calculated values $(M+1)^+$ | LC-MS Measured values $(M+1)^+$ |
|---|---|---|---|
| 44 | 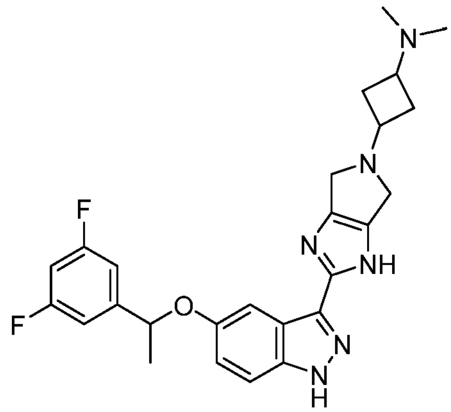 3-(2-(5-(1-(3,5-difluorophenyl) ethoxy)-1H-indazol-3-yl)-4,6-dihydropyrrolo[3,4-d]imidazol-5(1H)-yl)-N,N-dimethyl cyclobutan-1-amine | 479.2 | 479.2 |
| 45 | 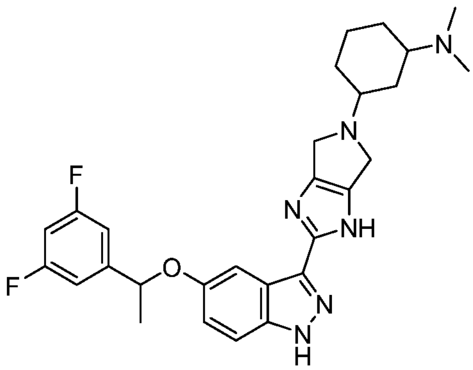 3 -(2-(5-(1-(3,5-difluorophenyl) ethoxy)-1H-indazol-3-yl)-4,6-dihydropyrrolo[3,4-d]imidazol-5(1H)-yl)-N,N-dimethyl cyclohexan-1-amine | 507.3 | 507.2 |
| 46 | 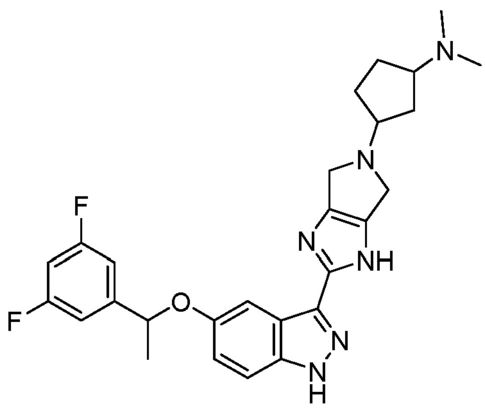 3-(2-(5-(1-(3,5-Difluorophenyl)ethoxy)-1H-indazol-3-yl)-4,6-dihydropyrrolo[3,4-d]imidazol-5 (1H)-yl)-N,N-dimethyl cyclopentan-1-amine | 493.2 | 493.2 |

**Examples 47-48:**

The compounds of Examples 47-48 were prepared according to the synthetic route of Example 9 using suitable

starting materials as follows.

| No. | Structure Formula and Name of Compound | LC-MS Theoretical Calculated values (M+1)+ | LC-MS Measured values (M+1)+ |
|---|---|---|---|
| 47 | 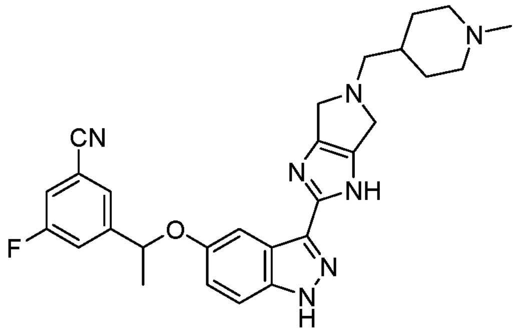 3-Fluoro-5-(1-(3-(5-((1-methylpiperidine-yl)methyl)-1,4,5,6-tetrahydropyrrolo [3,4-d]imidazol-2-yl)-1 H-indol-5-yl)oxy) ethyl)benzonitrile | 500.3 | 500.3 |
| 48 | 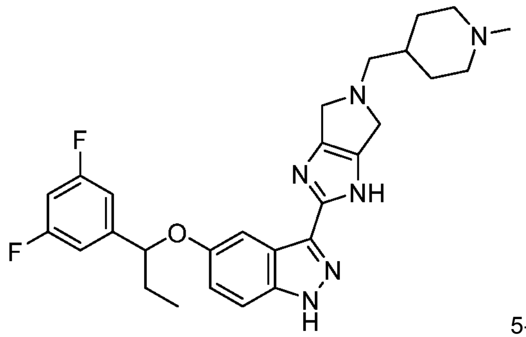 5-(1-(3,5-Difluorophenyl)propoxy)-3 -(5-((1-methylpiperidin-4-yl)methyl)-1,4,5,6-tetrahydropyrrolo[3,4-d ]imidazol-2-yl)-1 H-indazole | 507.3 | 507.3 |

**Assay: Determination of inhibitory activity against TRKA/B/C kinases**

**1. Materials and equipment**

| Materials and reagents | Manufacturer | Cat. No. |
|---|---|---|
| MgCl2 | Sigma | M1028 |
| ADP-Glo Kinase Assay | Promega | V9101 |
| DTT | Sigma | D0632 |
| DMSO | Sigma | D4540-1L |
| TRKA | Cama | 08-186 |
| TRKB | SignalChem | N17-11G |
| TRKC | Carna | 08-196 |
| ATP | Promega | V915B |
| Poly (4:1 Glu, Tyr) Peptide | SignalChem | P61-58 |
| Enterctinib | Selleck | S7998 |
| LOXO101 | MCE | HY-12866A |
| **Instruments and Equipment** | **Manufacturer** | **Cat. No. or Model No.** |
| 384-well plate, white, low volume, round-bottom | Greiner | 784075 |

(continued)

| Instruments and Equipment | Manufacturer | Cat. No. or Model No. |
|---|---|---|
| 96-well polypropylene plate | Nunc | 249944 |
| Microplate low-speed centrifuge | Xiang Zhi | TD5B |
| Biotek Enzyme Labeler | Biotek | Synergy 4 |

**2. Experimental steps**

2.1 Preparation of 1X kinase reaction buffer:

1x volume of 5X kinase reaction buffer and 4x volume of water; 5mM $MgCl_2$; and 1mMDTT.

2.2 Kinase and Substrate Preparation.

Preparation of 2.5X substrate mixture

| Kinase | ATP working concentration [μM] | Poly (4:1 Glu, Tyr) Peptide[mg/ml] |
|---|---|---|
| TRKA | 5 | 0.03 |
| TRKB | 20 | 0.03 |
| TRKC | 5 | 0.03 |

2.3 Screening of compounds:

1). Gradiently diluting a compound 4-fold in a dilution plate with DMSO, with a starting compound concentration of 1000 nM.
2). Diluting the compound 50-fold into 1X kinase reaction buffer and shaking it on a shaker for 20 minutes.
3). Formulating 2X kinase with 1X of enzyme reaction buffer.
4). Adding 2 μl kinase (formulated in step 3) to each well of the reaction plate.
5). Adding 1 μl of the compound diluted in buffer to each well, sealing the plate with a sealing membrane and centrifuging the plate at 1000g for 30 seconds and leaving it at room temperature for 10 minutes.
6). Formulating 4x ATP & sub mixture with 1X enzyme reaction buffer and adding 1μl of 4x ATP & sub mixture to the reaction plate.
7). Sealing the plate with a sealing membrane and centrifuging the plate at 1000g for 30 seconds and allowing it to react at room temperature for 60 minutes.
8).Transferring 4 μl of ADP-Glo to 384 reaction plate and centrifuging the plate at 1000 rpm/min for 1 min, and then incubating it at 25°C for 40 min.
9). Transferring 8 μl of Detection solution to 384 reaction plate and centrifuging the plate at 1000 rpm/min for 1 min and incubating it at 25°C for 40 min.
10) Reading the RLU (Relative luminescence unit) signal using a Biotek multifunctional plate reader. The signal intensity was used to characterize the activity degree of kinase.

**3. Data analysis**

3.1 The inhibition rate was calculated as follows: inhibition rate of compound (% inh) = 100% - (compound - positive control) / (negative control - positive control) * 100%.
3.2 Calculation of IC50 and plotting of inhibition curves for the compounds.

The IC50 (half inhibition concentration) of a compound was obtained using the following non-linear fitting equation, wherein data analysis was performed using Graphpad 6.0 software.

$$Y=Bottom + (Top-Bottom)/(1+10^{((LogIC50-X)*Hill\ Slope)})$$

X: log value of compound concentration; and Y: inhibition rate (% inhibition)

3.3 Validation of results

Data were exported from Biotek and analyzed manually. The ratios were converted to inhibition rates and IC50 was calculated by Prism GraphPad 6.0 from the inhibition rates. IC50 was calculated again by the ratios to verify accuracy of the results.

3.4 Quality control

Z-factor > 0.5; and S/B >2
Positive control IC50 was within 3 times of the average value

**4. Results**

The results were shown in table 1.

Table 1: Inhibition of TRKA/B/C kinases by compounds of examples

| **No.** | **$IC_{50}$ (nM)** | | |
|---|---|---|---|
| | **TRKA** | **TRKB** | **TRKC** |
| **LOXO-101** | 0.42 | 1.45 | 0.53 |
| **1** | 1.8 | 2.8 | 5.5 |
| **2** | 3.5 | 3.4 | 3.3 |
| **3** | 6.4 | 5.5 | 11 |
| **4** | 2.0 | 2.4 | 3.1 |
| **5** | 2.3 | 2.1 | 3.1 |
| **6** | 0.97 | 3.2 | 1.4 |
| **7** | 1.4 | 3.4 | 1.3 |
| **8** | 0.7 | 2.8 | 1.6 |
| **9** | 1.7 | 28 | 4.6 |
| **10** | 0.77 | 2.5 | 1.3 |
| **11** | 1.4 | 2.2 | 2.4 |
| **13** | 6.9 | 4.4 | 9.2 |
| **14** | 3.5 | 9.5 | 11.5 |
| **15** | 0.75 | 0.65 | 0.86 |
| **16** | 1.3 | 1.9 | 1.8 |
| **17** | 0.66 | 4.2 | 1.5 |
| **18** | 0.8 | 1.1 | 1.5 |
| **19** | 1.2 | 4.8 | 4.1 |
| **20** | 1.5 | 4.0 | 2.9 |
| **21** | 1.8 | 6.8 | 2.5 |
| **22** | 0.47 | 0.82 | 0.51 |
| **23** | 0.59 | 1.5 | 1.1 |
| **24** | 1.5 | 5.3 | 2.2 |
| **25** | 1.8 | 6.8 | 1.6 |

(continued)

| No. | $IC_{50}$ (nM) | | |
|---|---|---|---|
| | TRKA | TRKB | TRKC |
| **26** | 2.8 | 5.5 | 2.0 |
| **27** | 0.42 | 3.1 | 3.5 |
| **28** | 0.53 | 3.9 | 1.4 |
| **29** | 1.5 | 2.2 | 1.8 |
| **30** | 0.94 | 2.0 | 1.2 |
| **31** | 1.1 | 4.3 | 1.1 |
| **32** | 0.23 | 0.63 | 0.43 |
| **33** | 0.46 | 4.1 | 1.2 |
| **34** | 4.6 | 2.0 | 4.5 |
| **38** | 4.1 | 1.9 | 3.6 |
| **39** | 6.8 | 1.0 | 2.3 |
| **40** | 9.8 | 0.82 | 8.2 |
| **41** | 7.4 | 3.0 | 7.7 |
| **42** | 1.0 | 1.1 | 1.5 |
| **43** | 1.9 | 1.4 | 2.9 |
| **44** | 1.4 | 0.64 | 1.8 |
| **45** | 1.6 | 0.83 | 1.7 |
| **46** | 10.6 | 1.4 | 2.9 |
| **47** | 14.2 | 8.9 | 8.6 |
| **48** | 4.5 | 4.2 | 6.1 |

The tested example compounds each showed similar or better inhibitory activity against TRKA, TRKB and TRKC kinases compared to LOXO-101.

Although specific embodiments of the present disclosure have been illustrated and described, it does not mean that these embodiments illustrate and describe all possible implementation forms of the present disclosure. More precisely, the language used in this specification are only descriptive words and not restrictive. It will be obvious to those skilled in the art that various kinds of changes and modifications can be made without departing from the scope of the appended claims. Therefore, the appended claims are intended to include all these changes and modifications within the scope of the present invention.

## Claims

**1.** A compound of Formula (I),

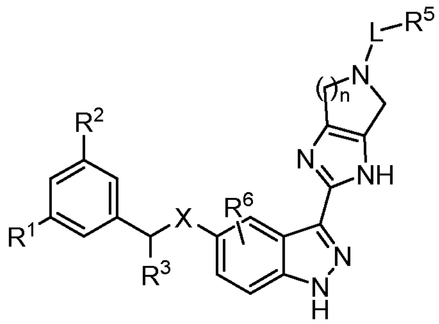

(I)

or an isotopically labeled compound thereof, or an optical isomer thereof, a geometric isomer thereof, a tautomer thereof, or a pharmaceutically acceptable salt thereof,

wherein

$R^1$ and $R^2$ are each independently selected from H, -CN, $C_{1-4}$ alkyl, $C_{1-4}$ haloalkyl, $C_{1-4}$ alkoxy, and halogen; and

$R^3$ is selected from H, $C_{1-4}$ alkyl, $C_{1-4}$ haloalkyl, $C_{1-4}$ alkoxy, $C_{3-6}$ alicyclic group, and 4-6 membered heteroalicyclic group; and

1, 2 or 3 $R^6$(s) are present in formula (I), and each $R^6$ is independently selected from H, halogen, -CN, -OH, $-NO_2$, $-NR^7R^8$, $C_{1-4}$ alkyl, $C_{1-4}$ haloalkyl, $C_{1-4}$ alkoxy, $C_{3-6}$ alicyclic group, and 4-6 membered heteroalicyclic group; and

X is a bond, O, S or ($NR^4$) in which $R^4$ is selected from H, $C_{1-4}$ alkyl, $C_{1-4}$ haloalkyl, $C_{1-4}$ alkoxy, $C_{3-6}$ alicyclic group, and 4-6 membered heteroalicyclic group; and

$R^7$ and $R^8$ are each independently selected from H, $C_{1-6}$ alkyl, $C_{1-4}$ haloalkyl, and $C_{1-4}$ alkoxy, or $R^7$ and $R^8$ and the atom(s) attached thereto together form a 3-6-membered ring; and

n=1, 2 or 3; and

L is (C=O), (O=S=O), ((C=O)-$CH_2$) or $CR^aR^b$ or a bond in which $R^a$ and $R^b$ are each indepdnently selected from H, $C_{1-4}$ alkyl, $C_{1-4}$ haloalkyl, $C_{1-4}$ alkoxy, $C_{3-6}$ alicyclic group, and 4-6 membered heteroalicyclic group, or $R^a$, $R^b$, and the carbon atom(s) attached thereto together form a 3-6-membered ring; and

$R^5$ is selected from H, halogen, -OH, $-NO_2$, -CN, $-SF_5$, -SH, -S-$C_{1-4}$ alkyl, $C_{1-6}$ alkyl, $C_{1-6}$ haloalkyl, $C_{1-6}$ alkoxy, $C_{1-6}$ haloalkoxy, $C_{2-8}$ alkenyl, $C_{2-8}$ alkynyl, $C_{3-7}$ alicyclic group, 3-10 membered heteroalicyclic group, $C_{6-12}$ bicyclic alicyclic group, 6-12 membered bicyclic heteroalicyclic group, $C_{8-15}$ tricyclic alicyclic group, 8-15 membered tricyclic heteroalicyclic group, $C_{5-8}$ aryl, 5-10 membered heteroaryl, $C_{7-11}$ bicyclic aryl, 7-11 membered bicyclic heteroaryl, -$C_{1-4}$ alkyl-($C_{3-7}$ alicyclic group), -$C_{1-4}$ alkyl-(3-10 membered heteroalicyclic group), -$C_{1-4}$ alkyl-($C_{6-12}$ bicyclic alicyclic group), -$C_{1-4}$ alkyl-(6-12 membered bicyclic heteroalicyclic group), -$C_{1-4}$ alkyl-($C_{8-15}$ tricyclic alicyclic group), -$C_{1-4}$ alkyl-(8-15 membered tricyclic heteroalicyclic group), -$C_{1-4}$ alkyl-($C_{5-8}$ aryl), -$C_{1-4}$ alkyl-(5-10 membered heteroaryl), -N($R^{10}$)($R^{11}$), -N($R^{10}$)(C(=O)$R^{11}$), -N($R^{10}$)(C(=O)-O$R^{11}$), -N($R^{12}$)(C(=O)-N($R^{10}$)($R^{11}$)),-C(=O)-N($R^{10}$)($R^{11}$), -C(=O)-$R^{12}$, -C(=O)-O$R^{12}$, -OC(=O)$R^{12}$, -N($R^{10}$)(S(=O)$_2R^{11}$), -S(=O)$_2$-N($R^{10}$)($R^{11}$), -S$R^{12}$, and -O$R^{12}$, wherein the -S-$C_{1-4}$ alkyl, $C_{1-6}$ alkyl, $C_{1-6}$ alkoxy, $C_{1-6}$ haloalkoxy, $C_{2-8}$ alkenyl, $C_{2-8}$ alkynyl, $C_{3-7}$ alicyclic group, 3-10 membered heteroalicyclic group, $C_{6-12}$ bicyclic alicyclic group, 6-12 membered bicyclic heteroalicyclic group, Cs_is tricyclic alicyclic group, 8-15 membered tricyclic heteroalicyclic group, $C_{5-8}$ aryl, 5-7 membered heteroaryl, $C_{7-11}$ bicycloaryl, 7-11 membered bicyclic heteroaryl, -$C_{1-4}$ alkyl-($C_{3-7}$ alicyclic group), -$C_{1-4}$ alkyl-(3-10 membered heteroalicyclic group), -$C_{1-4}$ alkyl-($C_{6-12}$ bicyclic alicyclic group), -$C_{1-4}$ alkyl-(6-12 membered bicyclic heteroalicyclic group), -$C_{1-4}$ alkyl-(Cs_is tricyclic alicyclic group), -$C_{1-4}$ alkyl-(8-15 membered tricyclic heteroalicyclic group), -$C_{1-4}$ alkyl-($C_{5-8}$ aryl), and -$C_{1-4}$ alkyl-(5-10 membered heteroaryl) are each optionally substituted with 0, 1, 2, 3 or 4 $R^{5a}$; and $R^{5a}$ is independently selected from halogen, -OH, $-NO_2$, -CN, $-SF_5$, -SH, -S-$C_{1-4}$ alkyl, oxo, $C_{1-4}$ alkyl, $C_{1-4}$ haloalkyl, $C_{1-4}$ alkoxy, $C_{1-4}$ haloalkoxy, $C_{2-8}$ alkenyl, $C_{2-8}$ alkynyl, $C_{3-7}$ alicyclic group, 3-10 membered heteroalicyclic group, $C_{5-8}$ aryl, 5-7 membered heteroaryl, -N($R^{13}$)($R^{14}$), -N($R^{13}$)(C(=O)$R^{14}$), -N($R^{13}$)(C(=O)-O$R^{14}$), -N($R^{15}$)(C(=O)-N($R^{13}$)($R^{14}$)), -C(=O)-N($R^{13}$)($R^{14}$), -C(=O)-$R^{15}$, -C(=O)-O$R^{15}$, -OC(=O)$R^{15}$,-N($R^{13}$)(S(=O)$_2R^{14}$), -S(=O)$_2$-N($R^{13}$)($R^{14}$), -S$R^{15}$, and -O$R^{15}$; and

$R^{10}$, $R^{11}$, $R^{12}$, $R^{13}$, $R^{14}$ and $R^{15}$, at each occurrence, are each independently selected from H, $C_{1-6}$ alkyl, $C_{1-6}$ haloalkyl, $C_{3-7}$ alicyclic group, 3-10 membered heteroalicyclic group, $C_{5-8}$ aryl, 5-7 membered heteroaryl, $C_{7-11}$ bicycloaryl, 7-11 membered bicyclic heteroaryl, -$C_{1-4}$ alkyl-($C_{3-7}$ alicyclic group), -$C_{1-4}$ alkyl-(3-10 membered heteroalicyclic group), -$C_{1-4}$ alkyl-($C_{6-12}$ bicyclic alicyclic group), -$C_{1-4}$ alkyl-(6-12 membered bicyclic heteroal-

icyclic group), -$C_{1-4}$ alkyl-(Cs_is tricyclic alicyclic group), -$C_{1-4}$ alkyl-(8-15 membered tricyclic heteroalicyclic group), -$C_{1-4}$ alkyl-($C_{5-8}$ aryl), and -$C_{1-4}$ alkyl-(5-10 membered heteroaryl), wherein each substituent listed in the group is optionally substituted with 0, 1, 2, 3, or 4 substituents each independently selected from a group consisting of: halogen, -OH, -$NH_2$, -$NH(CH_3)$, -$N(CH_3)_2$, -CN, -$NO_2$, -$SF_5$, -SH, -S-$C_{1-4}$ alkyl, oxo, $C_{1-4}$ alkyl, $C_{2-6}$ alkenyl, $C_{2-6}$ alkynyl, $C_{3-7}$ alicyclic group, 3-10 membered heteroalicyclic group, $C_{5-8}$ aryl, 5-7 membered heteroaryl, $C_{7-11}$ bicycloaryl, 7-11 membered bicyclic heteroaryl, $C_{1-4}$ hydroxyalkyl, -S-$C_{1-4}$ alkyl, -C(=O)H, -C(=O)-$C_{1-4}$ alkyl, -C(=O)-O-$C_{1-4}$ alkyl, -C(=O)-$NH_2$, -C(=O)-N($C_{1-4}$ alkyl)$_2$, $C_{1-4}$ haloalkyl, $C_{1-4}$ alkoxy and $C_{1-4}$ haloalkoxy;
or $R^{10}$, $R^{11}$, and the atom(s) attached thereto together form a 3-14-membered ring;
or $R^{13}$, $R^{14}$, and the atom(s) attached thereto together form a 3-14-membered ring.

**2.** The compound or an isotopically labeled compound thereof, or an optical isomer thereof, a geometric isomer thereof, a tautomer thereof, or a pharmaceutically acceptable salt thereof, according to claim 1, wherein n is 1 or 2.

**3.** The compound or an isotopically labeled compound thereof, or an optical isomer thereof, a geometric isomer thereof, a tautomer thereof or a pharmaceutically acceptable salt thereof, according to claim 1 or 2, wherein X is -O- or -NH-.

**4.** The compound or an isotopically labeled compound thereof, or an optical isomer thereof, a geometric isomer thereof, a tautomer thereof, or a pharmaceutically acceptable salt thereof, according to any one of claims 1 to 3, wherein $R^1$ and $R^2$ are each independently selected from H, F, Cl and Br.

**5.** The compound or an isotopically labeled compound thereof, or an optical isomer thereof, a geometric isomer thereof, a tautomer thereof, or a pharmaceutically acceptable salt thereof, according to any one of claims 1 to 4, wherein L is -(C=O)-, -(O=S=O)-, -$CH_2$-,-$C(CH_3)_2$-, -$CH(CH_3)$- or a bond.

**6.** The compound or an isotopically labeled compound thereof, or an optical isomer thereof, a geometric isomer thereof, a tautomer thereof, or a pharmaceutically acceptable salt thereof, according to any one of claims 1 to 5, wherein $R^6$ is H.

**7.** The compound or an isotopically labeled compound thereof, or an optical isomer thereof, a geometric isomer thereof, a tautomer thereof, or a pharmaceutically acceptable salt thereof according to any one of claims 1 to 6, wherein $R^5$ is selected from H, halogen, -OH, - $NO_2$, -CN, -$SF_5$, -SH, -S-$C_{1-4}$ alkyl, $C_{1-6}$ alkyl, $C_{1-6}$ haloalkyl, $C_{1-6}$ alkoxy, $C_{1-6}$ haloalkoxy, $C_{3-6}$ alicyclic group, 4-6 membered heteroalicyclic group, $C_{5-8}$ aryl, 5-10 membered heteroaryl, - $C_{1-4}$ alkyl-($C_{3-7}$ alicyclic group), -$C_{1-4}$ alkyl-(3-10 membered heteroalicyclic group), -$C_{1-4}$ alkyl-($C_{5-8}$ aryl), -$C_{1-4}$ alkyl-(5-10 membered heteroaryl), -N($R^{10}$)($R^{11}$), wherein the -S-$C_{1-4}$ alkyl, $C_{1-6}$ alkyl, $C_{1-6}$ haloalkyl, $C_{1-6}$ alkoxy, $C_{1-6}$ haloalkoxy, $C_{3-6}$ alicyclic group, 4-6 membered heteroalicyclic group, $C_{5-8}$ aryl, 5-10 membered heteroaryl, -$C_{1-4}$ alkyl-($C_{3-7}$ alicyclic group), - $C_{1-4}$ alkyl-(3-10 membered heteroalicyclic group), -$C_{1-4}$ alkyl-($C_{5-8}$ aryl), and -$C_{1-4}$ alkyl-(5-10 membered heteroaryl) are each optionally substituted with 0, 1, 2, 3 or 4 $R^{5a}$; and $R^{5a}$ is independently selected from halogen, -OH, -$NO_2$, -CN, oxo, $C_{1-4}$ alkyl, $C_{1-4}$ haloalkyl, $C_{1-4}$ alkoxy, $C_{1-4}$ haloalkoxy, $C_{3-7}$ alicyclic group, 3-10 membered heteroalicyclic group, $C_{5-8}$ aryl, 5-7 membered heteroaryl; and
$R^{10}$, $R^{11}$ and $R^{12}$, $R^{13}$, $R^{14}$ and $R^{15}$, at each occurrence, are each independently selected from H, $C_{1-6}$ alkyl, $C_{1-6}$ haloalkyl, $C_{3-7}$ alicyclic group, 3-10 membered heteroalicyclic group wherein each substituent listed in the group is optionally substituted with 0, 1, 2, 3, or 4 substituents each independently selected from a group consisting of: halogen, -OH, -$NH_2$, oxo, $C_{1-4}$ alkyl, $C_{1-4}$ hydroxyalkyl, $C_{1-4}$ haloalkyl, $C_{1-4}$ alkoxy and $C_{1-4}$ haloalkoxy; or $R^{10}$, $R^{11}$, and the atom(s) attached thereto together form a 3-8-membered ring.

**8.** The compound or an isotopically labeled compound thereof, or an optical isomer thereof, a geometric isomer thereof, a tautomer thereof, or a pharmaceutically acceptable salt thereof according to claim 7, wherein $R^5$ is selected from H, halogen, -OH, methyl, ethyl, propyl, butyl, pentyl, hexyl, methoxy, ethoxy, propoxy, butoxy, pentyloxy, hexyloxy, cyclopropyl, cyclobutyl, cyclopentyl, cyclohexyl, cycloheptyl, phenyl, piperazinyl, piperidinyl, morpholinyl, pyrrolidinyl, pyrrolyl, morpholinyl, pyridinyl, and pyrazolyl, wherein the methyl, ethyl, propyl, butyl, pentyl, hexyl, methoxy, ethoxy, propoxy, butoxy, pentyloxy, hexyloxy, cyclopropyl, cyclobutyl, cyclopentyl, cyclohexyl, cycloheptyl, phenyl, piperazinyl, piperidinyl, morpholinyl, pyrrolidinyl, pyrrolyl, morpholinyl, pyridinyl, and pyrazolyl are each optionally substituted with 1 or 2 $R^{5a}$, and $R^{5a}$ is independently selected from halogen, -OH, -$NO_2$, -CN, oxo, $C_{1-4}$ alkyl, $C_{1-4}$ haloalkyl, and $C_{1-4}$ alkoxy.

**9.** The compound or an isotopically labeled compound thereof, or an optical isomer thereof, a geometric isomer thereof, a tautomer thereof or a pharmaceutically acceptable salt thereof, according to claim 1, wherein the compound is

selected from:

N-(1-(1-(3,5-difluorophenyl)ethyl)-3-(1,4,5,6-tetrahydropyrrolo[3,4-d]imidazol-2-yl)-1H-indazol-5-amine;
N-(3,5-difluorobenzyl)-3-(5-(piperidin-4-ylmethyl)-1,4,5,6-tetrahydropyrrolo[3,4-d] imidazol-2-yl)-1H-indazol-5-amine;
5-(3,5-difluorobenzyl)-3-(5-(methylsulfonyl)-1,4,5,6-tetrahydropyrrolo[3,4-d]imidazol-2-yl)-1H-indazole;
5-(1-(3,5-difluorophenyl)ethoxy)-3-(5-(1-methylpiperidin-4-yl)-1,4,5,6-tetrahydropyrrolo [3,4-d]imidazol-2-yl)-1H-indazole;
2-(5-(1-(3,5-difluorophenyl)ethoxy)-1H-indazol-3-yl)-N,N-dimethyl-4,6-dihydropyrrolo [3,4-d]imidazole-5(1H) formamide;
2-(dimethylamino)ethyl 2-(5-(1-(3,5-difluorophenyl)ethoxy)-1H-indazol-3-yl)-4,6-dihydropyrrolo[3,4-d]imidazole-5(1H)-carboxylate;
(2-(5-(1-(3,5-difluorophenyl)ethoxy)-1H-indazol-3-yl)-4,6-dihydropyrrolo[3,4-d]imidazol-5(1H)-yl)(1-methylpiperidin-4-yl)ketone;
2-(5-(1-(3,5-difluorophenyl)ethoxy)-1H-indazol-3-yl)-5-(1-methylpiperidin-4-yl)-4,5,6,7 -tetrahydro-3H-imidazo[4,5-c]pyridine;
(S)-5-(1-(3,5-difluorophenyl)ethoxy)-3-(5-(((1-methylpiperidin-4-yl)methyl)-1,4,5,6-tetrahydropyrrolo[3,4 -d]imidazol-2-yl)-1H-indazole;
1-methylpiperidin-4-yl-2-(5-(1-(3,5-difluorophenyl)ethoxy)-1H-indazol-3-yl)-4,6-dihydropyrrolo[3,4 -d]imidazole-5(1H)-carboxylate;
N-(1-(3,5-difluorophenyl)ethyl)-3-(5-(piperidin-4-ylmethyl)-1,4,5,6-tetrahydropyrrolo[3,4-d]imidazol-2-yl)-1H-indazol-5-amine;
N-(1-(1-,3,5-difluorophenyl)ethyl)-3-(5-(((1-methylpiperidin-4-yl)methyl)-1,4,5,6-tetrahydropyrrolo[3,4-d]imidazol-2-yl)-1H-indazol-5-amine;
Cyclopropyl (2-(5-(3,5-difluorobenzyl)-1H-indazol-3-yl)-4,6-dihydropyrrolo[3,4-d]imidazol-5(1H)-yl)ketone;
5-(3,5-difluorobenzyl)-3-(5-(1-methylpiperidin-4-yl)-1,4,5,6-tetrahydropyrrolo[3,4-d]imidazol-2-yl)-1H-indazole;
5-(1-(3,5-difluorophenyl)ethoxy)-3-(5-(((1-methylpiperidin-4-yl)methyl)-1,4,5,6-tetrahydropyrrolo[3,4-d]imidazol-2-yl)-1H-indazole;
5-(1-(3,5-difluorophenyl)ethoxy)-3-(5-(piperidin-4-ylmethyl)-1,4,5,6-tetrahydropyrrolo[3,4-d]imidazol-2-yl)-1H-indazole;
5-(1-(3,5-difluorophenyl)ethoxy)-3-(5-(((1-ethylpiperidin-4-yl)methyl)-1,4,5,6-tetrahydropyrrolo[3,4-d]imidazol-2-yl)-1H-indazole;
2-(2-(5-(1-(3,5-difluorophenyl)ethoxy)-1H-indazol-3-yl)-4,6-dihydropyrrolo[3,4-d]imidazol-5(1H)-yl)-N,N-dimethylethan-1-amine;
1-(4-((2-(5-(1-(3,5-difluorophenyl)ethoxy)-1H-indazol-3-yl)-4,6-dihydropyrrolo[3,4-d]imidazol-5(1H)-yl)methyl)piperidin-1-yl)ethan-1-one;
5-(1-(3,5-difluorophenyl)ethoxy)-3-(5-(((1-methylpyrrolidin-3-yl)methyl)-1,4,5,6-tetrahydropyrrolo[3,4-d]imidazol-2-yl)-1H-indazole;
5-(1-(3,5-difluorophenyl)ethoxy)-3-(5-(2-(1-methylpiperidin-4-yl)ethyl)-1,4,5,6-tetrahydropyrrolo[3,4-d ]imidazol-2-yl)-1H-indazole;
5-(1-(3,5-difluorophenyl)ethoxy)-3-(5-(((1-isopropylpiperidin-4-yl)methyl)-1,4,5,6-tetrahydropyrrolo[3,4-d]imidazol-2-yl)-1H-indazole;
5-(1-(3,5-difluorophenyl)ethoxy)-3-(5-(1-(1-(1-methylpiperidin-4-yl)ethyl)-1,4,5,6-tetrahydropyrrolo[3,4-d ]imidazol-2-yl)-1H-indazole;
5-(1-(3,5-difluorophenyl)ethoxy)-3-(5-(piperidin-3-ylmethyl)-1,4,5,6-tetrahydropyrrolo[3,4-d]imidazol-2-yl)-1H-indazole;
5-(1-(3,5-difluorophenyl)ethoxy)-3-(5-(piperidin-2-ylmethyl)-1,4,5,6-tetrahydropyrrolo[3,4-d]imidazol-2-yl)-1H-indazole;
5-(1-(3,5-difluorophenyl)ethoxy)-3-(5-((1-methylazetidin-3-yl)methyl)-1,4,5,6-tetrahydropyrrolo[3,4-d]imidazol-2-yl)-1H-indazole;
3-(5-((1-cyclobutylpiperidin-4-yl)methyl)-1,4,5,6-tetrahydropyrrolo[3,4-d]imidazol-2-yl)-5-(1-(3,5-difluorophenyl)ethoxy)-1H-indazole;
5-(1-(3,5 -difluorophenyl)ethoxy)-3-(5-(((1-isopropyl-4-methylpiperidin-4-yl)methyl)-1,4,5,6-tetrahydropyrrolo[3,4-d]imidazol-2-yl)-1H-indazole;
(2-(5-(1-(3,5-difluorophenyl)ethoxy)-1H-indazol-3-yl)-4,6-dihydropyrrolo[3,4-d]imidazol-5(1H)-yl)(4-methylpiperazin-1-yl)ketone;
1-methylpyrrolidin-3-yl 2-(5-(1-(3,5-difluorophenyl)ethoxy)-1H-indazol-3-yl)-4,6-dihydropyrrolo[3,4-d]imidazole-5(1H)-carboxylate;

1-methylazetidin-3-yl 2-(5-(1-(3,5-difluorophenyl)ethoxy)-1H-indazol-3-yl)-4,6-dihydropyrrolo[3,4-d]imidazole-5(1H)-carboxylate;
(R)-5-(1-(3,5-difluorophenyl)ethoxy)-3-(5-(((1-methylpiperidin-4-yl)methyl)-1,4,5,6-tetrahydropyrrolo[3,4-d]imidazol-2-yl)-1H-indazole;
1-methylpiperidin-3-yl 2-(5-(1-(3,5-difluorophenyl)ethoxy)-1H-indazol-3-yl)-4,6-dihydropyrrolo[3,4-d]imidazole-5(1H)-carboxylate;
5-(1-(3,5-difluorophenyl)ethoxy)-3-(5-(1-(1-(1-methylazetidin-3-yl)ethyl)-1,4,5,6-tetrahydropyrrolo[3,4-d]imidazol-2-yl)-1H-indazole;
4-(2-(5-(1-(3,5-difluorophenyl)ethoxy)-1H-indazol-3-yl)-4,6-dihydropyrrolo[3,4-d]imidazol-5(1H)-yl)-N,N-dimethylcyclohexan-1-amine;
5-(1-(3,5-difluorophenyl)ethoxy)-3-(5-(1-(methylsulfonyl)piperidin-4-yl)methyl)-1,4,5,6-tetrahydropyrrolo[3,4-d]imidazol-2-yl)-1H indazole;
Methyl 4-((2-(5-(1-(3,5-difluorophenyl)ethoxy)-1H-indazol-3-yl)-4,6-dihydropyrrolo[3,4-d]imidazol-5(1H)-yl)methyl)piperidine-1-carboxylate;
5-(1-(3,5-difluorophenyl)ethoxy)-3-(5-(1-ethylpiperidin-4-yl)-1,4,5,6-tetrahydropyrrolo[3,4-d]imidazol-2-yl)-1H indazole;
5-(1-(3,5-difuurophenyl)ethoxy)-3-(5-(2-methyl-2-azaspiro[3.3]heptan-6-yl)-1,4,5,6-tetrahydropyrrolo[3, 4-d]imidazol-2-yl)-1H-indazole;
5-(1-(3,5-difluorophenyl)ethoxy)-3-(5-(7-methyl-7-azaspiro[3.5]nonan-2-yl)-1,4,5,6-tetrahydropyrrolo[3, 4-d]imidazol-2-yl)-1H-indazole;
5-(1-(3,5-difluorophenyl)ethoxy)-3-(5-(1-methylpyrrolidin-3-yl)-1,4,5,6-tetrahydropyrrolo[3,4-d]imidazol-2-yl)-1H-indazole;
5-(1-(3,5-difluorophenyl)ethoxy)-3-(5-(pyrrolidin-2-ylmethyl)-1,4,5,6-tetrahydropyrrolo[3,4-d]imidazol-2-yl)-1H-indazole;
5-(1-(3,5-difluorophenyl)ethoxy)-3-(5-(1-ethylpiperidin-3-yl)-1,4,5,6-tetrahydropyrrolo[3,4-d]imidazol-2-yl)-1H-indazole;
3-(2-(5-(1-(3,5-difluorophenyl)ethoxy)-1H-indazol-3-yl)-4,6-dihydropyrrolo[3,4-d]imidazol-5(1H)-yl)-N,N-dimethylcyclobutan-1-amine;
3-(2-(5-(1-(3,5-difluorophenyl)ethoxy)-1H-indazol-3-yl)-4,6-dihydropyrrolo[3,4-d]imidazol-5(1H)-yl)-N,N-dimethylcyclohexan-1-amine;
3-(2-(5-(1-(3,5-difluorophenyl)ethoxy)-1H-indazol-3-yl)-4,6-dihydropyrrolo[3,4-d]imidazol-5(1H)-yl)-N,N-dimethylcyclopentan-1-amine;
3-fluoro-5-(1-(3-(3-(5-((1-methylpiperidin-4-yl)methyl)-1,4,5,6-tetrahydropyrrolo[3,4-d ]imidazol-2-yl)-1H-indol-5-yl)oxy)ethyl)benzonitrile;
5-(1-(3,5-difluorophenyl)propoxy)-3-(5-((1-methylpiperidin-4-yl)methyl)-1,4,5,6-tetrahydropyrrolo[3,4-d ]imidazol-2-yl)-1 H-indazole.

**10.** The compound or an isotopically labeled compound thereof, or an optical isomer thereof, a geometric isomer thereof, a tautomer thereof, or a pharmaceutically acceptable salt thereof, according to claim 1, wherein the compound of formula (I) is

compounds shown in formula (Ia):

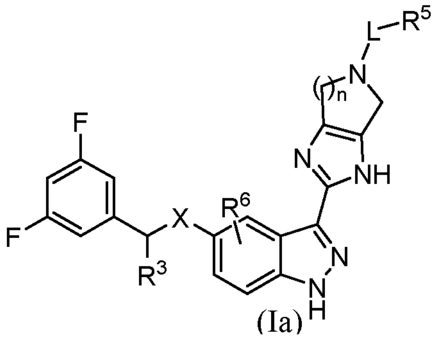

(Ia)

wherein X is -O- or -(NH)-; L, n, $R^3$, $R^5$, $R^6$ are defined in claim 1; or
compounds shown in formula (Ib):

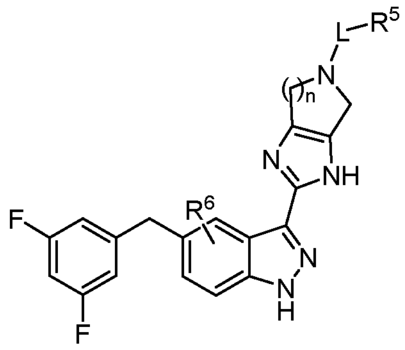

(Ib)

wherein L, n, $R^5$, $R^6$ are defined in claim 1; or compounds shown in formula (Ic):

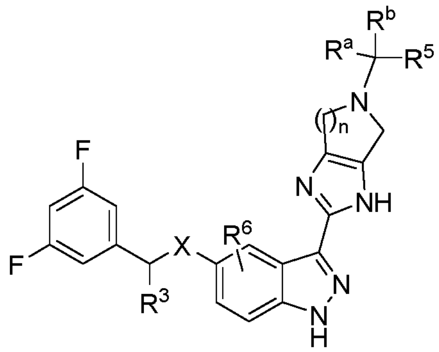

(Ic)

wherein X is -O- or -(NH)-; n, $R^3$, $R^5$, $R^6$, $R^a$ and $R^b$ are defined in claim 1; or compounds shown in formula (IIa):

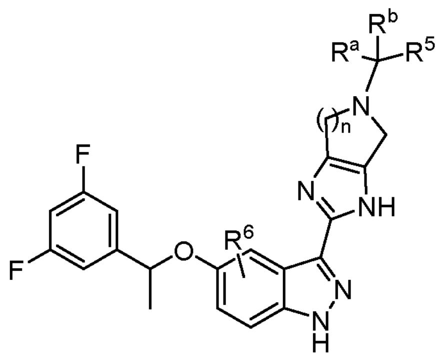

wherein n, $R^3$, $R^5$, $R^6$, $R^a$ and $R^b$ are defined in claim 1; or compounds shown in formula (IIb):

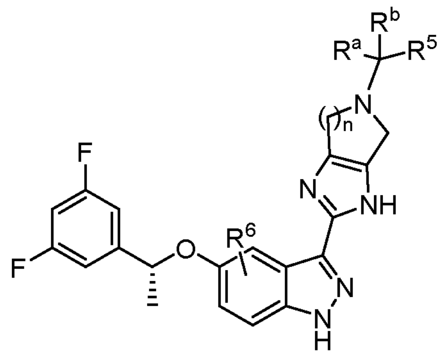

(IIb)

wherein n, $R^3$, $R^5$, $R^6$, $R^a$ and $R^b$ are defined in claim 1.

**11.** A pharmaceutical composition comprising the compound according to any one of claims 1 to 10, or an isotopically labeled compound thereof, or an optical isomer thereof, a geometric isomer thereof, a tautomer thereof, or a pharmaceutically acceptable salt thereof, and one or more pharmaceutically acceptable carriers, adjuvants, or excipients.

**12.** The compound according to any one of claims 1 to 10, or an isotopically labeled compound thereof, or an optical isomer thereof, a geometric isomer thereof, a tautomer thereof, or a pharmaceutically acceptable salt thereof, , or the pharmaceutical composition according to claim 11, for use in the treatment of diseases or conditions associated with TRK kinases or NTRK genes,wherein the diseases or conditions associated with TRK kinases or NTRK genes are selected from cancer, pain, inflammation, neurodegenerative diseases and cell proliferation disorders, preferably selected from hepatocellular carcinoma, breast cancer, bladder cancer, colorectal cancer, melanoma, mesothelioma, lung cancer, prostate cancer, membrane adenocarcinoma, pancreatic cancer, esophageal cancer, stomach cancer, lymphoma, leukemia, nasopharyngeal cancer, testicular cancer, thyroid cancer, squamous cell carcinoma, glioblastoma, neuroblastoma, uterine cancer, and rhabdomyosarcoma.

## Patentansprüche

**1.** Eine Verbindung der Formel (I),

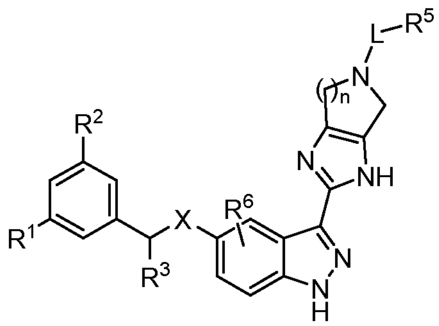

(I)

oder eine isotopenmarkierte Verbindung davon oder ein optisches Isomer davon, ein geometrisches Isomer davon, ein Tautomer davon oder ein pharmazeutisch verträgliches Salz davon,
wobei
$R^1$ und $R^2$ jeweils unabhängig aus H, -CN, $C_{1-4}$-Alkyl, $C_{1-4}$-Haloalkyl, $C_{1-4}$Alkoxy, und Halogen ausgewählt sind; und
$R^3$ aus H, $C_{1-4}$-Alkyl, $C_{1-4}$-Haloalkyl, $C_{1-4}$-Alkoxy, $C_{3-6}$-alicyclischer Gruppe und 4-6-gliedriger heteroalicyclischer Gruppe ausgewählt ist; und

1, 2 oder 3 $R^6$ in Formel (I) vorhanden sind und jedes $R^6$ unabhängig aus H, Halogen, - CN, -OH, $-NO_2$, $-NR^7R^8$, $C_{1-4}$-Alkyl, $C_{1-4}$-Haloalkyl, $C_{1-4}$-Alkoxy, $C_{3-6}$-alicyclischer Gruppe und 4-6-gliedriger heteroalicyclischer Gruppe ausgewählt ist; und

X eine Bindung, O, S oder ($NR^4$) ist, wobei $R^4$ aus H, $C_{1-4}$-Alkyl, $C_{1-4}$-Haloalkyl, $C_{1-4}$-Alkoxy, $C_{3-6}$-alicyclischer Gruppe und 4-6-gliedriger heteroalicyclischer Gruppe ausgewählt ist; und

$R^7$ und $R^8$ jeweils unabhängig aus H, $C_{1-6}$-Alkyl, $C_{1-4}$-Haloalkyl, und $C_{1-4}$-Alkoxy, oder $R^7$ und $R^8$ und die daran gebundenen Atome einen 3-6-gliedrigen Ring zusammen bilden; und

n = 1, 2 oder 3; und

L (C=O), (O=S=O), ((C=O)-$CH_2$), oder $CR^aR^b$ oder eine Bindung ist, in der $R^a$ und $R^b$ jeweils unabhängig ausgewählt sind aus H, $C_{1-4}$-Alkyl, $C_{1-4}$-Haloalkyl, $C_{1-4}$-Alkoxy, $C_{3-6}$-alicyclischer Gruppe und 4-6-gliedriger heteroalicyclischer Gruppe, oder $R^a$, $R^b$ und die daran gebundenen Kohlenstoffatome einen 3-6-gliedrigen Ring zusammen bilden; und

$R^5$ ausgewählt ist aus H, Halogen, -OH, $-NO_2$, -CN, $-SF_5$, -SH, -S-$C_{1-4}$-Alkyl, $C_{1-6}$-Alkyl, $C_{1-6}$-Haloalkyl, $C_{1-6}$-Alkoxy, $C_{1-6}$-Haloalkoxy, $C_{2-8}$-Alkenyl, $C_{2-8}$-Alkinyl, $C_{3-7}$-alicyclischer Gruppe, 3-10-gliedriger heteroalicyclischer Gruppe, $C_{6-12}$-bicyclischer alicyclischer Gruppe, 6-12-gliedriger bicyclischer heteroalicyclischer Gruppe, $C_{8-15}$-tricyclischer alicyclischer Gruppe, 8-15-gliedriger tricyclischer heteroalicyclischer Gruppe, $C_{5-8}$-Aryl, 5-10-gliedrigem Heteroaryl, $C_{7-11}$-bicyclischem Aryl, 7-11-gliedrigem bicyclischen Heteroaryl, -$C_{1-4}$-Alkyl-($C_{3-7}$-alicyclische Gruppe), -$C_{1-4}$-Alkyl-(3-10-gliedriger heteroalicyclische Gruppe), -$C_{1-4}$-Alkyl-($C_{6-12}$-bicyclische alicyclische Gruppe), -$C_{1-4}$-Alkyl-(6-12-gliedrige bicyclische heteroalicyclische Gruppe), -$C_{1-4}$-Alkyl-($C_{8-15}$-tricyclische alicyclische Gruppe), -$C_{1-4}$-Alkyl-(8-15-gliedrige tricyclische heteroalicyclische Gruppe), -$C_{1-4}$-Alkyl-($C_{5-8}$-Aryl), -$C_{1-4}$-Alkyl-(5-10-gliedriges Heteroaryl), $-N(R^{10})(R^{11})$, $-N(R^{10})(C(=O)R^{11})$, $-N(R^{10})(C(=O)-OR^{11})$,$-N(R^{12})(C(=O)-N(R^{10})(R^{11}))$, $-C(=O)-N(R^{10})(R^{11})$, $-C(=O)-R^{12}$, $-C(=O)-OR^{12}$, $-OC(=O)R^{12}$,$-N(R^{10})(S(=O)_2R^{11})$, $-S(=O)_2-N(R^{10})(R^{11})$, $-SR^{12}$, und $-OR^{12}$, wobei -S-$C_{1-4}$-Alkyl, $C_{1-6}$-Alkyl, $C_{1-6}$-Alkoxy, $C_{1-6}$-Haloalkoxy, $C_{2-8}$-Alkenyl, $C_{2-8}$-Alkinyl, $C_{3-7}$-alicyclische Gruppe, 3-10-gliedrige heteroalicyclische Gruppe, $C_{6-12}$-bicyclische alicyclische Gruppe, 6-12-gliedrige bicyclische heteroalicyclische Gruppe, $C_{8-15}$-tricyclische alicyclische Gruppe, 8-15-gliedrige tricyclische heteroalicyclische Gruppe, $C_{5-8}$-Aryl, 5-7-gliedriges Heteroaryl, $C_{7-11}$-Bicycloaryl, 7-11-gliedriges bicyclisches Heteroaryl, -$C_{1-4}$-Alkyl-($C_{3-7}$-alicyclische Gruppe), -$C_{1-4}$-Alkyl-( 3-10-gliedrige heteroalicyclische Gruppe), -$C_{1-4}$-Alkyl-($C_{6-12}$-bicyclische alicyclische Gruppe), -$C_{1-4}$-Alkyl-(6-12-gliedrige bicyclische heteroalicyclische Gruppe), - $C_{1-4}$-Alkyl-($C_{8-15}$-tricyclische alicyclische Gruppe), -$C_{1-4}$-Alkyl-(8-15-gliedrige tricyclische heteroalicyclische Gruppe), -$C_{1-4}$-Alkyl-($C_{5-8}$-Aryl) und -$C_{1-4}$-Alkyl-(5-10-gliedriges Heteroaryl) jeweils optional mit 0, 1, 2, 3 oder 4 $R^{5a}$ substituiert sind; und $R^{5a}$ unabhängig ausgewählt ist aus Halogen, -OH, $-NO_2$, -CN, $-SF_5$, -SH, -S-$C_{1-4}$-Alkyl, Oxo, $C_{1-4}$-Alkyl, $C_{1-4}$-Haloalkyl,$C_{1-4}$-Alkoxy, $C_{1-4}$-Haloalkoxy, $C_{2-8}$-Alkenyl, $C_{2-8}$-Alkinyl, $C_{3-7}$-alicyclischer Gruppe, 3-10-gliedriger heteroalicyclischer Gruppe, $C_{5-8}$-Aryl, 5-7-gliedrigem Heteroaryl,$-N(R^{13})(R^{14})$, $-N(R^{13})(C(=O)R^{14})$, $-N(R^{13})(C(=O)-OR^{14})$, $-N(R^{15})(C(=O)-N(R^{13})(R^{14}))$,$-C(=O)-N(R^{13})(R^{14})$, $-C(=O)-R^{15}$, $-C(=O)-OR^{15}$, $-OC(=O)R^{15}$, $-N(R^{13})(S(=O)_2R^{14})$, $-S(=O)_2-N(R^{13})(R^{14})$, $-SR^{15}$, und $-OR^{15}$; und

$R^{10}$, $R^{11}$, $R^{12}$, $R^{13}$, $R^{14}$ und $R^{15}$ bei jedem Vorkommen jeweils unabhängig ausgewählt sind aus H, $C_{1-6}$ -Alkyl, $C_{1-6}$ -Haloalkyl, $C_{3-7}$-alicyclischer Gruppe, 3-10-gliedriger heteroalicyclischer Gruppe, $C_{5-8}$-Aryl, 5-7-gliedrigem Heteroaryl, $C_{7-11}$-Bicycloaryl, 7-11-gliedrigem bicyclischen Heteroaryl, -$C_{1-4}$-Alkyl-($C_{3-7}$-alicyclische Gruppe), -$C_{1-4}$-Alkyl-(3-10-gliedrige heteroalicyclische Gruppe), -$C_{1-4}$-Alkyl-($C_{6-12}$-bicyclische alicyclische Gruppe), -$C_{1-4}$-Alkyl-(6-12-gliedrige bicyclische heteroalicyclische Gruppe), -$C_{1-4}$-Alkyl-($C_{8-15}$-tricyclische alicyclische Gruppe), -$C_{1-4}$-Alkyl-(8-15-gliedrige tricyclische heteroalicyclische Gruppe), Alkyl-(Cs-s-Aryl), und -$C_{1-4}$-Alkyl-(5-10-gliedriges Heteroaryl), wobei jeder in der Gruppe aufgeführte Substituent optional mit 0, 1, 2, 3 oder 4 Substituenten substituiert ist, die jeweils unabhängig aus einer Gruppe ausgewählt sind, die bestehend aus: Halogen, -OH, - $NH_2$, $-NH(CH_3)$, $-N(CH_3)_2$, -CN, $-NO_2$, $-SF_5$, -SH, -S-$C_{1-4}$-Alkyl, Oxo, $C_{1-4}$-Alkyl, $C_{2-6}$-Alkenyl, $C_{2-6}$-Alkinyl, $C_{3-7}$-alicyclischer Gruppe, 3-10-gliedriger heteroalicyclischer Gruppe, $C_{5-8}$-Aryl, 5-7-gliedrigem Heteroaryl, $C_{7-11}$-Bicycloaryl, 7-11-gliedrigem bicyclischen Heteroaryl, $C_{1-4}$-Hydroxyalkyl, -S-$C_{1-4}$-Alkyl, -C(=O)H, -C(=O)-$C_{1-4}$-Alkyl, -C(=O)-O-$C_{1-4}$-Alkyl, $-C(=O)-NH_2$, -C(=O)-N($C_{1-4}$-Alkyl$)_2$, $C_{1-4}$-Haloalkyl, $C_{1-4}$-Alkoxy und $C_{1-4}$-Haloalkoxy;

oder $R^{10}$, $R^{11}$, und die daran gebundenen Atome bilden zusammen einen 3-14-gliedrigen Ring;

oder $R^{13}$, $R^{14}$, und die daran gebundenen Atome bilden zusammen einen 3-14-gliedrigen Ring.

**2.** Die Verbindung oder eine isotopenmarkierte Verbindung davon oder ein optisches Isomer davon, ein geometrisches Isomer davon, ein Tautomer davon oder ein pharmazeutisch verträgliches Salz davon gemäß Anspruch 1, wobei n 1 oder 2 ist.

**3.** Die Verbindung oder eine isotopenmarkierte Verbindung davon oder ein optisches Isomer davon, ein geometrisches Isomer davon, ein Tautomer davon oder ein pharmazeutisch verträgliches Salz davon gemäß Anspruch 1 oder 2,

wobei X -O- oder -NH- ist.

4. Die Verbindung oder eine isotopenmarkierte Verbindung davon oder ein optisches Isomer davon, ein geometrisches Isomer davon, ein Tautomer davon oder ein pharmazeutisch verträgliches Salz davon gemäß einem der Ansprüche 1 bis 3, wobei $R^1$ und $R^2$ jeweils unabhängig aus H, F, Cl und Br ausgewählt sind.

5. Die Verbindung oder eine isotopenmarkierte Verbindung davon oder ein optisches Isomer davon, ein geometrisches Isomer davon, ein Tautomer davon oder ein pharmazeutisch verträgliches Salz davon gemäß einem der Ansprüche 1 bis 4, wobei L -(C=O)-, -(O=S=O)-, $-CH_2-$, $-C(CH_3)_2-$, $-CH(CH_3)-$ oder eine Bindung ist.

6. Die Verbindung oder eine isotopenmarkierte Verbindung davon oder ein optisches Isomer davon, ein geometrisches Isomer davon, ein Tautomer davon oder ein pharmazeutisch verträgliches Salz davon gemäß einem der Ansprüche 1 bis 5, wobei $R^6$ H ist.

7. Die Verbindung oder eine isotopenmarkierte Verbindung davon oder ein optisches Isomer davon, ein geometrisches Isomer davon, ein Tautomer davon oder ein pharmazeutisch verträgliches Salz davon gemäß einem der Ansprüche 1 bis 6, wobei $R^5$ ausgewählt ist aus H, Halogen, -OH, $-NO_2$, -CN, $-SF_5$, -SH, -S-$C_{1-4}$-Alkyl, $C_{1-6}$-Alkyl, $C_{1-6}$6-Haloalkyl, $C_{1-6}$-Alkoxy, $C_{1-6}$-Haloalkoxy, $C_{3-6}$-alicyclischer Gruppe, 4-6-gliedriger heteroalicyclischer Gruppe, $C_{5-8}$-Aryl, 5-10-gliedriger Heteroarylgruppe, -$C_{1-4}$-Alkyl-($C_{3-7}$-alicyclische Gruppe), -$C_{1-4}$-Alkyl-(3-10-gliedrige heteroalicyclische Gruppe), -$C_{1-4}$-Alkyl-($C_{5-8}$-Aryl), -$C_{1-4}$-Alkyl-(5-10-gliedriges Heteroaryl), -N($R^{10}$)($R^{11}$), wobei -S-$C_{1-4}$-Alkyl, $C_{1-6}$Alkyl, $C_{1-6}$-Haloalkyl, $C_{1-6}$-Alkoxy, $C_{1-6}$Haloalkoxy, $C_{3-6}$-alicyclische Gruppe, 4-6-gliedrige heteroalicyclische Gruppe, $C_{5-8}$-Aryl, 5-10-gliedriges Heteroaryl, -$C_{1-4}$-Alkyl-($C_{3-7}$-alicyclische Gruppe), -$C_{1-4}$-Alkyl-(3-10-gliedrige heteroalicyclische Gruppe), -$C_{1-4}$-Alkyl-(C5-8-Aryl) und -$C_{1-4}$-Alkyl-(5-10-gliedriges Heteroaryl) jeweils optional mit 0, 1, 2, 3 oder 4 $R^{5a}$ substituiert sind; und $R^{5a}$ unabhängig ausgewählt ist aus Halogen, -OH, $-NO_2$, -CN, Oxo, $C_{1-4}$-Alkyl, $C_{1-4}$-Haloalkyl, $C_{1-4}$-Alkoxy, $C_{1-4}$-Haloalkoxy, $C_{3-7}$-alicyclischer Gruppe, 3-10-gliedriger heteroalicyclischer Gruppe, $C_{5-8}$-Aryl, 5-7-gliedrigem Heteroaryl; und
$R^{10}$, $R^{11}$ und $R^{12}$, $R^{13}$, $R^{14}$ und $R^{15}$ bei jedem Vorkommen jeweils unabhängig ausgewählt sind aus H, $C_{1-6}$-Alkyl, $C_{1-6}$-Haloalkyl, $C_{3-7}$-alicyclischer Gruppe, 3-10-gliedriger heteroalicyclischer Gruppe, wobei jeder in der Gruppe aufgeführte Substituent optional mit 0, 1, 2, 3 oder 4 Substituenten substituiert ist, die jeweils unabhängig aus einer Gruppe ausgewählt sind, die bestehend aus: Halogen, -OH, $-NH_2$, Oxo, $C_{1-4}$-Alkyl, $C_{1-4}$-Hydroxyalkyl, $C_{1-4}$-Haloalkyl, $C_{1-4}$-Alkoxy und $C_{1-4}$-Haloalkoxy; oder $R^{10}$, $R^{11}$, und die daran gebundenen Atome einen 3-8-gliedrigen Ring zusammen bilden.

8. Die Verbindung oder eine isotopenmarkierte Verbindung davon oder ein optisches Isomer davon, ein geometrisches Isomer davon, ein Tautomer davon oder ein pharmazeutisch verträgliches Salz davon gemäß Anspruch 7, wobei $R^5$ ausgewählt ist aus H, Halogen, -OH, Methyl, Ethyl, Propyl, Butyl, Pentyl, Hexyl, Methoxy, Ethoxy, Propoxy, Butoxy, Pentyloxy, Hexyloxy, Cyclopropyl, Cyclobutyl, Cyclopentyl, Cyclohexyl, Cycloheptyl, Phenyl, Piperazinyl, Piperidinyl, Morpholinyl, Pyrrolidinyl, Pyrrolyl, Morpholinyl, Pyridinyl und Pyrazolyl, wobei Methyl, Ethyl, Propyl, Butyl, Pentyl, Hexyl, Methoxy, Ethoxy, Propoxy, Butoxy, Pentyloxy, Hexyloxy, Cyclopropyl, Cyclobutyl, Cyclopentyl, Cyclohexyl, Cycloheptyl, Phenyl, Piperazinyl, Piperidinyl, Morpholinyl, Pyrrolidinyl, Pyrrolyl, Morpholinyl, Pyridinyl und Pyrazolyl jeweils optional mit 1 oder 2 $R^{5a}$ substituiert sind, und $R^{5a}$ unabhängig ausgewählt ist aus Halogen, -OH, $-NO_2$, -CN, Oxo, $C_{1-4}$-Alkyl, $C_{1-4}$-Haloalkyl und $C_{1-4}$-Alkoxy.

9. Die Verbindung oder eine isotopenmarkierte Verbindung davon oder ein optisches Isomer davon, ein geometrisches Isomer davon, ein Tautomer davon oder ein pharmazeutisch verträgliches Salz davon gemäß Anspruch 1, wobei die Verbindung ausgewählt ist aus:

N-(1-(1-(3,5-Difluorphenyl)ethyl)-3-(1,4,5,6-Tetrahydropyrrolo[3,4-d]imidazol-2-yl)-1H-Indazol-5-Amin;
N-(3,5-Difluorbenzyl)-3-(5-(Piperidin-4-ylmethyl)-1,4,5,6-Tetrahydropyrrolo[3,4-d] Imidazol-2-yl)-1H-Indazol-5-Amin;
5-(3,5-Difluorbenzyl)-3-(5-(Methylsulfonyl)-1,4,5,6-Tetrahydropyrrolo[3,4-d]imidazol-2-yl)-1H-Indazol;
5-(1-(3,5-Difluorphenyl)ethoxy)-3-(5-(1-Methylpiperidin-4-yl)-1,4,5,6-Tetrahydropyrrolo[3,4-d]imidazol-2-yl)-1H-Indazol;
2-(5-(1-(3,5-Difluorphenyl)ethoxy)-1H-Indazol-3-yl)-N,N-Dimethyl-4,6-Dihydropyrrolo[3,4-d]imidazol-5(1H)formamid;
2-(Dimethylamino)ethyl 2-(5-(1-(3,5-Difluorphenyl)ethoxy)-1H-Indazol-3-yl)-4,6-Dihydropyrrolo[3,4-d]imidazol-5(1H)-Carboxylat;
(2-(5-(1-(3,5-Difluorphenyl)ethoxy)-1H-Indazol-3-yl)-4,6-Dihydropyrrolo[3,4-d]imidazol-5(1H)-yl)(1-Methylpiperidin-4-yl)keton;
2-(5-(1-(3,5-Difluorphenyl)ethoxy)-1H-Indazol-3-yl)-5-(1-Methylpiperidin-4-yl)-4,5,6,7-Tetrahydro-3H-Imidazo[4,5-c]pyridin;
(S)-5-(1-(3,5-Difluorphenyl)ethoxy)-3-(5-(((1-Methylpiperidin-4-yl)methyl)-1,4,5,6-Tetrahydropyrrolo[3,4-d]imidazol-2-yl)-1H-Indazol;
1-Methylpiperidin-4-yl-2-(5-(1-(3,5-Difluorphenyl)ethoxy)-1H-Indazol-3-yl)-4,6-Dihydropyrrolo[3,4-d]imidazol-5(1H)-Carboxylat;
N-(1-(3,5-Difluorphenyl)ethyl)-3-(5-(Piperidin-4-ylmethyl)-1,4,5,6-Tetrahydropyrrolo[3,4-d]imidazol-2-yl)-1H-Indazol-5-Amin;
N-(1-(1-,3,5-Difluorphenyl)ethyl)-3-(5-(((1-Methylpiperidin-4-yl)methyl)-1,4,5,6-Tetrahydropyrrolo[3,4-d]imidazol-2-yl)-1H-Indazol-5-Amin;
Cyclopropyl (2-(5-(3,5-Difluorbenzyl)-1H-Indazol-3-yl)-4,6-Dihydropyrrolo[3,4-d]imidazol-5(1H)-yl)keton;
5-(3,5-Difluorbenzyl)-3-(5-(1-Methylpiperidin-4-yl)-1,4,5,6-Tetrahydropyrrolo[3,4-d]imidazol-2-yl)-1H-Indazol;
5-(1-(3,5-Difluorphenyl)ethoxy)-3-(5-(((1-Methylpiperidin-4-yl)methyl)-1,4,5,6-Tetrahydropyrrolo[3,4-d]imidazol-2-yl)-1H-Indazol;
5-(1-(3,5-Difluorphenyl)ethoxy)-3-(5-(Piperidin-4-ylmethyl)-1,4,5,6-Tetrahydropyrrolo[3 ,4-d]imidazol-2-yl)-1H-Indazol;
5-(1-(3,5-Difluorphenyl)ethoxy)-3-(5-(((1-Ethylpiperidin-4-yl)methyl)-1,4,5,6-Tetrahydropyrrolo[3,4-d]imidazol-2-yl)-1H-Indazol;
2-(2-(5-(1-(3,5-Difluorphenyl)ethoxy)-1H-Indazol- 3-yl)-4,6-Dihydropyrrolo[3,4-d]imidazol-5(1H)-yl)-N,N-Dimethylethan-1-Amin;
1-(4-((2-(5-(1-(3,5-Difluorphenyl)ethoxy)-1H-Indazol-3-yl)-4,6-Dihydropyrrolo[3,4-d]imidazol-5(1H)-yl)methyl)piperidin-1-yl)ethan-1-On;
5-(1-(3,5-Difluorphenyl)ethoxy)-3-(5-(((1-Methylpyrrolidin-3-yl)methyl)-1,4,5,6-Tetrahydropyrrolo[3,4-d]imidazol-2-yl)-1H-Indazol;
5-(1-(3,5-Difluorphenyl)ethoxy)-3-(5-(2-(1-Methylpiperidin-4-yl)ethyl)-1,4,5,6-Tetrahydropyrrolo[3,4-d ]imidazol-2-yl)-1H-Indazol;
5-(1-(3,5-Difluorphenyl)ethoxy)-3-(5-(((1-Isopropylpiperidin-4-yl)methyl)-1,4,5,6-Tetrahydropyrrolo[3,4-d]imidazol-2-yl)-1H-Indazol;
5-(1-(3,5-Difluorphenyl)ethoxy)-3-(5-(1-(1-(1-Methylpiperidin-4-yl)ethyl)-1,4,5,6-Tetrahydropyrrolo[3,4-d ]imidazol-2-yl)-1H-Indazol;
5-(1-(3,5-Difluorphenyl)ethoxy)-3-(5-(Piperidin-3-ylmethyl)-1,4,5,6-Tetrahydropyrrolo[3 ,4-d]imidazol-2-yl)-1H-Indazol;
5-(1-(3,5-Difluorphenyl)ethoxy)-3-(5-(Piperidin-2-ylmethyl)-1,4,5,6-Tetrahydropyrrolo[3 ,4-d]imidazol-2-yl)-1H-Indazol;
5-(1-(3,5-Difluorphenyl)ethoxy)-3-(5-((1-Methylazetidin-3-yl)methyl)-1,4,5,6-Tetrahydropyrrolo[3,4-d]imidazol-2-yl)-1H-Indazol;
3-(5-((1-Cyclobutylpiperidin-4-yl)methyl)-1,4,5,6-Tetrahydropyrrolo[3,4-d]imidazol-2-yl)-5-(1-(3,5-Difluorphenyl)ethoxy)-1H-Indazol,
5-(1-(3,5-Difluorphenyl)ethoxy)-3-(5-(((1-Isopropyl-4-Methylpiperidin-4-yl)methyl)-1,4,5,6-Tetrahydropyrrolo[3,4-d]imidazol-2-yl)-1H-Indazol;
(2-(5-(1-(3,5-Difluorphenyl)ethoxy)-1H-Indazol-3-yl)-4,6-Dihydropyrrolo[3,4-d]imidazol-5(1H)-yl)(4-Methylpiperazin-1-yl)keton;
1-Methylpyrrolidin-3-yl 2-(5-(1-(3,5-Difluorphenyl)ethoxy)-1H-Indazol-3-yl)-4,6-Dihydropyrrolo[3,4-d]imidazol-5(1H)-Carboxylat;
1-Methylazetidin-3 -yl 2-(5-(1-(3,5-Difluorphenyl)ethoxy)-1H-Indazol-3 -yl)-4,6-Dihydropyrrolo[3,4-d]imidazol-5(1H)-Carboxylat;
(R)-5-(1-(3,5-Difluorphenyl)ethoxy)-3-(5-(((1-Methylpiperidin-4-yl)methyl)-1,4,5,6-Tetrahydropyrrolo[3,4-d]imidazol-2-yl)-1H-Indazol;
1-Methylpiperidin-3 -yl 2-(5-(1-(3,5-Difluorphenyl)ethoxy)-1H-Indazol-3 -yl)-4,6-Dihydropyrrolo[3,4-d]imidazol-5(1H)-Carboxylat;
5-(1-(3,5-Difluorphenyl)ethoxy)-3-(5-(1-(1-(1-Methylazetidin-3-yl)ethyl)-1,4,5,6-Tetrahydropyrrolo[3,4-d]imidazol-2-yl)-1H-Indazol;
4-(2-(5-(1-(3,5-Difluorphenyl)ethoxy)-1H-Indazol-3-yl)-4,6-Dihydropyrrolo[3,4-d]imidazol-5(1H)-yl)-N,N-Dimethylcyclohexan-1-Amin;
5-(1-(3,5-Difluorphenyl)ethoxy)-3-(5-(1-(Methylsulfonyl)piperidin-4-yl)methyl)-1,4,5,6-Tetrahydropyrrolo[3,4-d]imidazol-2-yl)-1H-Indazol;
Methyl 4-((2-(5-(1-(3,5-Difluorphenyl)ethoxy)-1H-Indazol-3-yl)-4,6-Dihydropyrrolo[3,4-d]imidazol-5(1H)-yl)methyl)piperidin-1-Carboxylat;
5-(1-(3,5-Difluorphenyl)ethoxy)-3-(5-(1-Ethylpiperidin-4-yl)-1,4,5,6-Tetrahydropyrrolo[3,4-d]imidazol-2-yl)-1H-Indazol;
5-(1-(3,5-Difluorphenyl)ethoxy)-3-(5-(2-Methyl-2-Azaspiro[3.3]heptan-6-yl)-1,4,5,6-Tetrahydropyrrolo[3, 4-d]imidazol-2-yl)-1H-Indazol,
5-(1-(3,5-Difluorphenyl)ethoxy)-3-(5-(7-Methyl-7-Azaspiro[3.5]nonan-2-yl)-1,4,5,6-Tetrahydropyrrolo[3, 4-d]imidazol-2-yl)-1H-Indazol,
5-(1-(3,5-Difluorphenyl)ethoxy)-3-(5-(1-Methylpyrrolidin-3-yl)-1,4,5,6-Tetrahydropyrrolo[3,4-d]imidazol-2-yl)-1H-Indazol;
5-(1-(3,5-Difluorphenyl)ethoxy)-3-(5-(Pyrrolidin-2-ylmethyl)-1,4,5,6-Tetrahydropyrrolo[3,4-d]imidazol-2-yl)-1H-Indazol;
5-(1-(3,5-Difluorphenyl)ethoxy)-3-(5-(1-Ethylpiperidin-3-yl)-1,4,5,6-Tetrahydropyrrolo[3,4-d]imidazol-2-yl)-1H-Indazol;
3-(2-(5-(1-(3,5-Difluorphenyl)ethoxy)-1H-Indazol-3-yl)-4,6-Dihydropyrrolo[3,4-d]imidazol-5(1H)-yl)-N,N-Dimethylcyclobutan-1-Amin;
3-(2-(5-(1-(3,5-Difluorphenyl)ethoxy)-1H-Indazol-3-yl)-4,6-Dihydropyrrolo[3,4-d]imidazol-5(1H)-yl)-N,N-Dimethylcyclohexan-1-Amin;
3-(2-(5-(1-(3,5-Difluorphenyl)ethoxy)-1H-Indazol-3-yl)-4,6-Dihydropyrrolo[3,4-d]imidazol-5(1H)-yl)-N,N-Dimethylcyclopentan-1-Amin;
3-Fluor-5-(1-(3-(3-(5-((1-Methylpiperidin-4-yl)methyl)-1,4,5,6-Tetrahydropyrrolo[3,4-d]imidazol-2-yl)-1H-Indol-5-yl)oxy)ethyl)benzonitril;
5-(1-(3,5-Difluorphenyl)propoxy)-3-(5-((1-Methylpiperidin-4-yl)methyl)-1,4,5,6-Tetrahydropyrrolo[3,4-d]imidazol-2-yl)-1 H-Indazol.

**10.** Die Verbindung oder eine isotopenmarkierte Verbindung davon oder ein optisches Isomer davon, ein geometrisches Isomer davon, ein Tautomer davon oder ein pharmazeutisch verträgliches Salz davon gemäß Anspruch 1, wobei die Verbindung der Formel (I)

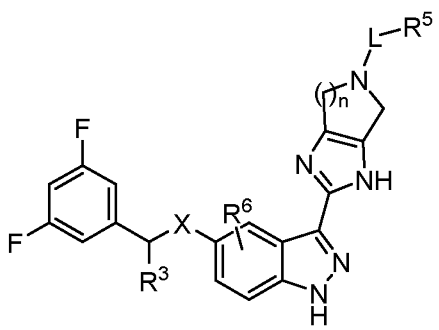

(Ia)

Verbindungen ist, die in Formel (Ia) gezeigt sind: wobei X -O- oder -(NH)- ist; L, n, $R^3$, $R^5$, $R^6$ in Anspruch 1 definiert sind; oder
Verbindungen ist, die in Formel (Ib) gezeigt sind:

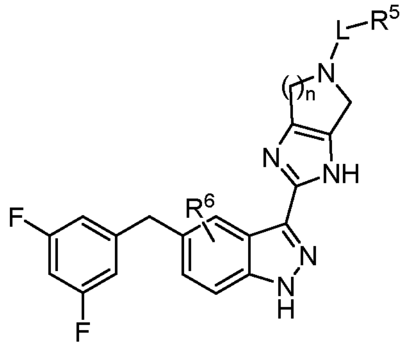

(Ib)

wobei L, n, $R^5$, $R^6$ in Anspruch 1 definiert sind; oder Verbindungen ist, die in Formel (Ic) gezeigt sind:

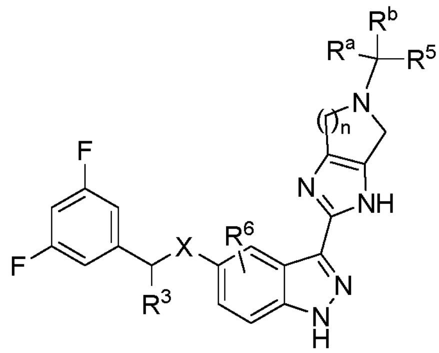

(Ic)

wobei X -O- oder -(NH)- ist; L, n, $R^3$, $R^5$, $R^6$ und $R^b$ in Anspruch 1 definiert sind; oder Verbindungen ist, die in Formel (IIa) gezeigt sind:

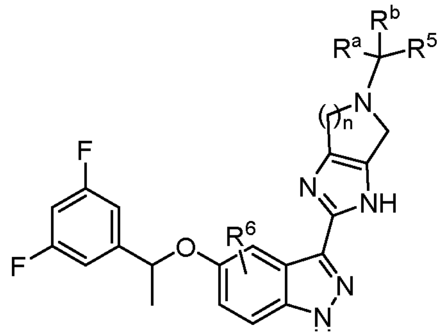

(IIa)

wobei n, $R^3$, $R^5$, $R^6$, $R^a$ und $R^b$ in Anspruch 1 definiert sind; oder Verbindungen ist, die in Formel (IIb) gezeigt sind:

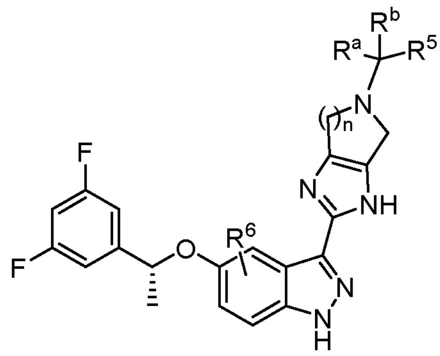

(IIb)

wobei n, $R^3$, $R^5$, $R^6$, $R^a$ und $R^b$ in Anspruch 1 definiert sind.

**11.** Eine pharmazeutische Zusammensetzung, die die Verbindung gemäß einem der Ansprüche 1 bis 10 oder eine isotopenmarkierte Verbindung davon oder ein optisches Isomer davon, ein geometrisches Isomer davon, ein Tautomer davon, oder ein pharmazeutisch verträgliches Salz davon und einen oder mehrere pharmazeutisch verträgliche Träger, Adjuvantien oder Hilfsstoffe umfasst.

**12.** Die Verbindung gemäß einem der Ansprüche 1 bis 10 oder eine isotopenmarkierte Verbindung davon oder ein optisches Isomer davon, ein geometrisches Isomer davon, ein Tautomer davon oder ein pharmazeutisch verträgliches Salz davon oder die pharmazeutische Zusammensetzung gemäß Anspruch 11 zur Verwendung bei der Behandlung von Krankheiten oder Zuständen, die mit TRK-Kinasen oder NTRK-Genen in Zusammenhang stehen.wobei die Krankheiten oder Zustände, die mit TRK-Kinasen oder NTRK-Genen in Zusammenhang stehen, ausgewählt sind aus Krebs, Schmerzen, Entzündungen, neurodegenerativen Erkrankungen und Zellproliferationsstörungen, vorzugsweise ausgewählt aus Leberzellkarzinom, Brustkrebs, Blasenkrebs, Dickdarmkrebs, Melanom, Mesotheliom, Lungenkrebs, Prostatakrebs, Membranadenokarzinom, Bauchspeicheldrüsenkrebs, Speiseröhrenkrebs, Magenkrebs, Lymphom, Leukämie, Nasopharynxkarzinom, Hodenkrebs, Schilddrüsenkrebs, Plattenepithelkarzinom, Glioblastom, Neuroblastom, Gebärmutterkrebs und Rhabdomyosarkom.

## Revendications

**1.** Composé de Formule (I),

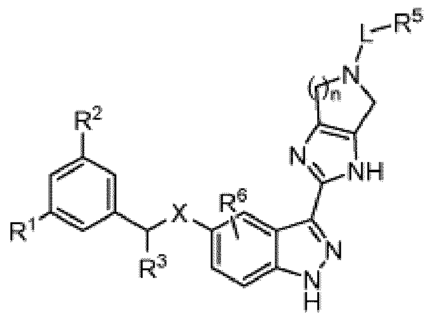

(I)

ou un composé isotopiquement marqué de celui-ci, ou un isomère optique de celui-ci, un isomère géométrique de celui-ci, un tautomère de celui-ci ou un sel pharmaceutiquement acceptable de celui, dans lequel,
$R^1$ et $R^2$ sont choisis chacun indépendamment parmi H, -CN, alkyle en C1-4, haloalkyle en C1-4, alcoxyle en C1-4 et halogène, et
$R^3$ est choisi parmi H, alkyle en C1-4, haloalkyle en C1-4, alcoxyle en C1-4, un groupe alicyclique en C3-6 et un groupe hétéroalicyclique à 4-6 chaînons ; et

1, 2 ou 3 $R^6$(s) se présentent dans la Formule (I), et chaque $R^6$ est choisis indépendamment parmi H, halogène, -CN, -OH, $-NO_2$, $-NR^7R^8$, alkyle en C1-4, haloalkyle en C1-4, alcoxyle en C1-4, un groupe alicyclique en C3-6 et un groupe hétéroalicyclique à 4-6 chaînons ; et

X est une liaison, O, S ou ($NR^4$), où $R^4$ est choisis parmi H, alkyle en C1-4, haloalkyle en C1-4, alcoxyle en C1-4, un groupe alicyclique en C3-6 et un groupe hétéroalicyclique à 4-6 chaînons ; et

$R^7$ et $R^8$ sont choisis chacun indépendamment parmi H, alkyle en C1-6, haloalkyle en C1-4 et alcoxyle en C1-4, ou $R^7$ et $R^8$ et les atomes attachés à ceux-ci forment ensemble un cycle à 3 à 6 chaînons ; et

n=1, 2 ou 3 ; et

L est (C=O), (O=S=O), ((C=O)-$CH_2$) ou $CR^aR^b$ ou une liaison, où $R^a$ et $R^b$ sont choisis chacun indépendamment parmi H, alkyle en C1-4, halcoxyle en C1-4, alcoxyle en C1-4, un groupe alicyclique en C3-6 et un groupe hétéroalicyclique à 4-6 chaînons, ou $R^a$, $R^b$ et les atomes de carbone attachés à ceux-ci forment ensemble un cycle à 3 à 6 chaînons ; et

$R^5$ est choisi parmi H, halogène, -OH, $-NO_2$, -CN, $-SF_5$, -SH, $-S-C_{1-4}$ alkyle, alkyle en C1-6, halcoxyle en C1-6, alcoxyle en C1-6, halcoxyle en C1-6, alcényle en C2-8, alcynyle en C2-8, un groupe alicyclique en C3-7, un groupe hétéroalicyclique à 3-10 chaînons, un groupe alicyclique bicyclique en C6-12, un groupe hétéroalicyclique bicyclique à 6-12 chaînons, un groupe alicyclique tricyclique en C8-15, un groupe hétéroalicyclique tricyclique à 8-15 chaînons, aryle en C5-8, hétéroaryle à 5-10 chaînons, aryle bicyclique en C7-11, hétéroaryle bicyclique à 7-11 chaînons, -alkyle en C1-4-(groupe alicyclique en C3-7), -alkyle en C1-4-(groupe hétéroalicyclique à 3 à 10 chaînons), alkyle en C1-4 (groupe alicyclique bicyclique en C6-12), alkyle en C1-4 (groupe hétéroalicyclique bicyclique à 6-12 chaînons), alkyle en C1-4 (groupe alicyclique tricyclique en C8-15), -alkyle en C1-4 (groupe hétéroalicyclique tricyclique à 8 à 15 chaînons), -alkyle en C1-4(aryle en C5-8), -alkyle en C1-4-(hétéroaryle à 5-10 chaînons), $-N(R^{10})(R^{11})$, $-N(R^{10})(C(=O)R^{11})$, $-N(R^{10})(C(=O)-OR^{11})$, $-N(R^{12})(C(=O)-N(R^{10})(R^{11}))$, $-C(=O)-N(R^{10})(R^{11})$, $-C(=O)-R^{12}$, $-C(=O)-OR^{12}$, $-OC(=O)R^{12}$, $-N(R^{10})(S(=O)_2R^{11})$, $-S(=O)_2-N(R^{10})(R^{11})$, $-SR^{12}$, et $-OR^{12}$, où $-S-C_{1-4}$ alkyle, alkyle en C1-6, alcoxyle en C1-6, haloalcoxyle en C1-6, alcényle en C2-8, alcynyle en C2-8, un groupe alicyclique en C3-7, un groupe hétéroalicyclique à 3-10 chaînons, un groupe alicyclique bicyclique en C6-12, un groupe hétéroalicyclique bicyclique à 6-12 chaînons, un groupe alicyclique tricyclique C8-15, un groupe hétéroalicyclique tricyclique à 8-15 chaînons, aryle en C5-8, hétéroaryle à 5-7 chaînons, bicycloaryle en C7-11, hétéroaryle bicyclique à 7-11 chaînons, -alkyle en C1-4 -(groupe alicyclique en C3-7), -alkyle en C1-4 -(groupe hétéroalicyclique à 3-10 chaînon), -alkyl en C1-4-(groupe alicyclique bicyclique en C6-12), -alkyle en C1-4-(groupe hétéroalicyclique bicyclique à 6-12 chaînons), -alkyle en C1-4-(groupe alicyclique tricyclique en C8-15), -alkyle en C1-4-(groupe hétéroalicyclique tricyclique à 8-15 chaînons), -alkyle en C1-4-(aryle en C5-8 ), et -alkyl en C1-4 -(hétéroaryle à 5-10 chaînons) sont éventuellement substitués chacun par 0, 1, 2, 3 ou 4 $R^{5a}$ ; et $R^{5a}$ est indépendamment choisi parmi halogène, -OH, $-NO_2$, -CN, $-SF_5$, -SH, $-S-C_{1-4}$ alkyle, oxo, alkyle en C1-4, haloalkyle en C1-4, alcoxyle en C1-4, haloalcoxyle en C1-4, alcényle en C2-8, alcynyle en C2-8, groupe alicyclique en C3-7, groupe hétéroalicyclique à 3-10 chaînons, aryle en C5-8, hétéroaryle à 5-7 chaînons, $-N(R^{13})(R^{14})$, $-N(R^{13})(C(=O)R^{14})$, $-N(R^{13})(C(=O)-OR^{14})$, $-N(R^{15})(C(=O)-N(R^{13})(R^{14}))$, $-C(=O)-N(R^{13})(R^{14})$, $-C(=O)-R^{15}$, $-C(=O)-OR^{15}$, $-OC(=O)R^{15}$, $-N(R^{13})(S(=O)_2R^{14})$, $-S(=O)_2-N(R^{13})(R^{14})$, $-SR^{15}$, et $-OR^{15}$ ; et

$R^{10}$, $R^{11}$, $R^{12}$, $R^{13}$, $R^{14}$ et $R^{15}$, à chaque événement, sont choisis chacun indépendamment parmi H, alkyle en C1-6, haloalkyle en C1-6, un groupe alicyclique en C3-7, un groupe hétéroalicyclique à 3-10 chaînons, aryle en C5-8, hétéroaryle à 5-7 chaînons, bicycloaryle en C7-11, hétéroaryle bicyclique à 7-11 chaînons, -alkyle en C1-4-(groupe alicyclique C3-7), -alkyle en C1-4 (groupe hétéroalicyclique à 3-10 chaînons), -alkyle en C1-4-(groupe alicyclique bicyclique en C6-12), -alkyle en C1-4-(groupe hétéroalicyclique bicyclique à 6-12 chaînons), -alkyle en C1-4-(groupe alicyclique tricyclique en C8-15), -alkyle en C1-4-(groupe hétéroalicyclique tricyclique à 8-15 chaînons), -alkyle en C1-4-(aryl en C5-8), et -alkyle en C1-4-(hétéroaryle à 5-10 chaînons), dans lequel chaque substituant listé dans le groupe est éventuellement substitué par 0, 1, 2, 3 ou 4 substituants chacun choisi indépendamment dans un groupe composé de : halogène, -OH, $-NH_2$, $-NH(CH_3)$, $-N(CH_3)_2$, -CN, $-NO_2$, $-SF_5$, -SH, $-S-C_{1-4}$ alkyle, oxo, alkyle en C1-4, alcényle en C2-6, alcynyle en C2-6, un groupe alicyclique en C3-7, un groupe hétéroalicyclique à 3-10 chaînons, aryle en C5-8, hétéroaryle à 5-7 chaînons, bicycloaryle en C7-11, hétéroaryle bicyclique à 7-11 chaînon, hydroxyalkyle en C1-4 , $-S-C_{1-4}$ alkyle, -C(=O)H,- $C(=O)-C_{1-4}$ alkyle, $-C(=O)-O-C_{1-4}$ alkyle, $-C(=O)-NH_2$, $-C(=O)-N(C_{1-4}$ alkyle$)_2$, haloalkyle en C1-4, alcoxyle en C1-4 et haloalcoxyle en C1-4 ;

ou $R^{10}$, $R^{11}$, et les atomes attachés à ceux-ci forment ensemble un cycle à 3 à 14 chaînons ;

ou $R^{13}$, $R^{14}$, et les atomes attachés à ceux-ci forment ensemble un cycle à 3 à 14 chaînons.

**2.** Composé ou un composé isotopiquement marqué de celui-ci, ou un isomère optique de celui-ci, un isomère géométrique de celui-ci, un tautomère de celui-ci ou un sel pharmaceutiquement acceptable de celui selon la revendication 1, dans lequel n est égale à 1 ou 2.

**3.** Composé ou un composé isotopiquement marqué de celui-ci, ou un isomère optique de celui-ci, un isomère géométrique de celui-ci, un tautomère de celui-ci ou un sel pharmaceutiquement acceptable de celui selon la revendication 1 ou 2, dans lequel X est -O- ou -NH-.

**4.** Composé ou un composé isotopiquement marqué de celui-ci, ou un isomère optique de celui-ci, un isomère géométrique de celui-ci, un tautomère de celui-ci ou un sel pharmaceutiquement acceptable de celui selon l'une quelconque des revendications 1 à 3, dans lequel $R^1$ et $R^2$ sont choisis chacun indépendamment parmi H, F, Cl et Br.

**5.** Composé ou un composé isotopiquement marqué de celui-ci, ou un isomère optique de celui-ci, un isomère géométrique de celui-ci, un tautomère de celui-ci ou un sel pharmaceutiquement acceptable de celui selon l'une quelconque des revendications 1 à 4, dans lequel L est -(C=O)-, -(O=S=O)-, $-CH_2-$,- $C(CH_3)_2-$, $-CH(CH_3)-$ ou une liaison.

**6.** Composé ou un composé isotopiquement marqué de celui-ci, ou un isomère optique de celui-ci, un isomère géométrique de celui-ci, un tautomère de celui-ci ou un sel pharmaceutiquement acceptable de celui selon l'une quelconque des revendications 1 à 5, dans lequel $R^6$ est H.

**7.** Composé ou un composé isotopiquement marqué de celui-ci, ou un isomère optique de celui-ci, un isomère géométrique de celui-ci, un tautomère de celui-ci ou un sel pharmaceutiquement acceptable de celui selon l'une quelconque des revendications 1 à 6, dans lequel $R^5$ est choisi parmi H, halogène, -OH,- $NO_2$, -CN, $-SF_5$, -SH, $-S-C_{1-4}$ alkyle, alkyle en C1-6, haloalkyle en C1-6, alcoxyle en C1-6, haloalcoxyle en C1-6, un groupe alicyclique en C3-6, un groupe hétéroalicyclique à 4-6 chaînons, aryle en C5-8, hétéroaryle à 5-10 chaînons, -alkyl en C1-4 -(groupe alicyclique en C3-7), -alkyle en C1-4-(groupe hétéroalicyclique à 3-10 chaînons), -alkyle en C1-4- (aryle en C5-8), -alkyle en C1-4-(hétéroaryle à 5-10 chaînons), $-N(R^{10})(R^{11})$, dans lequel $-S-C_{1-4}$ alkyle, alkyle en C1-6, haloalkyle en C1-6, alcoxyle en C1-6, haloalcoxyle en C1-6, un groupe alicyclique en C3-6, un groupe hétéroalicyclique à 4-6 chaînons, aryle en C5-8, hétéroaryle à 5-10 chaînons, -alkyle en C1-4-(groupe alicyclique en C3-7), - alkyle en C1-4-(groupe hétéroalicyclique à 3-10 chaînons), -alkyle en C1-4-(aryle en C5-8) et -alkyle en C1-4-( hétéroaryle à 5-10 chaînons) sont éventuellement substitués chacun par 0, 1, 2, 3 or 4 $R^{5a}$ ; et $R^{5a}$ est indépendamment choisi parmi halogène, -OH, $-NO_2$, -CN, oxo, alkyle en C1-4, haloalkyle en C1-4, alcoxyle en C1-4, haloalcoxyle en C1-4, un groupe alicyclique en C3-7 , un groupe hétéroalicyclique à 3-10 chaînons, aryle en C5-8 et hétéroaryle à 5-7 chaînons ; et
$R^{10}$, $R^{11}$ et $R^{12}$, $R^{13}$, $R^{14}$ et $R^{15}$, à chaque événement, sont choisis chacun indépendamment parmi H, alkyle en C1-6, haloalkyle en C1-6, un groupe alicyclique en C3-7, un groupe hétéroalicyclique à 3-10 chaînons, dans lequel chaque substituant listé dans le groupe est éventuellement substitué par 0, 1, 2, 3 ou 4 substituants chacun choisi indépendamment dans un groupe composé de : halogène, -OH, $-NH_2$, oxo, alkyle en C1-4, hydroxyalkyle en C1-4, haloalkyle en C1-4, alcoxyle en C1-4 et haloalcoxyle en C1-4 ; ou $R^{10}$, $R^{11}$, et les atomes attachés à ceux-ci forment ensemble un cycle à 3 à 8 chaînons.

**8.** Composé ou un composé isotopiquement marqué de celui-ci, ou un isomère optique de celui-ci, un isomère géométrique de celui-ci, un tautomère de celui-ci ou un sel pharmaceutiquement acceptable de celui selon la revendication 7, dans lequel $R^5$ est choisi parmi H, halogène, -OH, le méthyle, l'éthyle, le propyle, le butyle, le pentyle, le hexyle, le méthoxy, l'éthoxy, le propoxy, le butoxy, le pentyloxy, l'hexyloxy, le cyclopropyle, le cyclobutyle, le cyclopentyle, le cyclohexyle, le cycloheptyle, le phényle, le pipérazinyle, le pipéridinyle, le morpholinyle, le pyrrolidinyle, le pyrrolyle, le morpholinyle, le pyridinyle et le pyrazolyle, dans lesquels le méthyle, l'éthyle, le propyle, le butyle, le pentyle, l'hexyle, le méthoxy, l'éthoxy, le propoxy, le butoxy, le pentyloxy, l'hexyloxy, le cyclopropyle, le cyclobutyle, le cyclopentyle, le cyclohexyle, le cycloheptyle, le phényle, le pipérazinyle, le pipéridinyle, le morpholinyle, le pyrrolidinyle, le pyrrolyle, le morpholinyle, le pyridinyle et le pyrazolyle sont chacun éventuellement substitués par 1 ou 2 $R^{5a}$, et $R^{5a}$ est choisi indépendamment parmi halogène, -OH, $-NO_2$, -CN, oxo, alkyle en C1-4, haloalkyle en C1-4 et alcoxyle en C1-4.

**9.** Composé ou un composé isotopiquement marqué de celui-ci, ou un isomère optique de celui-ci, un isomère géométrique de celui-ci, un tautomère de celui-ci ou un sel pharmaceutiquement acceptable de celui selon la revendication 1, dans lequel le composé est choisi parmi :

N-(1-(1-(3,5-difluorophényl)éthyl)-3-(1,4,5,6-tétrahydropyrrolo[3,4-d]imidazol-2-yl)-1H-indaz ol-5-amine ;
N-(3,5-difluorobenzyl)-3-(5-(pipéridine-4-ylméthyl)-1,4,5,6-tétrahydropyrrolo[3,4-d]imidazol-2-yl)-1H-indazol-5-amine ;
5-(3,5-difluorobenzyl)-3-(5-(méthylsulfonyl)-1,4,5,6-tétrahydropyrrolo[3,4-d]imidazol-2-yl)-1 H-indazole ;
5-(1-(3,5-difluorophényl)éthoxy)-3-(5-(1-méthylpipéridine-4-yl)-1,4,5,6-tétrahydropyrrolo[3,4 -d]imidazol-2-yl)-1H-indazole ;
2-(5-(1-(3,5-difluorophényl)éthoxy)-1H-indazol-3-yl)-N,N-diméthyl-4,6-dihydropyrrolo[3,4-d ]imidazole-5(1H) formamide ;
2-(diméthylamino)éthyl 2-(5-(1-(3,5-difluorophényl)éthoxy)-1H-indazol-3-yl)-4,6-dihydropyrrolo[3,4-d]imidazole-5(1H)-ca rboxylate ;
(2-(5-(1-(3,5-difluorophényl)éthoxy)-1H-indazol-3-yl)-4,6-dihydropyrrolo[3,4-d]imidazol-5(1 H)-yl)(1-méthylpipéridine-4-yl)cétone ;
2-(5-(1-(3,5-difluorophényl)éthoxy)-1H-indazol-3-yl)-5-(1-méthylpipéridine-4-yl)-4,5,6,7-tétr ahydro-3H-imidazo[4,5-c]pyridine ;
(S)-5-(1-(3,5-difluorophényl)éthoxy)-3-(5-(((1-méthylpipéridine-4-yl)méthyl)-1,4,5,6-tétrahyd ropyrrolo[3,4-d] imidazol-2-yl)-1H-indazole ;
1-méthylpipéridine-4-yl-2-(5-(1-(3,5-difluorophényl)éthoxy)-1H-indazol-3-yl)-4,6-dihydropyr rolo[3,4-d]imidazole-5(1H)-carboxylate ;
N-(1-(3,5-difluorophényl)éthyl)-3-(5-(pipéridine-4-ylméthyl)-1,4,5,6-tétrahydropyrrolo[3,4-d]i midazol-2-yl)-1H-indazol-5-amine ;
N-(1-(1-,3,5-difluorophényl)éthyl)-3-(5-(((1-méthylpipéridine-4-yl)méthyl)-1,4,5,6-tétrahydro pyrrolo[3,4-d]imidazol-2-yl)-1H-indazol-5-amine ;
cyclopropyle(2-(5-(3,5-difluorobenzyl)-1H-indazol-3-yl)-4,6-dihydropyrrolo[3,4-d]imidazol-5 (1H)-yl)cétone ;
5-(3,5-difluorobenzyl)-3-(5-(1-méthylpipéridine-4-yl)-1,4,5,6-tétrahydropyrrolo[3,4-d]imidazo l-2-yl)-1H-indazole ;
5-(1-(3,5-difluorophényl)éthoxy)-3-(5-(((1-méthylpipéridine-4-yl)méthyl)-1,4,5,6-tétrahydrop yrrolo[3,4-d]imidazol-2-yl)-1H-indazole ;
5-(1-(3,5-difluorophényl)éthoxy)-3-(5-(pipéridine-4-ylméthyl)-1,4,5,6-tétrahydropyrrolo[3,4-d ]imidazol-2-yl)-1H-indazole ;
5-(1-(3,5-difluorophényl)éthoxy)-3-(5-(((1-éthylpipéridine-4-yl)méthyl)-1,4,5,6-tétrahydropyrr olo[3,4-d]imidazol-2-yl)-1H-indazole ;
2-(2-(5-(1-(3,5-difluorophényl)éthoxy)-1H-indazol-3-yl)-4,6-dihydropyrrolo[3,4-d]imidazol-5( 1H)-yl)-N,N-diméthyléthanethan-1-amine ;
1-(4-((2-(5-(1-(3,5-difluorophényl)éthoxy)-1H-indazol-3-yl)-4,6-dihydropyrrolo[3,4-d]imidazo l-5(1H)-yl)méthyl)pipéridine-1-yl)éthane-1-one;
5-(1-(3,5-difluorophényl)éthoxy)-3-(5-(((1-méthylpyrrolidine-3-yl)méthyl)-1,4,5,6-tétrahydrop yrrolo[3,4-d]imidazol-2-yl)-1H-indazole ;
5-(1-(3,5-difluorophényl)éthoxy)-3-(5-(2-(1-méthylpipéridine-4-yl)éthyl)-1,4,5,6-tétrahydropy rrolo[3,4-d]imidazol-2-yl)-1H-indazole ;
5-(1-(3,5-difluorophényl)éthoxy)-3-(5-(((1-isopropylpipéridine-4-yl)méthyl)-1,4,5,6-tétrahydr opyrrolo[3,4-d]imidazol-2-yl)-1H-indazole ;
5-(1-(3,5-difluorophényl)éthoxy)-3-(5-(1-(1-(1-méthylpipéridine-4-yl)éthyl)-1,4,5,6-tétrahydro pyrrolo[3,4-d]imidazol-2-yl)-1H-indazole ;
5-(1-(3,5-difluorophényl)éthoxy)-3-(5-(pipéridine-3-ylméthyl)-1,4,5,6-tétrahydropyrrolo[3,4-d ]imidazol-2-yl)-1H-indazole ;
5-(1-(3,5-difluorophényl)éthoxy)-3-(5-(pipéridine-2-ylméthyl)-1,4,5,6-tétrahydropyrrolo[3,4-d ]imidazol-2-yl)-1H-indazole ;
5-(1-(3,5-difluorophényl)éthoxy)-3-(5-((1-méthylazétidine-3-yl)méthyl)-1,4,5,6-tétrahydropyrr olo[3,4-d]imidazol-2-yl)-1H-indazole ;
3-(5-((1-cyclobutylpipéridine-4-yl)méthyl)-1,4,5,6-tétrahydropyrrolo[3,4-d]imidazol-2-yl)-5-(1 -(3,5-difluorophényl)éthoxy)-1H-indazole ;
5-(1-(3,5-difluorophényl)éthoxy)-3-(5-(((1-isopropyl-4-méthylpipéridine-4-yl)méthyl)-1,4,5,6-tétrahydropyrrolo[3,4-d]imidazol-2-yl)-1H-indazole ;
(2-(5-(1-(3,5-difluorophényl)éthoxy)-1H-indazol-3-yl)-4,6-dihydropyrrolo[3,4-d]imidazol-5(1 H)-yl)(4-méthylpipérazine-1-yl)cétone ;
1-méthylpyrrolidine-3-yl 2-(5-(1-(3,5-difluorophényl)éthoxy)-1H-indazol-3-yl)-4,6-dihydropyrrolo[3,4-d]imidazole-5(1H)-ca rboxylate ;
1-méthylazétidine-3-yl 2-(5-(1-(3,5-difluorophényl)éthoxy)-1H-indazol-3-yl)-4,6-dihydropyrrolo[3,4-d]imidazole-5(1H)-ca rboxylate ;
(R)-5-(1-(3,5-difluorophényl)éthoxy)-3-(5-(((1-méthylpipéridine-4-yl)méthyl)-1,4,5,6-tétrahyd ropyrrolo[3,4-d] imidazol-2-yl)-1H-indazole ;
1-méthylpipéridine-3-yl 2-(5-(1-(3,5-difluorophényl)éthoxy)-1H-indazol-3-yl)-4,6-dihydropyrrolo[3,4-d]imidazole-5(1H)-ca rboxylate ;

5-(1-(3,5-difluorophényl)éthoxy)-3-(5-(1-(1-(1-méthylazétidine-3-yl)éthyl)-1,4,5,6-tétrahydrop yrrolo[3,4-d]imidazol-2-yl)-1H-indazole ;
4-(2-(5-(1-(3,5-difluorophényl)éthoxy)-1H-indazol-3-yl)-4,6-dihydropyrrolo[3,4-d]imidazol-5( 1H)-yl)-N,N-diméthylcyclohexane-l-amine ;
5-(1-(3,5-difluorophényl)éthoxy)-3-(5-(1-(méthylsulfonyl)pipéridine-4-yl)méthyl)-1.4.5.6-tétra hydropyrrolo[3,4-d]imidazol-2-yl)-1H indazole ;
Méthyl 4-((2-(5-(1-(3,5-difluorophényl)éthoxy)-1H-indazol-3-yl)-4,6-dihydropyrrolo[3,4-d]imidazol-5(1H) -yl)méthyl)pipéridine-1-carboxylate ;
5-(1-(3,5-difluorophényl)éthoxy)-3-(5-(1-éthylpipéridine-4-yl)-1,4,5,6-tétrahydropyrrolo[3,4-d ]imidazol-2-yl)-1H indazole ;
5-(1-(3,5-difluorophényl)éthoxy)-3-(5-(2-méthyl-2-azaspiro[3.3]heptan-6-yl)-1,4,5,6-tétrahydr opyrrolo[3,4-d] imidazol-2-yl)-1H-indazole ;
5-(1-(3,5-difluorophényl)éthoxy)-3-(5-(7-méthyl-7-azaspiro[3.5]nonan-2-yl)-1,4,5,6-tétrahydr opyrrolo[3,4-d] imidazol-2-yl)-1H-indazole ;
5-(1-(3,5-difluorophényl)éthoxy)-3-(5-(1-méthylpyrrolidine-3-yl)-1,4,5,6-tétrahydropyrrolo[3, 4-d]imidazol-2-yl)-1H-indazole ;
5-(1-(3,5-difluorophényl)éthoxy)-3-(5-(pyrrolidine-2-ylméthyl)-1,4,5,6-tétrahydropyrrolo[3,4-d]imidazol-2-yl)-1H-indazole ;
5-(1-(3,5-difluorophényl)éthoxy)-3-(5-(1-éthylpipéridine-3-yl)-1,4,5,6-tétrahydropyrrolo[3,4-d ]imidazol-2-yl)-1H-indazole ;
3-(2-(5-(1-(3,5-difluorophényl)éthoxy)-1H-indazol-3-yl)-4,6-dihydropyrrolo[3,4-d]imidazol-5( 1H)-yl)-N,N-diméthylcyclobutan-l-amine ;
3-(2-(5-(1-(3,5-difluorophényl)éthoxy)-1H-indazol-3-yl)-4,6-dihydropyrrolo[3,4-d]imidazol-5( 1H)-yl)-N,N-diméthylcyclohexane-l-amine ;
3-(2-(5-(1-(3,5-difluorophényl)éthoxy)-1H-indazol-3-yl)-4,6-dihydropyrrolo[3,4-d]imidazol-5( 1H)-yl)-N,N-diméthylcyclopentan-l-amine ;
3-fluoro-5-(1-(3-(3-(5-((1-méthylpipéridine-4-yl)méthyl)-1,4,5,6-tétrahydropyrrolo[3,4-d]imid azol-2-yl)-1H-indol-5-yl)oxy)éthyl)benzonitrile ;
5-(1-(3,5-difluorophényl)propoxy)-3-(5-((1-méthylpipéridine-4-yl)méthyl)-1,4,5,6-tétrahydrop yrrolo[3,4-d]imidazol-2-yl)-1H-indazole.

**10.** Composé ou un composé isotopiquement marqué de celui-ci, ou un isomère optique de celui-ci, un isomère géométrique de celui-ci, un tautomère de celui-ci ou un sel pharmaceutiquement acceptable de celui selon la revendication 1, dans lequel le composé de formule (I) est

composé représenté par la Formule (Ia) :

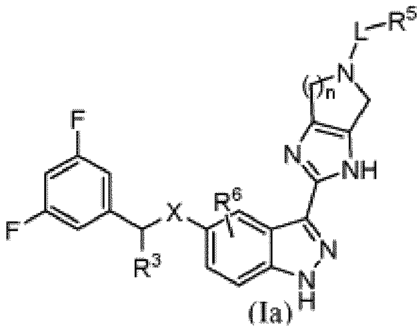

(Ia)

où X est -O- ou -(NH)- ; L, n, $R^3$, $R^5$ et $R^6$ sont définis dans la revendication 1 ; ou
composé représenté par la Formule (Ib) :

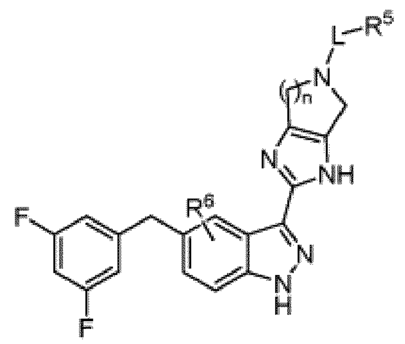

(Ib)

où L, n, $R^5$ et $R^6$ sont définis dans la revendication 1 ; ou composé représenté par la Formule (Ic) :

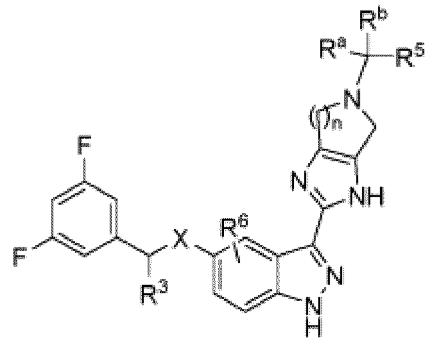

(Ic)

où X est -O- ou -(NH)- ; n, $R^3$, $R^5$, $R^6$, $R^a$ et $R^b$ sont définis dans la revendication 1 ; ou composé représenté par la Formule (IIa) :

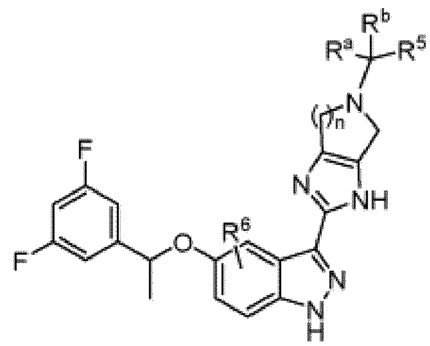

(IIa)

où n, $R^3$, $R^5$, $R^6$, $R^a$ et $R^b$ sont définis dans la revendication 1 ; ou composé représenté par la Formule (IIb) :

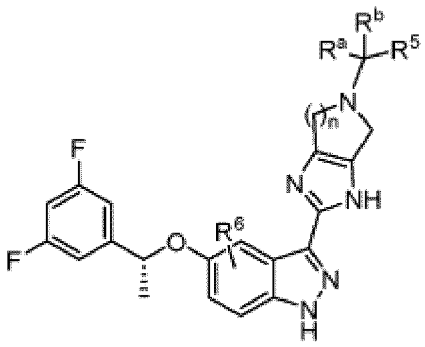

(IIb)

où n, $R^3$, $R^5$, $R^6$, $R^a$ et $R^b$ sont définis dans la revendication 1.

11. Composition pharmaceutique comprenant le composé selon l'une quelconque des revendications 1 à 10, ou un composé isotopiquement marqué de celui-ci, ou un isomère optique de celui-ci, un isomère géométrique de celui-ci, un tautomère de celui-ci ou un sel pharmaceutiquement acceptable de celui, et un ou plusieurs véhicules, adjuvants ou excipients pharmaceutiquement acceptables.

12. Composé selon l'une quelconque des revendications 1 à 10, ou un composé isotopiquement marqué de celui-ci, ou un isomère optique de celui-ci, un isomère géométrique de celui-ci, un tautomère de celui-ci ou un sel pharmaceutiquement acceptable de celui, ou composition pharmaceutique selon la revendication 11, pour utilisation dans le traitement de maladies ou des affections associées aux kinases TRK ou aux gènes NTRK, dans lesquels les maladies ou affections associées aux kinases TRK ou aux gènes NTRK sont choisies parmi le cancer, la douleur, l'inflammation, les maladies neurodégénératives et les troubles de la prolifération cellulaire, de préférence choisies parmi le carcinome hépatocellulaire, le cancer du sein, le cancer de la vessie, le cancer colorectal, le mélanome, le mésothéliome, le cancer pulmonaire, le cancer prostatique, l'adénocarcinome membranaire, le cancer pancréatique, le cancer de l'oesophage, le cancer de l'estomac, le lymphome, la leucémie, le cancer du nasopharynx, le cancer des testicules, le cancer thyroïdien, le carcinome épidermoïde, le glioblastome, le neuroblastome, le cancer de l'utérus et le rhabdomyosarcome.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021088859 A
- KR 20140019055


### Non-patent literature cited in the description


- **STAHL ; WERMUTH.** Handbook of Pharmaceutical Salts: Properties, Selection and Use. Wiley-VCH, 2002